(19) Europäisches Patentamt
European Patent Office
Office européen des brevets

(11) Publication number: 0 022 634 B1

(12) # EUROPEAN PATENT SPECIFICATION

(45) Date of publication of patent specification: 06.04.83

(21) Application number: 80302180.7

(22) Date of filing: 27.06.80

(51) Int. Cl.³: C 07 D 209/12, C 07 D 401/12, C 07 D 403/12, C 07 D 409/06, C 07 D 417/12, A 61 K 31/40, A 61 K 31/41, A 61 K 31/435 //(C07D401/12, 213/70, 209/12), (C07D403/12, 233/84, 209/12), (C07D409/06, 333/22, 209/14), (C07D417/12, 285/08, 209/12)

(54) **Acylated indole derivatives, processes for their preparation and pharmaceutical compositions and drugs comprising them.**

(30) Priority: 28.06.79 JP 80802/79
05.09.79 JP 112755/79
03.10.79 JP 126725/79
29.10.79 JP 138644/79
29.01.80 JP 8354/80

(43) Date of publication of application: 21.01.81 Bulletin 81/3

(45) Publication of the grant of the patent: 06.04.83 Bulletin 83/14

(84) Designated Contracting States: DE FR GB IT SE

(56) References cited:
GB - A - 869 775
US - A - 3 505 354

Chemical Abstracts, vol. 84, no. 3, 19 January 1976
Columbus, Ohio, USA
H. INION et al. "Indole series. III. 1-Alkyl- or aminoalkyl-3-indolyl pyridyl ketones having antiinflammatory and fibrinolytic properties" pages 443 to 444, abstract no. 17097u.

(73) Proprietor: TEIJIN LIMITED
11, 1-Chome, Minamihonmachi Higashi-ku
Osaka-shi Osaka (JP)

(72) Inventor: Oba, Takeo
Room 146, Teijin Tama Apartment 3-18-4, Tamadaira
Hino-shi Tokyo (JP)
Inventor: Tanaka, Toshio
5-15-6, Tamadaira
Hino-shi Tokyo (JP)
Inventor: Okamura, Noriaki
1-31-1, Sengawa-machi
Chofu-shi Tokyo (JP)
Inventor: Watanabe, Kenzo
3-5-18, Tamadaira
Hino-shi Tokyo (JP)
Inventor: Bannai, Kiyoshi
2-33-14, Asahigaoka
Hino-shi Tokyo (JP)
Inventor: Ohtsu, Akira
Room 223, Teijin Tama Apartment 3-18-4, Tamadaira
Hino-shi Tokyo (JP)
Inventor: Naruchi, Tatsuyuki
Room 141, Teijin Tama Apartment 3-18-4, Tamadaira
Hino-shi Tokyo (JP)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 022 634 B1

(72) Inventor: **Kurozumi, Seizi**
**5-20-2, Tamadaira**
**Hino-shi Tokyo (JP)**
Inventor: **Toru, Takeshi**
**973-69, Yano-machi**
**Hachioji-shi Tokyo (JP)**

(74) Representative: **Myerscough, Philip Boyd et al,**
**J.A.Kemp & Co. 14, South Square Gray's Inn**
**London, WC1R 5EU (GB)**

# Acylated indole derivatives, processes for their preparation and pharmaceutical compositions and drugs comprising them

This invention relates to acylated indole derivatives, a process for production thereof, and to a pharmaceutical composition containing such an acylated indole derivative as an active ingredient.

As acylated indole derivatives having pharmacological activities, indomethacin of the following formula

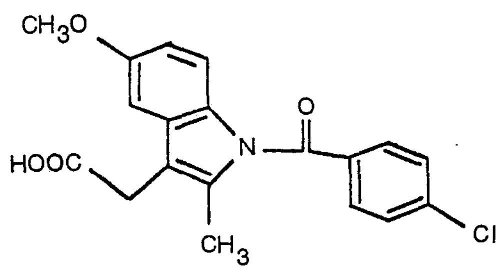

and clomethacin of the formula

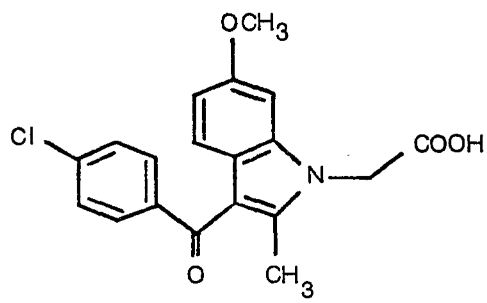

have been well known as compounds having anti-inflammatory activities. It is well known on the other hand that these compounds have a strong tendency to induce ulcer formation, and should still be improved.

Henri Invon et al. reported that acylated indole compounds of the following formula

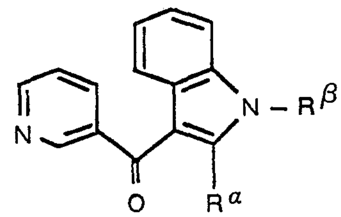

wherein $R^{\alpha}$ represents methyl, ethyl, isopropyl, iso-butyl, cyclohexyl, methoxy, or Cl- or Br-substituted phenyl, and $R^{\beta}$ represents hydrogen, methyl, ethyl, iso-propyl, iso-butyl, vinyl, n-pentyl, benzyl, chloro- or methoxy-substituted benzyl, carboxymethyl, ethoxycarbonylmethyl or $\alpha$-carboxyethyl $\beta$-(dimethylamino)ethyl,

have fibrinolytic activity (see Eur. J. Med. Chem.- Chimica Therapeutica, May-June 1975-10, No. 3, pp. 276—285).

Ryszard J. Grgglewiski et al. reported that a compound of the following formula

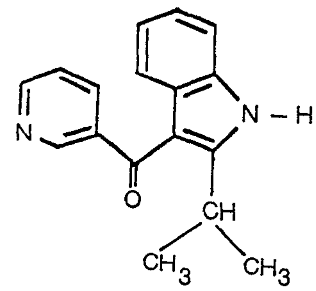

has an action of inhibiting platelet aggregation (see Pharmacological Research Communications, vol. 9, No. 2, 1977).

Investigations of the present inventors have shown that the compound reported by Ryszart J. Grgglewski et al. in which the 3-position of the indole skeleton is substituted by a pyridylcarbonyl group and its 1-position does not have a substituent, certainly has an action of inhibiting platelet aggregation, but on the other hand, has an unnegligible tendency to induce ulcer formation.

US—A—3505354 discloses acylated indole derivatives in which the 1-position of the indole skeleton is substituted by an alkyl group having a terminal carboxyl group, and the $\alpha$-carbon atom of the 1-position substituent is either primary or secondary. However, compounds having a carboxyl terminated substituent group in the 1-position have low activity in the inhibition of platelet aggregation (see Table 1, Example 78, Comparison 1 below).

This invention provides novel acylated indole derivatives wherein the 3-position of the indole skeleton is substituted by a substituted or unsubstituted phenyl or thienyl group and the 1-position is substituted by an alkyl or substituted alkyl group having a secondary $\alpha$-carbon atom.

The invention also provides a pharmaceutical composition having the ability to inhibit platelet aggregation comprising such a novel acylated indole derivative. Preferably the pharmaceutical composition has the ability to inhibit platelet aggregation with a reduced tendency to induce ulcer formation, and even more preferably has the ability to inhibit platelet aggregation with a biologically acceptable low level of various side-effects such as toxicity in single and continual administration, influences on the components which make up the blood, influences on various organs, and tendency toward ulcer formation.

According to this invention there are provided acylated indole derivatives of the general formula

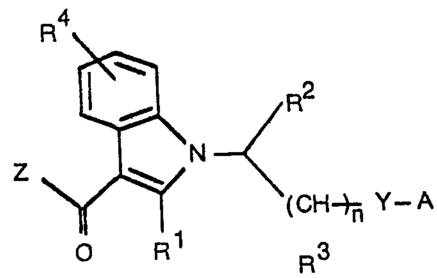

[I]

wherein $R^1$ represents hydrogen or alkyl of 1 to 6 carbon atoms, $R^2$ represents alkyl of 1 to 6 carbon atoms, $R^3$ represents hydrogen or alkyl of 1 to 6 carbon atoms, $R^4$ represents hydrogen, hydroxyl, halogen, alkyl of 1 to 6 carbon atoms, or alkoxy of 1 to 6 carbon atoms, Z represents phenyl or thienyl optionally substituted by the group $R^4$, Y represents a bond, —O—, —S—, —SO—, or —$SO_2$—, A represents hydrogen, alkyl of 1 to 6 carbon atoms, acyl of 1 to 6 carbon atoms, phenyl, phenylalkyl, pyridinyl, imidazolyl or thiadiazolyl, or phenyl, phenylalkyl, pyridinyl, imidazolyl or thiadiazolyl substituted by the group $R^4$, and n represents zero or an integer of 1 to 10 provided that when n is zero, Y represents a bond and A represents acyl of 1 to 6 carbon atoms;

or an optically active isomer thereor or a salt thereof.

The acylated indole derivatives of formula [I] provided by the present invention are structurally characterized by the fact that the 3-position of the indole skeleton is substituted by a substituted or unsubstituted benzoyl or thenoyl group and the 1-position is substituted by an unsubstituted or substituted alkyl group branched at the $\alpha$-carbon atom.

Alkyl groups represented by $R^1$, $R^2$, $R^3$ and $R^4$ have 1 to 6 carbon atoms. These alkyl groups may be linear or branched, and include, for example, methyl, ethyl, n-propyl, isopropyl, n-butyl, iso-butyl, sec-butyl, n-pentyl, and n-hexyl.

The halogen atoms for $R^4$ are fluorine, chlorine, bromine, and iodine.

Alkoxy groups represented by $R^4$ have 1 to 6 carbon atoms, preferably 1 to 4 carbon atoms. Examples include methoxy, ethoxy, n-propoxy, iso-propoxy, n-butoxy, n-pentoxy, and n-hexoxy.

$R^1$ is preferably hydrogen, methyl, ethyl, n-propyl, or iso-propyl, especially methyl.

$R^2$ is preferably an alkyl group having 1 to 3 carbon atoms, i.e. methyl, ethyl, n-propyl or iso-propyl, especially methyl, ethyl or n-propyl.

$R^3$ is preferably hydrogen or methyl, especially hydrogen.

$R^4$ is preferably hydrogen, hydroxyl, chloro, methyl or methoxy.

Z in formula [I] represents a phenyl or thienyl group optionally substituted by $R^4$. As the thienyl or substituted thienyl group, 2-thienyl or substituted 2-thienyl are preferred. As the substituted phenyl or substituted thienyl, mono-substituted phenyl or thienyl is preferred.

Examples of the group Z include phenyl, 2-thienyl, o-fluorophenyl, p-fluorophenyl, o-chlorophenyl, m-chlorophenyl, p-chlorophenyl, o-tolyl, m-tolyl, p-tolyl, o-methoxyphenyl, p-methoxyphenyl, p-hydroxyphenyl, 3-chloro-2-thienyl, 4-fluoro-2-thienyl, 5-methyl-2-thienyl, and 4-methoxy-2-thienyl.

Preferred groups Z are phenyl, 2-thienyl and substituted phenyl. Phenyl and 2-thienyl are especially preferred, and phenyl is most preferred.

In formula [I], Y represents a bond, —O—, —S—, —SO— or —$SO_2$—.

A in formula [I] represents hydrogen atom, a lower alkyl group, lower acyl group, phenyl group, a phenylalkyl group, a 5- or 6-membered heterocyclic group containing an oxygen, sulfur or nitrogen atom, or a phenyl, phenylalkyl or 5- or 6-membered O—, S— or N-containing heterocyclic group substituted by the group $R^4$.

Alkyl groups represented by A in formula [I] are as defined and exemplified hereinabove with regard to $R^1$ to $R^4$.

Acyl groups represented by A have 1 to 6 carbon atoms, preferably 1 to 3 carbon atoms, and include, for example, formyl, acetyl, n-propanoyl, n-butanoyl, iso-butanoyl, n-pentanoyl, and n-hexanoyl.

Examples of the phenylalkyl group are benzyl, $\alpha$-phenethyl, $\beta$-phenethyl, and $\gamma$-phenylpropyl. Benzyl and $\beta$-phenethyl are preferred.

The phenyl, phenylalkyl, pyridinyl, imidazolyl thiadiazolyl group may be substituted by the group $R^4$ defined hereinabove.

In formula [I], n represents zero or an integer of 1 to 10, preferably zero or an integer of 1 to 3, provided that n is zero only when Y represents a bond and A represents acyl.

Examples of the acylated indole derivatives of formula [I] are given below. For convenience, they are classified by the definition of Y in formula [I].

<u>Compounds wherein Y is an oxygen atom</u>

(100) 3-Benzoyl-1-(1-hydroxy-2-propyl)-2-methylindole
(101) 3-Benzoyl-1-(1-hydroxy-2-butyl)-2-methylindole
(102) 3-Benzoyl-1-(1-hydroxy-2-hexyl)-2-methylindole
(103) 3-Benzoyl-1-(1-hydroxy-2-octyl)-2-methylindole
(104) 3-Benzoyl-1-(3-hydroxy-2-butyl)-2-methylindole
(105) 3-Benzoyl-1-(4-hydroxy-2-butyl)-2-methylindole
(106) 3-Benzoyl-1-(1-hydroxy-3-pentyl)-2-methylindole
(107) 3-Benzoyl-1-(6-hydroxy-2-hexyl)-2-methylindole
(108) 3-Benzoyl-1-(7-hydroxy-2-heptyl)-2-methylindole
(109) 3-Benzoyl-1-(9-hydroxy-2-nonyl)-2-methylindole
(110) 3-Benzoyl-1-(11-hydroxy-2-undecyl)-2-methylindole
(111) 3-Benzoyl-1-(1-hydroxy-2-propyl)indole
(112) 3-Benzoyl-1-(4-hydroxy-2-butyl)indole
(113) 3-Benzoyl-5-chloro-1-(1-hydroxy-2-propyl)-2-methylindole
(114) 3-Benzoyl-1-(1-hydroxy-2-propyl)-2,5-dimethylindole
(115) 3-Benzoyl-5-chloro-1-(4-hydroxy-2-butyl)-2-methylindole
(116) 3-Benzoyl-1-(1-hydroxy-2-propyl)-5-methoxy-2-methylindole
(117) 3-Benzoyl-5-hydroxy-1-(1-hydroxy-2-propyl)-2-methylindole
(118) 3-Benzoyl-(4-hydroxy-2-butyl)-5-methoxy-2-methylindole
(119) 3-Benzoyl-1-(1-hydroxy-2-propyl)-2-isopropylindole
(120) 3-Benzoyl-1-(6-hydroxy-2-hexyl)-2-isopropylindole
(121) 3-Benzoyl-5-chloro-1-(1-hydroxy-2-propyl)indole
(122) 3-Benzoyl-1-(4-hydroxy-2-butyl)-2-isopropyl-5-methylindole
(123) 3-(p-Chlorobenzoyl)-1-(1-hydroxy-2-propyl)-2-methylindole
(124) 3-(o-Chlorobenzoyl)-1-(4-hydroxy-2-butyl)-2-methylindole
(125) 3-(o-Fluorobenzoyl)-1-(4-hydroxy-2-butyl)-2-methylindole
(126) 1-(1-Hydroxy-2-propyl)-2-methyl-3-(o-tolyl)indole
(127) 1-(1-Hydroxy-2-propyl)-2-methyl-3-(2-thenoyl)indole
(128) 5-Chloro-1-(1-hydroxy-2-propyl)-2-methyl-3-(2-thenoyl)indole
(129) 3-(4-Chloro-2-thenoyl)-1-(1-hydroxy-2-propyl)-2-methylindole
(130) 5-Chloro-3-(p-fluorobenzoyl)-1-(1-hydroxy-2-propyl)-2-methylindole
(131) 3-Benzoyl-5-bromo-1-(1-hydroxy-2-propyl)-2-methylindole
(132) 3-Benzoyl-5-fluoro-1-(4-hydroxy-2-butyl)-2-methylindole
(133) 1-(4-Hydroxy-2-butyl)-2-methyl-3-(m-tolylcarbonyl)indole
(134) 3-(m-Fluorobenzoyl)-1-(1-hydroxy-2-propyl)-2-methylindole
(135) 3-Benzoyl-1-[1-hydroxy-2(R)-propyl]-2-methylindole
(136) 3-Benzoyl-1-[1-hydroxy-2(S)-propyl]-2-methylindole
(137) 3-Benzoyl-1-(4-hydroxy-2-butyl)-2,5-dimethylindole
(200) 1-(1-Acetoxy-2-propyl)-3-benzoyl-2-methylindole
(201) 1-(4-Acetoxy-2-butyl)-3-benzoyl-2-methylindole
(202) 1-(1-Acetoxy-2-propyl)-3-benzoylindole
(203) 1-(4-Acetoxy-2-butyl)-3-benzoyl-2-isopropylindole
(204) 1-(1-Acetoxy-3-pentyl)-3-benzoyl-2-methylindole
(205) 1-(1-Acetoxy-2-(butyl)-3-benzyl-2-methylindole
(206) 1-(1-Acetoxy-2-hexyll-3-benzoyl-2-methylindole

(207) 1-(1-Acetoxy-2-octyl)-3-benzoyl-2-methylindole
(208) 1-(1-Acetoxy-2-propyl)-2-methyl-3-thenoylindole
(209) 3-Benzoyl-1-(1-formyloxy-2-propyl)-2-methylindole
(210) 3-Benzoyl-2-methyl-1-(4-propanoyl-2-butyl)indole
(211) 1-(1-Acetoxy-2-propyl)-3-benzoyl-5-chloro-2-methylindole
(212) 1-(4-Acetoxy-2-butyl)-3-benzoyl-2,5-dimethylindole
(213) 1-(1-Acetoxy-2-propyl)-5-chloro-2-methyl-3-(p-tolylcarbonyl)indole
(214) 1-(4-Acetoxy-2-butyl)-3-benzoyl-5-methoxy-2-methylindole
(215) 1-(1-Acetoxy-2-propyl)-3-benzoyl-5-methoxy-2-methylindole
(216) 1-(1-Acetoxy-2-propyl)-3-(p-chlorobenzoyl)-5-methoxy-2-methylindole
(217) 1-[1-Acetoxy-2-(R)-propyl]-3-benzoyl-2-methylindole
(218) 1-[1-Acetoxy-2(S)-propyl]-3-benzoyl-2-methylindole
(219) 1-(1-Acetoxy-2-propyl)-3-benzoyl-5-methoxy-2-methylindole
(300) 3-Benzoyl-1-(1-methoxy-2-propyl)-2-methylindole
(301) 3-Benzoyl-1-(1-methoxy-2-propyl)indole
(302) 3-Benzoyl-1-(1-ethoxy-2-propyl)-2-methylindole
(303) 3-Benzoyl-1-(1-methoxy-2-propyl)-2-isopropylindole
(304) 3-Benzoyl-5-chlor-1-(1-methoxy-2-propyl)-2-methylindole
(305) 3-Benzoyl-1-(1-isopropoxy-2-propyl)-2-methylindole
(306) 3-Benzoyl-1-(4-methoxy-2-butyl)-2-methylindole
(307) 3-Benzoyl-1-(4-ethoxy-2-butyl)-2-methylindole
(308) 3-Benzoyl-1-(4-ethoxy-2-butyl)-5-methoxy-2-methylindole
(309) 1-(4-Ethoxy-2-butyl)-2-methyl-3-(p-tolylcarbonyl)indole
(310) 3-Benzoyl-1-(4-methoxy-2-butyl-2,5-dimethyindole
(311) 3-Benzoyl-1-(1-methoxy-2-butyl)-2-methylindole
(312) 3-Benzoyl-1-(1-ethoxy-2-butyl)-2-methylindole
(313) 3-Benzoyl-1-(1-benzyloxy-2-butyl)-2-methylindole
(314) 3-Benzoyl-5-methoxy-1-(1-methoxy-2-butyl)-2-methylindole
(315) 1-(1-Methoxy-2-propyl)-2-methyl-3-thenoylindole
(316) 3-(p-Chlorobenzoyl)-1-(1-methoxy-2-butyl)-2-methylindole
(317) 3-(p-Chlorobenzoyl)-1-(1-methoxy-2-propyl)-2-methylindole
(318) 3-Benzoyl-5-methoxy-1-(1-methoxy-2-propyl)-2-methylindole
(319) 3-Benzoyl-1-(1-methoxy-3-pentyl)-2-methylindole
(320) 3-Benzoyl-1-(1-ethoxy-3-pentyl)-2-methylindole
(321) 3-Benzoyl-5-chloro-1-(1-methoxy-3-pentyl)-2-methylindole
(322) 3-Benzoyl-2-methyl-1-(1-$\alpha$-phenethyl-2-pentyl)indole
(323) 3-Benzoyl-1-(1-ethoxy-2-pentyl)-5-hydroxy-2-methylindole
(324) 3-Benzoyl-1-(1-butoxy-2-pentyl)-2-methylindole
(325) 3-Benzoyl-1-(1-heptyloxy-2-propyl)-2-methylindole
(326) 3-Benzoyl-1-(4-hexyloxy-2-butyl)-2-methylindole
(327) 3-Benzoyl-1-(7-methoxy-2-heptyl)-2-methylindole
(328) 3-Benzoyl-1-(7-ethoxy-2-heptyl)-2-methylindole
(329) 3-Benzoyl-1-(11-methoxy-2-undecyl)-2-methylindole
(330) 3-Benzoyl-1-(11-ethoxy-2-undecyl)-2-methylindole
(331) 3-Benzoyl-2-methyl-1-(7-propoxy-2-heptyl)indole
(332) 1-(11-Benzyloxy-2-undecyl)-3-benzoyl-2-methylindole
(333) 3-Benzoyl-1-(1-ethoxy-2-hexyl)-2-methylindole
(334) 3-Benzoyl-1-(1-methoxy-2-hexyl)-2-methylindole
(335) 3-Benzoyl-1-(1-methoxy-2-octyl)-2-methylindole
(336) 3-Benzoyl-1-(1-ethoxy-2-octyl)-2-methylindole
(337) 3-Benzoyl-2-methyl-1-(1-propoxy-2-octyl)indole
(338) 3-Benzoyl-5-chloro-1-(1-methoxy-2-hexyl)-2-methylindole
(339) 3-Benzoyl-1-(1-methoxy-2-propyl)-2,5-dimethylindole
(340) 3-(Benzoyl-1-(4-ethoxy-2-butyl)-2,5-dimethylindole
(341) 3-Benzoyl-5-bromo-1-(1-ethoxy-2-propyl)-2-methylindole
(342) 3-(p-Methoxybenzoyl)-1-(1-methoxy-2-propyl)-2-methylindole
(343) 3-Benzoyl-1-[1-(o-chlorobenzyloxy)-2-propyl]-2-methylindole
(344) 3-Benzoyl-1-(4-ethoxy-2-butyl)-5-fluoro-2-methylindole
(345) 3-(o-Chlorobenzoyl)-1-(4-methoxy-2-butyl)-2-methylindole
(346) 3-Benzoyl-1-[1-methoxy-2(R)-propyl]-2-methylindole
(347) 3-Benzoyl-1-[1-methoxy-2(S)-propyl]-2-methylindole
(348) 3-Benzoyl-2-methyl-1-(1-phenoxy-2-propyl)indole
(349) 3-Benzoyl-2-methyl-1-(4-phenoxy-2-butyl)indole
(350) 3-Benzoyl-1-[1-(p-chlorophenoxy)-2-butyl]-2-methylindole
(351) 3-Benzoyl-1-[1-(p-methoxyphenoxy)-2-propyl]-2-methylindole

(352) 3-Benzoyl-5-methoxy-1-[1-(p-methoxyphenoxy)-2-propyl]-2-methylindole

Compounds wherein Y is —S—, —SO— or —$SO_2$—

(400) 3-Benzoyl-1-(1-ethylthio-2-propyl)-2-methylindole
(401) 3-Benzoyl-2-methyl-1-(1-phenylthio-2-propyl)indole
(402) 3-Benzoyl-2-methyl-1-(1-methylthio-2-propyl)indole
(403) 3-Benzoyl-1-(1-isopropylthio-2-propyl)-2-methylindole
(404) 3-Benzoyl-2-methyl-1-[1-(3-pyridylthio)-2-propyl]indole
(405) 3-Benzoyl-2-methyl-1-[1-(2-pyridylthio)-2-propyl]indole
(406) 1-(1-Benzylthio-2-propyl)-3-benzoyl-2-methylindole
(407) 3-Benzoyl-2-methyl-1-[1-(5-methyl-1,3,5-thiadiazol-2-ylthio)propyl]indole
(408) 3-Benzoyl-1-[1-(2-imidazolylthio)propyl]-2-methylindole
(409) 3-Benzoyl-1-(4-ethylthio-2-butyl)-2-methylindole
(410) 3-Benzoyl-2-methyl-1-(4-phenylthio-2-butyl)-indole
(411) 3-Benzoyl-2-methyl-1-(4-methylthio-2-butyl)-indole
(412) 3-Benzoyl-1-(4-butylthio-2-butyl)-2-methylindole
(413) 3-Benzoyl-2-methyl-1-[4-(4-pyridylthio)-2-butyl]indole
(414) 3-Benzoyl-2-methy-1-[4-(2-pyridylthio)-2-butyl]indole
(415) 3-Benzoyl-2-methyl-2-methyl-1-[4-($\alpha$-phenethylthio)-2-butyl]-indole
(416) 1-(4-Acetylthio-2-butyl)-3-benzoyl-2-methylindole
(417) 3-Benzoyl-1-(1-ethylthio-2-butyl)-2-methylindole
(418) 3-Benzoyl-2-methyl-1-(phenylthio-2-butyl)indole
(419) 3-Benzoyl-2-methyl-1-(1-methylthio-2-butyl)indole
(420) 3-Benzoyl-2-methyl-1-[1-(4-pyridylthio)-2-butyl]indole
(421) 3-Benzoyl-2-methyl-1-[1-(2-pyridylthio)-2-butyl]indole
(422) 3-Benzoyl-1-(1-mercapto-2-propyl)-2-methylindole
(423) 3-Benzoyl-1-(4-mercapto-2-butyl)-2-methylindole
(424) 3-Benzoyl-1-(1-mercapto-2-butyl)-2-methylindole
(425) 3-Benzoyl-1-(1-ethylthio-3-pentyl)-2-methylindole
(426) 3-Benzoyl-2-methyl-1-(7-phenylthio-2-heptyl)indole
(427) 3-Benzoyl-5-chloro-2-methyl-1-(1-methylthio-2-propyl)indole
(428) 3-Benzoyl-1-(4-ethylthio-2-butyl)-5-methoxy-2-methylindole
(500) 3-Benzoyl-1-(1-ethanesulfinyl-2-propyl)-2-methylindole
(501) 1-(1-Benzenesulfinyl-2-propyl)-3-benzoyl-2-methylindole
(502) 3-Benzoyl-1-(1-methanesulfinyl-2-propyl)-2-methylindole
(503) 3-Benzoyl-2-methyl-1-[1-(4-pyridinesulfinyl)-2-propyl]indole
(504) 3-Benzoyl-2-methyl-1-[1-(2-pyridinesulfinyl)-2-propyl]indole
(505) 3-Benzoyl-1-(4-ethanesulfinyl-2-butyl)-2-methylindole
(506) 3-Benzoyl-1-(4-methanesulfinyl-2-butyl)-2-methylindole
(507) 1-(4-Benzenesulfinyl-2-butyl)-3-benzoyl-2-methylindole
(508) 3-Benzoyl-2-methyl-1-[4-(4-pyridinesulfinyl)-2-butyl]indole
(509) 3-Benzoyl-2-methyl-1-[4-(2-pyridinesulfinyl)-2-butyl]indole
(510) 3-Benzoyl-1-(1-ethanesulfinyl-2-butyl)-2-methylindole
(511) 1-(1-Benzenesulfinyl-2-butyl)-3-benzoyl-2-methylindole
(512) 3-Benzoyl-2-methyl-1-[1-(4-pyridinesulfinyl)-2-butyl]indole
(513) 3-Benzoyl-2-methyl-1-[1-(2-pyridinesulfonyl)-2-butyl]indole
(514) 3-Benzoyl-1-(1-methanesulfinyl-2-butyl)-2-methylindole
(600) 3-Benzoyl-1-(1-ethanesulfonyl-2-propyl)-2-methylindole
(601) 1-(1-Benzenesulfonyl-2-propyl)-3-benzoyl-2-methylindole
(602) 3-Benzoyl-1-(1-ethanesulfonyl-2-propyl)-2-methylindole
(603) 3-Benzoyl-2-methyl-1-[1-(4-pyridinesulfonyl)-2-propyl]indole
(604) 3-Benzoyl-2-methyl-1-[1-(2-pyridinesulfonyl)-2-propyl]indole
(605) 3-Benzoyl-1-(4-ethanesulfonyl-2-butyl)-2-methylindole
(606) 3-Benzoyl-1-(4-methanesulfonyl-2-butyl)-2-methylindole
(607) 1-(4-Benzenesulfonyl-2-butyl)-3-benzoyl-2-methylindole
(608) 3-Benzoyl-2-methyl-1-[4-(4-pyridinesulfonyl)-2-butyl]indole
(609) 3-Benzoyl-2-methyl-1-[4-(2-pyridinesulfonyl)-2-butyl]indole
(610) 3-Benzoyl-1-(1-ethanesulfonyl-2-butyl)-2-methylindole
(611) 1-(1-Benzenesulfonyl-2-butyl)-3-benzoyl-2-methylindole
(612) 3-Benzoyl-2-methyl-1-[1-(4-pyridinesulfonyl)-2-butyl]indole
(613) 3-Benzoyl-2-methyl-1-[1-(2-pyridinesulfonyl)-2-butyl]indole
(614) 3-Benzoyl-1-(1-methanesulfonyl-2-butyl)-2-methylindole

Compounds wherein Y represents a bond

(700) 3-Benzoyl-1-isopropyl-2-methylindole
(701) 3-Benzoyl-1-(2-butyl)-2-methylindole
(702) 3-Benzoyl-2-methyl-1-(1-methyl-2-propyl)indole
(703) 3-Benzoyl-2-methyl-1-(2-pentyl)indole
(704) 3-Benzoyl-2-methyl-1-(3-pentyl)indole
(705) 3-Benzoyl-1-(2-hexyl)-2-methylindole
(706) 3-Benzoyl-1-(2-heptyl)-2-methylindole
(707) 3-Benzoyl-2-methyl-1-(2-octyl)indole
(708) 3-Benzoyl-2-methyl-1-(2-undecyl)indole
(709) 3-Benzoy-1-($\alpha$-phenethyl)-2-methylindole
(800) 3-Benzoyl-2-methyl-1-(3-oxo-2-butyl)indole
(801) 3-Benzoyl-2-methyl-2-(4-oxo-2-pentyl)indole
(802) 3-Benzoyl-2-methyl-1-(3-oxo-2-pentyl)indole
(804) 3-Benzoyl-2-methyl-1-(5-oxo-3-hexyl)indole
(805) 3-Benzoyl-1-(1-formyl-1-ethyl)-2-methylindole
(806) 3-Benzoyl-1-(1-formyl-2-propyl)-2-methylindole
(807) 3-Benzoyl-1-(1-formylpropyl)-2-methylindole
(808) 3-Benzoyl-1-(1-formyl-2-butyl)-2-methylindole

Of the 3-acylated indole derivatives of formula [I], provided by this invention, those of the following formula

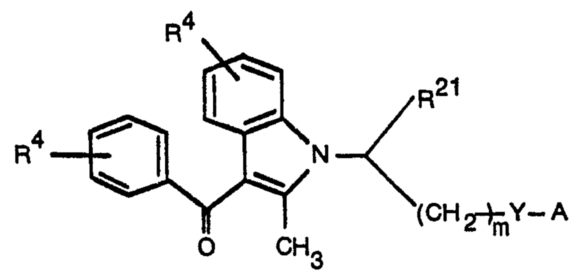

[I]-1

wherein $R^{21}$ represents an alkyl group having 1 to 3 carbon atoms, two groups $R^4$ are identical or different and each represents hydrogen, hydroxyl, halogen, alkyl or alkoxy, Y represents a bond, —O—, —S—, —SO— or —$SO_2$—, A represents hydrogen, alkyl, acyl, phenyl, phenylalkyl, pyridinyl, imidazolyl or thiadiazolyl, or phenyl, phenylalkyl, pyridinyl, imidazolyl or thiadiazolyl substituted by the group $R^4$, and m is zero or an integer of 1 to 3 provided that when m is zero, Y represents a bond and A represents acyl,

and the optically active isomers or salts thereof; especially those of the following formula

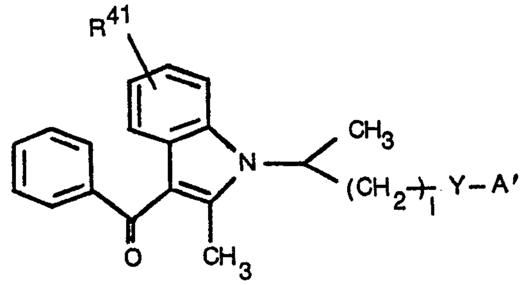

[2]-2

wherein $R^{41}$ represents a hydrogen atom, a hydroxyl group, a chlorine atom, a methyl group or a methoxy group, Y is as defined with regard to formula [I]—1, $A^1$ represents a hydrogen atom, an acyl group having 1 to 4 carbon atoms, an acyl group having 1 to 4 carbon atoms, a phenyl group, pyridinyl imidazolyl or thiadiazolyl, and l is 0, 1 or 2 provided that when l is 0, Y represents a bond and A′ represents acyl,

and the optically active isomers or salts thereof have biological activities, particularly the ability to inhibit platelet aggregation, and therefore, are provided by this invention as preferred compounds.

In the acylated indole derivatives of formula [I] including formulae [I]—1 and [I]—2, the substituent at the 1-position is branched at the $\alpha$-position, and therefore when the group $R^2$ (or $R^{21}$ or $CH^3$) differs from the group

$$-\!(\underset{\substack{|\\R^3}}{CH})_{\overline{n}}\!-Y-A \text{ [or } -\!(CH_2)_{\overline{m}}\!-Y-A \quad\text{or}\quad -\!(CH_2)_{\overline{l}}\!-Y-A^1],$$

the carbon atom at the $\alpha$-position becomes an asymmetric carbon atom. Accordingly, the above formula [I] also encompasses optically active isomers with respect to the carbon atom at the $\alpha$-position.

The acylated indole derivatives of formula [I] including formulae [I]—1 and [I]—2 can form salts with basic compounds when the substituent $R^4$ is a hydroxyl group, and can form acid addition salts with acids when the substituent A is a nitrogen-containing heterocyclic group such as pyridyl.

Examples of such basic compounds include hydroxides, carbonates and hydrogen carbonates of alkali metals such as lithium, sodium and potassium or alkaline earth metals such as calcium and magnesium. They also include ammonia, primary, secondary or tertiary amines such as methylamine, ethylamine, butylamine, dimethylamine, diethylamine, cyclohexylmethylamine, triethylamine and pyridine, and quaternary ammonium hydroxides such as tetramethyl ammonium hydroxide.

Examples of the acids include mineral acids such as hydrochloric acid, hydrobromic acid, sulfuric acid and phosphoric acid, organic carboxylic acids such as acetic acid, propionic acid, oxalic acid, mandelic acid, maleic acid, fumaric acid, lactic acid and glutamic acid, and organic sulfonic acids such as methanesulfonic acid, ethanesulfonic acid, benzenesulfonic acid, p-toluenesulfonic acid, and cumylsulfonic acid.

According to this invention, compounds of formula [I] can be produced in the following manner.

[A] Compounds of formula [I] wherein Y is an oxygen atom:—

(a) Compounds of general formula [I] wherein Y is an oxygen atom and A is a hydrogen atom or acyl can be produced in accordance with Reaction Schemes 1 to 4 below.

(b) Compounds of general formula [I] wherein Y is an oxygen atom, A is alkyl, a phenylalkyl group or a phenylalkyl group substituted by $R^4$ can be produced in accordance with Reaction Schemes 5 to 8 below.

(c) Compounds of general formula [I] wherein Y is an oxygen atom, and A is phenyl, phenyl substituted by $R^4$, or pyridinyl, imidazolyl or thiadiazolyl optionally substituted by $R^4$ can be produced in accordance with Reaction Scheme 9 below.

These reactions are described below.

(a) Production of compounds of general formula [I] wherein Y is an oxygen atom and A is a hydrogen atom or acyl group in accordance with Reaction Schemes 1 to 4:—

(1)

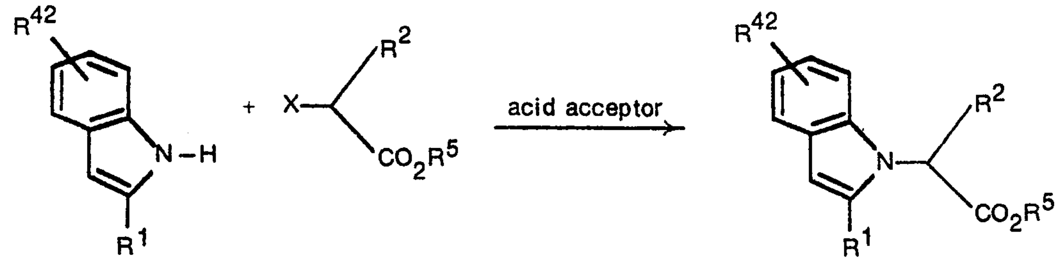


10 11 12

12

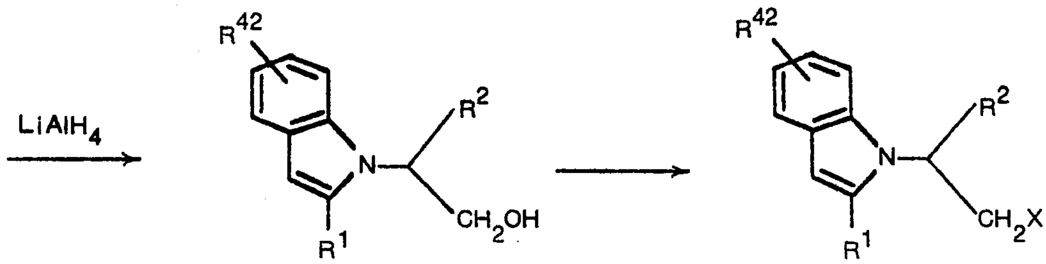


13 14

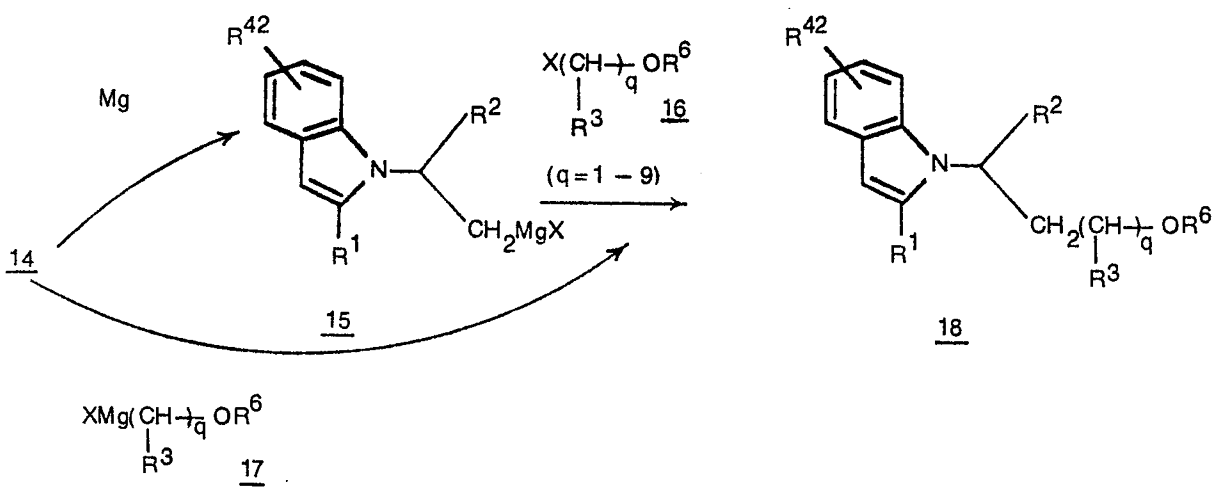


14 15 16 17 18

18

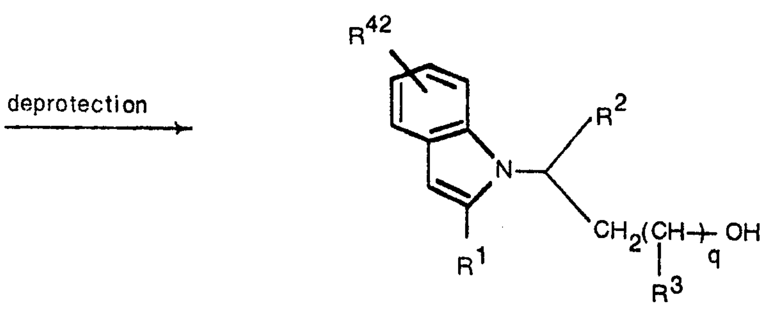


19

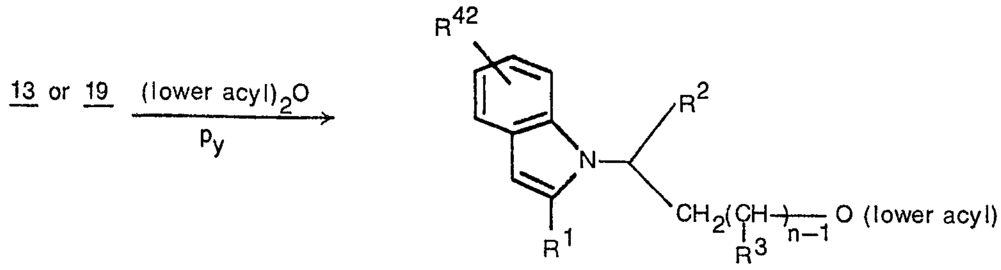


20 (n = 1 – 10)

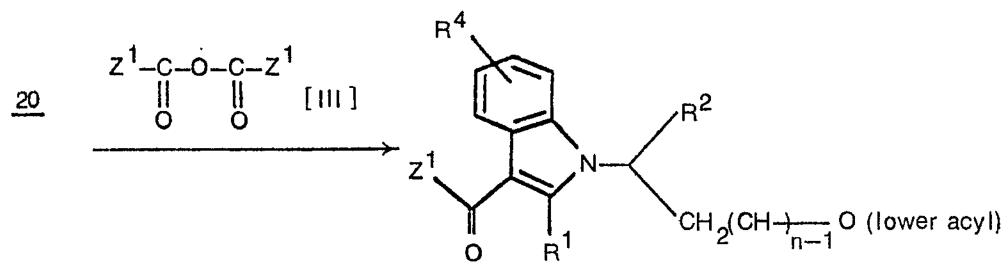


21

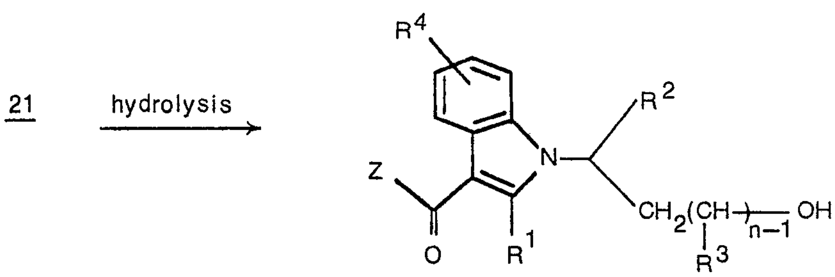


22

(2) REACTION SCHEME 1

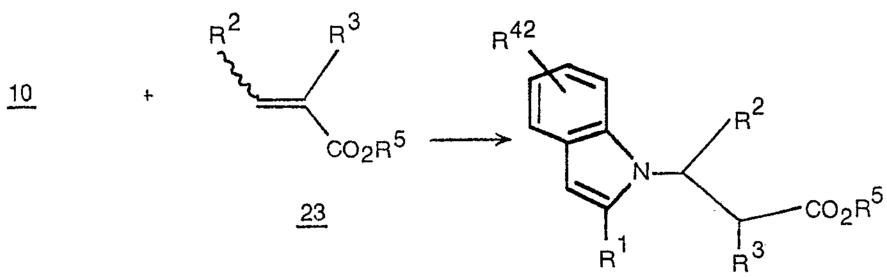


24

24
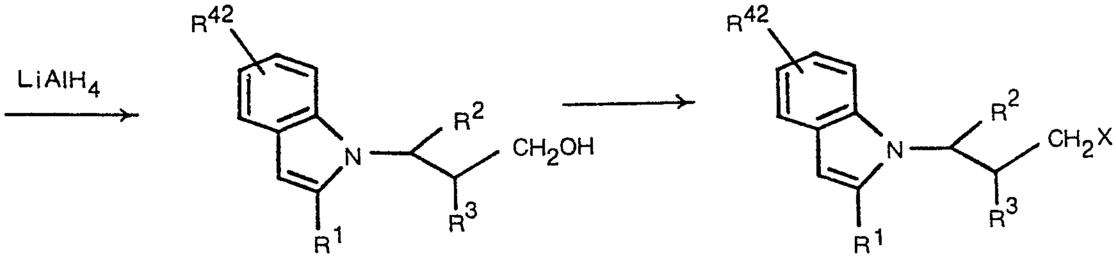


25

26

26
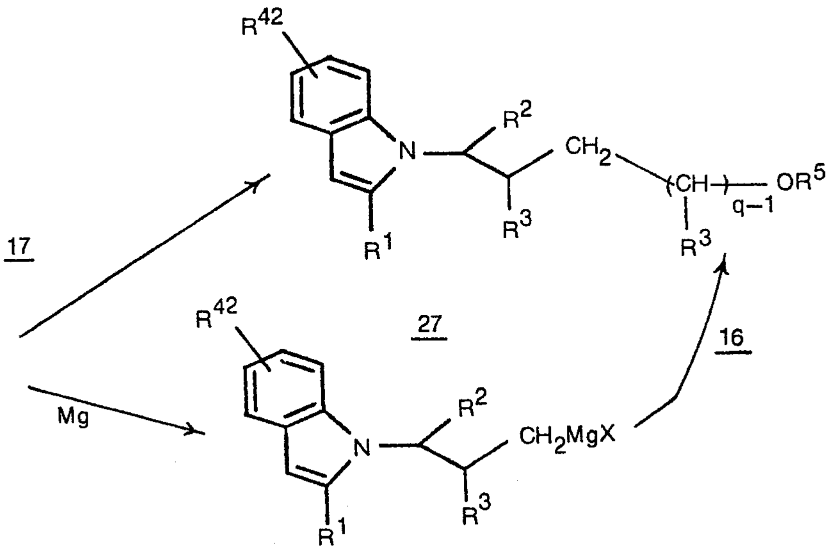


27

28

27
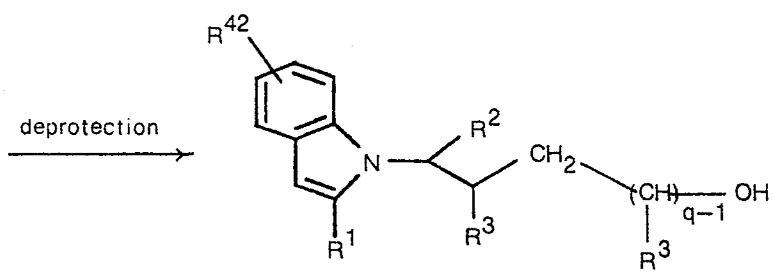


29

25 or 29
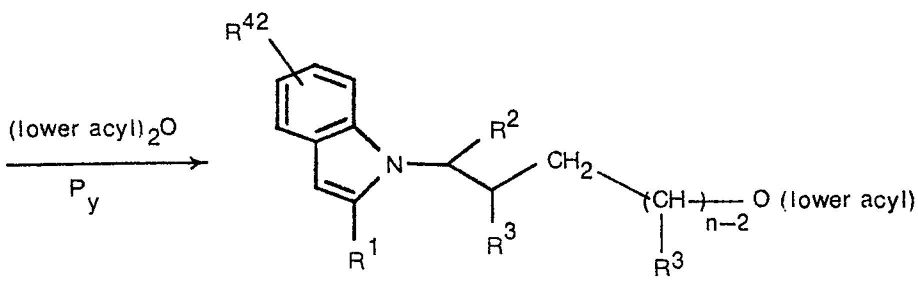


30

30

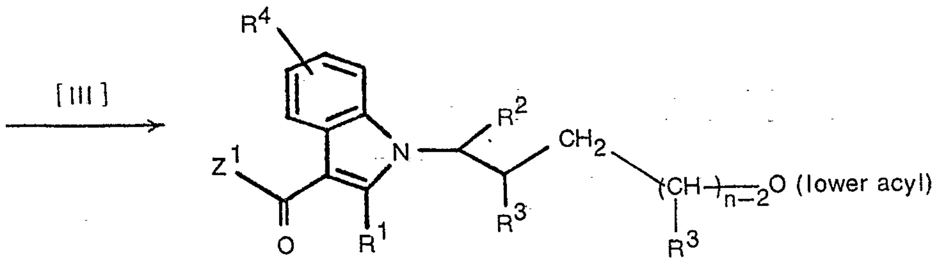


31

31

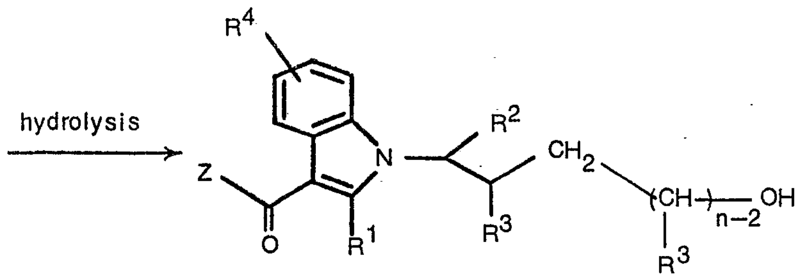


32

REACTION SCHEME 2

(3)

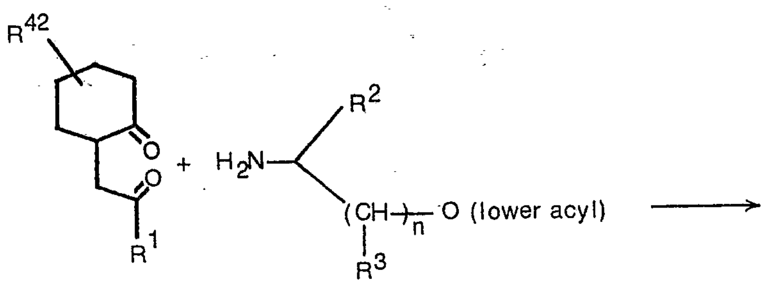

33 34

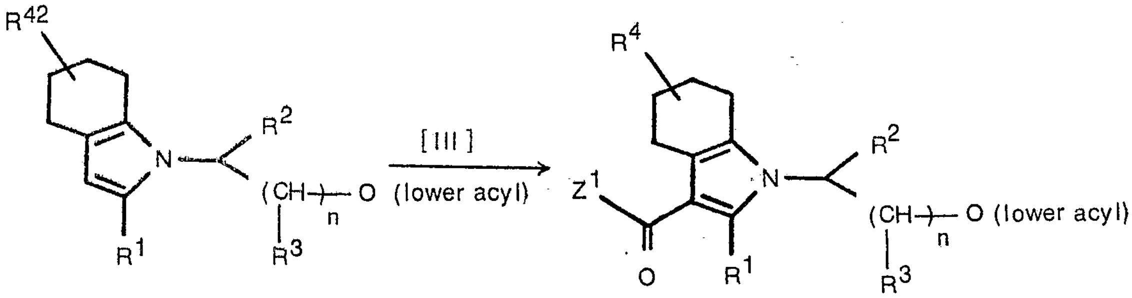


35 36

36 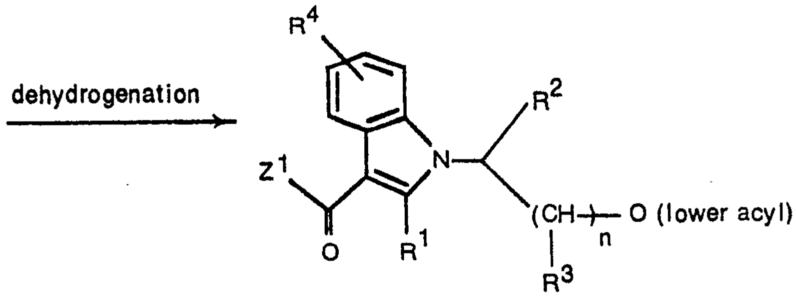


37

37 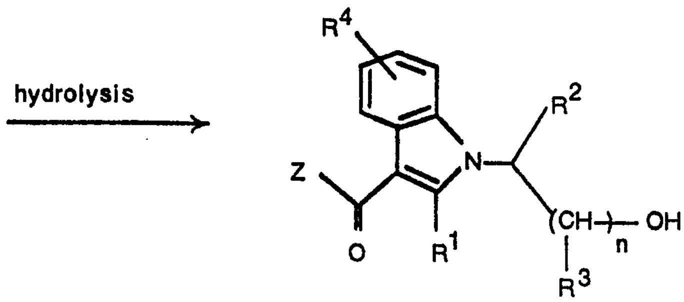


38

## REACTION SCHEME 3

(4)

33 + 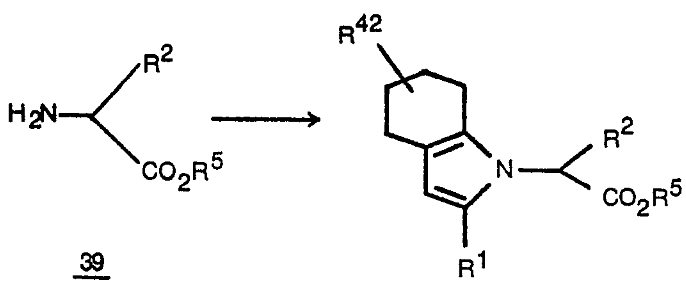

39

40

40 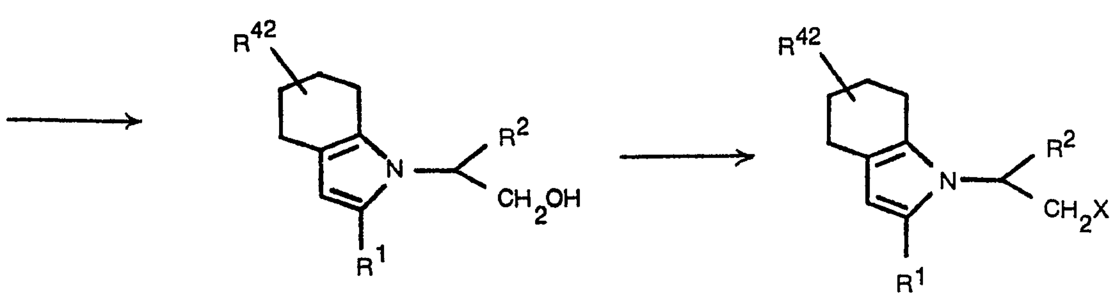

41

42

42

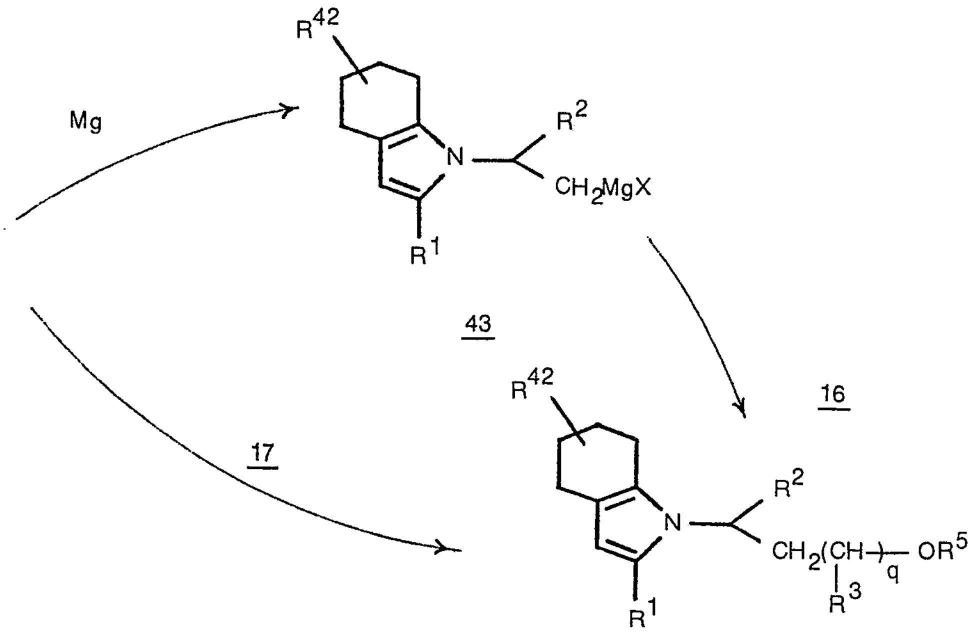


43

16

44

44

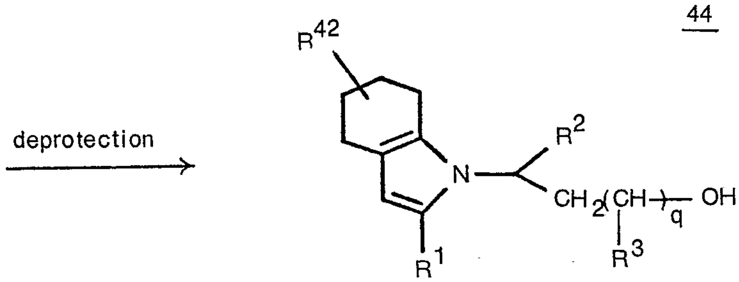


45

41 or 45

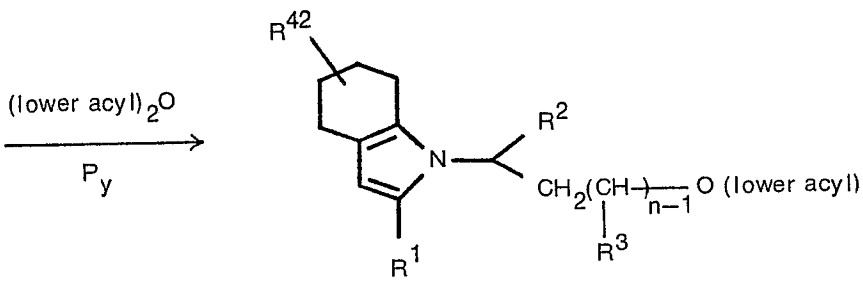


46

46

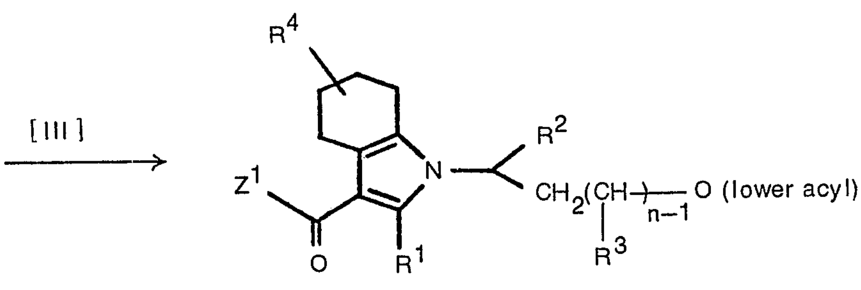


47

47

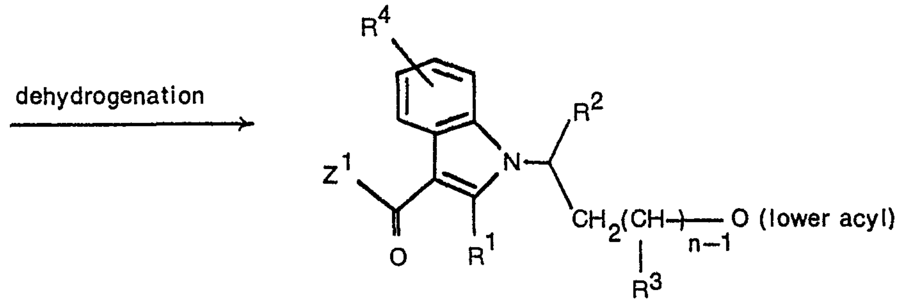


21

48

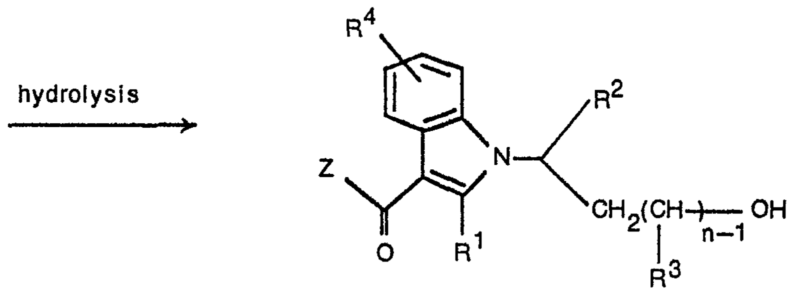


22

REACTION SCHEME 4

As shown in the above Reaction Schemes 1 to 4, the compounds of general formula [I] wherein Y is an oxygen atom and A is acyl (*21, 31 35, 46*) can be produced by condensing the corresponding 1-($\omega$-acyloxyalkyl)-3-unsubstituted indoles (*20, 30*) with acid anhydrides of formula [III] in the presence of hydrogen iodine (Reaction Schemes 1 and 2); or condensing the corresponding 1-($\omega$-acyloxyalkyl)-3-unsubstituted tetrahydroindoles (*35, 46*) with the acid anhydrides of formula [III] in the presence of hydrogen iodide, and then dehydrogenating the condensation product (Reaction Schemes 3 and 4).

The compounds of general formula [I] wherein Y is an oxygen atom and A is a hydrogen atom (*22, 32, 36, 47*) are produced by hydrolyzing the compounds (*21, 31, 35, 46*) produced by the aforesaid acylation reaction using the acid anhydrides.

The acylation reaction with the acid anhydrides of formula [III] constitutes one aspect of the process of this invention.

(a)-1 Reaction Scheme 1

According to Reaction Scheme 1, the acylation reaction using acid anhydrides is carried out by condensing a 1-($\omega$-acyloxyalkyl)-3-unsubstituted indole of the following formula *20*

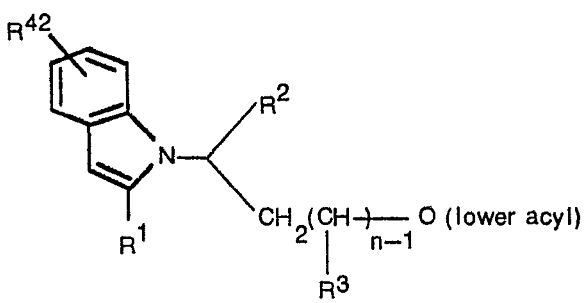

wherein $R^1$ represents a hydrogen atom or alkyl group, $R^2$ represents alkyl, $R^3$ represents a hydrogen atom or alkyl group, $R^{42}$ represents a hydrogen atom, a halogen atom, alkyl group or alkoxy group, and n is an integer of 1 to 10,

with an acid anhydride of the following formula [III]

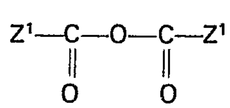

(III)

wherein $Z^1$ represents a phenyl or thienyl group or a phenyl or thienyl group, substituted by $R^{42}$ or a protected hydroxyl group,

in the presence of hydrogen iodide to give a 3-acyl-1-(ω-lower acyloxyalkyl)-indole of the following formula *21*

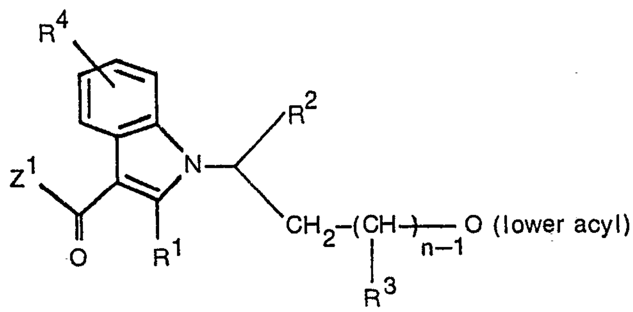

wherein $R^1$, $R^2$, $R^3$, n and $Z^1$ are as defined above, and $R^4$ represents a hydrogen atom, a hydroxy group, a halogen atom, alkyl group or alkoxy group.

Usually, the condensation reaction is carried out by heating 1 mole of the 1-(ω-acyloxyalkyl)-3-unsubstituted indole of formula *20* and 0.5 to 10 moles, preferably 1 to 5 moles, of the acid anhydride of formula [III] in the presence or absence of an inert organic solvent in the presence of 0.001 to 2 moles, preferably 0.01 to 0.5 mole, of hydrogen iodide at a temperature of 50 to 180°C, preferably 80°C to 150°C.

When the starting compounds are melted at the reaction temperature, the inert organic solvent is generally not absolutely required. From the standpoint of after-treatment, such a solvent should better be not used in such a case. Suitable solvents are aprotic inert organic solvents, for example haloalkanes such as carbon tetrachloride, chloroform, and tetrachloroethane, and aromatic hydrocarbon solvents such as nitrobenzene, chlorobenzene, toluene and xylene.

The reaction can usually be monitored by thin-layer chromatography. The reaction is completed usually in 10 minutes to 24 hours. In most cases, it comes to an end in about 20 minutes to about 3 hours.

According to the acylation reaction, the 3-position of the indole skeleton of the compound of formula *20* is acylated, and when the group $R^{42}$ in the starting compound of formula *20* is alkoxy, a reaction may sometimes take place which induces the conversion of the group $R^{42}$ into a hydroxyl group by the effect of hydrogen iodide.

The final desired product can be isolated from the resulting reaction mixture and purified usually by dissolving it in a water-insoluble, good solvent for the final product, such as ethyl acetate, ether, chloroform, methylene chloride or benzene, then washing the solution successively with water, a saturated aqueous solution of sodium bicarbonate, water, and a saturated aqueous solution of sodium chloride, drying the washed product over anhydrous sodium sulfate, concentrating the product and chromatographing the resulting crude product on a column of silica gel carrier. Thus, the 3-acyl-1-(ω-lower acyloxyalkyl)indole of formula *21* is isolated in pure form.

According to Reaction Scheme 1, the 3-acyl-1-(ω-acyloxyalkyl)indole of formula *21* obtained by the above-described acylation reaction may be hydrolyzed to give a 3-acyl-1-(ω-hydroxyalkyl)indole of the following formula *22*

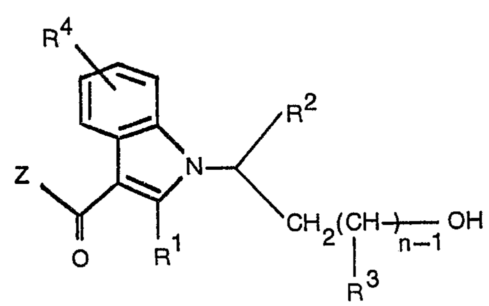

(22)

wherein $R^1$, $R^2$, $R^3$, $R^4$ and n are as defined above and Z represents phenyl or thienyl optionally substituted by $R^4$.

This hydrolysis reaction can be applied to the reaction mixture obtained by the above acylation reaction, the crude product obtained by treating it, or to the final purified product.

When it is to be applied to the reaction mixture, the reaction mixture is dissolved in an organic solvent such as ethanol, methanol, tetrahydrofuran or dioxane (when the reaction mixture contains a reaction solvent, the solvent is distilled off as much as possible beforehand). Then, an aqueous alkali

solution such an aqueous solution or sodium hydroxide or potassium hydroxide is added in an amount slightly excessive with respect to a protective group to be removed by hydrolysis, the remaining acid anhydride and a carboxylic acid formed as a by-product in the reaction. The mixture then stirred at room temperature or optionally at an elevated temperature for 1 to several hours.

After performing the hydrolysis in the above manner, the solvent in the reaction system is distilled off, and the residue is subjected to the same isolation and purifying procedure as described above for the 3-acyl-1-($\omega$-acyloxyalkyl)indole to obtain the 3-acyl-1-($\omega$-hydroxyalkyl)indole of formula *22* in pure form.

When the hydrolysis reaction is to be applied to the crude product or purified product, the operation of removing the reaction solvent is omitted, and otherwise, the same operation as above can be performed.

According to the above hydrolysis reaction, the acyl group in the $\omega$-acyloxyalkyl group is removed, and when the substituent which may be at $Z^1$ is a protected hydroxyl group, i.e. an acyloxy group such as acetyloxy, formyloxy or benzoyloxy, this acyl group is usually removed together. Accordingly, the compound of formula *22* is prepared from the compound of formula *21*.

Thus, according to Reaction Scheme 1, 3-acylated indole derivatives of formulae *21* and *22* which fall within the formula [I] can be obtained which are structurally characterized by the fact that a carbon atom directly bonded to the $\alpha$-carbon atom in the substituent at the 1-position, i.e. the $\beta$-carbon atom, forms a methylene group ($-CH_2-$) having two hydrogen atoms.

The 1-($\omega$-acyloxyalkyl)-3-unsubstituted indole (*20*) used in the acylation reaction in Reaction Scheme 1 can be produced in the following manner.

A 1-(alkoxycarbonylmethyl)indole of formula *12* is prepared by reacting an indole *10* (commercially available, or can be prepared by the Fisher's indole synthesizing method; in formula *10*, $R^1$ and $R^{42}$ are as defined hereinabove) with an $\alpha$-halocarboxylic acid ester *11* (wherein X represents a halogen atom such as chlorine, bromine or iodine, $R^5$ represents a lower alkyl group having 1 to 4 carbon atoms, and $R^2$ is as defined hereinabove) in the presence of an acid acceptor such as sodium hydride or potassium hydride in an inert organic solvent at a temperature between −20°C and the refluxing temperature of the reaction system. Examples of suitable inert organic solvents used in the above condensation reaction are benzene, toluene, xylene, ethylene glycol dimethyl ether, diether, dioxane, tetrahydrofuran (THF), dimethylformamide (DMF), dimethylsulfoxide (DMSO), and hexamethylphosphoramide (HMPA).

The resulting compound of formula *12* is then reduced with lithium aluminum hydride in an inert solvent to form a 1-(hydroxyethyl)-indole. Diethyl ether and THF are preferred as the inert solvent. The reaction is carried out for 10 minutes to several hours at a temperature between −20°C and the refluxing temperature of the reaction system.

The resulting compound of formula *13* is directly reacted with a halogenating agent such as phosphorus tribromide, thionyl chloride, thionyl bromide or hydrobromic acid, or with carbon tetrabromide in the presence of triphenylphosphine to produce a 1-(haloethyl)indole of formula *14*. Each of these reactions is carried out in an inert organic solvent such as diethyl ether, THF, benzene, dichloromethane or chlorform, and is terminated in 30 minutes to 24 hours by performing it at the same temperature as used in the aforesaid reducing reaction.

The resulting compound of formula *14* is then converted to a Grignard reagent, and then reacted with a protected derivative of an $\omega$-hydroxyalkyl halide of formula *16* (wherein $R^6$ is a group capable of being easily removed by acid hydrolysis, such as tetrahydropyranyl, ethoxyethyl, methoxymethyl or t-butyldimethylsilyl) in the presence of a cuprous ion in a solvent such as diethyl ether or THF at −78°C to room temperature for 1 to 24 hours. Or the compound of formula *14* is reacted with a Grignard reagent of formula *17* under the same reaction conditions as above. As a result, a 1-($\omega$-protected hydroxyalkyl)indole of formula *18* is obtained.

The resulting compound of formula *18* is then deprotected by an acid such as hydrochloric acid, sulfuric acid, acetic acid or oxalic acid to be converted to a 1-($\omega$-hydroxyalkyl)indole of formula *19*. Such a deprotection itself is well known.

The resulting 1-(hydroxyethyl)indole of formula *13* or 1-($\omega$-hydroxyalkyl)indole of formula *19* is condensed with a monocarboxylic acid anhydride in pyridine to form a 1-(acyloxyethyl)indole from the compound of formula *13*, and a 1-($\omega$-acyloxyalkyl) indole from the comoun compound of formula *19*. These products are inclusively represented by formula *20*.

(a)-2 Reaction Scheme 2

According to Reaction Scheme 2, the acylation reaction using the acid anhydride [III] which constitutes one aspect of this invention is carried out by condensing a 1-($\omega$-acyloxyalkyl)-3-unsubstituted indole expressed by the following formula *30*

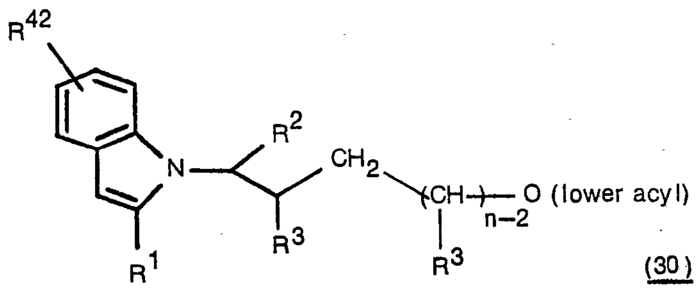

(30)

wherein $R^1$, $R^2$, $R^3$, $R^{42}$ and n are as defined hereinabove,
with the acid anhydride of formula [III] in the presence of hydrogen iodide to give a 3-acyl-1-($\omega$-acyloxyalkyl)-indole of the following formula *31*

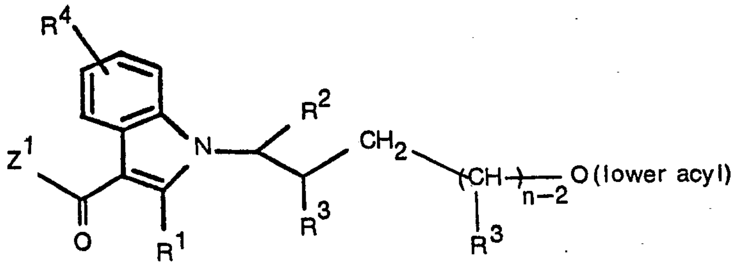

(31)

wherein all symbols are as defined above.

This acylation reaction is carried out under the same reaction conditions as described hereinabove with respect to Reaction Scheme 1, and the final product is obtained by the same isolating and purifying operation as described hereinabove.

According to Reaction Scheme 2, hydrolysis of the compound of formula 31 obtained by the aforesaid acylation reaction gives a 3-acyl-1-($\omega$-hydroxyalkyl)-indole of the following formula *32*

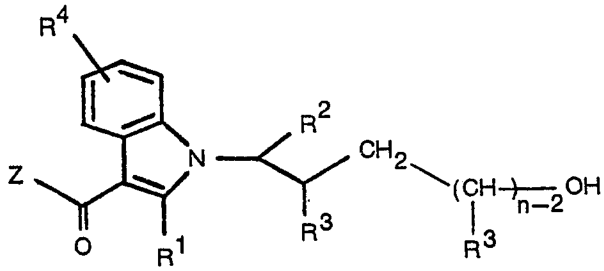

(32)

wherein all symbols are as defined hereinabove.

This hydrolysis reaction is carried out under the same reaction conditions as described above in regard to Reaction Scheme I, and the final product is obtained by the same isolating and purifying operation.

According to Reaction Scheme 2, 3-acylated indole derivatives of formula *31* or *32* which is embraced within formula [I] are obtained which are structurally characterized by the fact the carbon atom at the $\gamma$-position in the substituent at the 1-position is a methylene group.

The 1-($\omega$-acyloxyalkyl)-3-unsubstituted indole (*30*) used in the acylation in Reaction Scheme 2 can be prepared in the following manner.

The indole *10* is reacted with an $\alpha,\beta$-unsaturated carboxylic acid ester of formula *23* (wherein $R^2$, $R^3$ and $R^5$ are as defined above) in an inert organic solvent in the presence of, as a catalyst, a basic compound such as sodium hydride, sodium methoxide, sodium ethoxide, potassium hydride, potassium t-butoxide, or potassium carbonate at a temperature of from –20°C to the refluxing temperature of the reaction system to give a 1-(alkoxycarbonylethyl)indole of formula *24*. This reaction is known as the Michael's addition reaction. The same solvent as used in the reaction of the compounds of formula *10* and *11* above can be used as the inert solvent in this reaction.

The resulting compound of formula *24* is then reduced with lithium aluminum hydride to give a 1-(hydroxypropyl)indole of formula *25*. The compound of formula 25 is halogenated to a compound of

formula *26* which is then converted to a compound of formula *27* with or without going through a compound of formula *28* by the Grignard reaction. Deprotection of the compound of formula *27* gives a 1-($\omega$-hydroxyalkyl)indole of formula *29*.

The resulting compound of formula *25* or *29* is then condensed with a monocarboxylic acid anhydride in pyridine to give a 1-(acyloxypropyl)indole from the compound of formula 25 or a 1-($\omega$-acyloxyalkyl)indole from the compound of formula *29*. These products are inclusively represented by formula *30*.

A series of the above reactions for preparing the compound of formula *30* from the compound of formula 24 are performed under the same conditions as in a series of the corresponding reactions described with regard to Reaction Scheme 1.

(3)-3 Reaction Scheme 3

According to Reaction Scheme 3, the acylation reaction using the acid anhydride [III] which constitutes one aspect of this invention is carried out by condensing a tetrahydroindole derivative of the following formula *35*

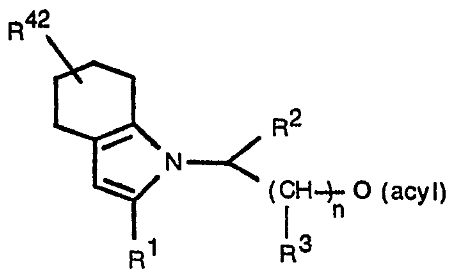

(35)

wherein $R^1$, $R^2$, $R^3$, $R^{42}$ and n are as defined hereinabove,
with the acid anhydride of formula [III] in the presence of hydrogen iodide to give a 3-acyl-1-($\omega$-acyloxyalkyl)-tetrahydroindole of the following formula *36*

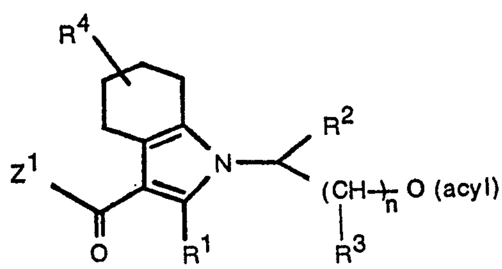

(36)

wherein $R^1$, $R^2$, $R^3$, $R^4$, n and $Z^1$ are as defined hereinabove.

This acylation reaction is performed under the same conditions for the acylation of 1-($\omega$-acyloxyalkyl)-3-unsubstituted indoles described hereinabove with regard to Reaction Scheme 1, and the final product of formula *36* is obtained by the same isolating and purifying operation.

According to Reaction Scheme 3, the isolated compound of formula *36* is then dehydrogenated to give a 3-acyl-1-($\omega$-acyloxyalkyl)indole of the following formula *37*

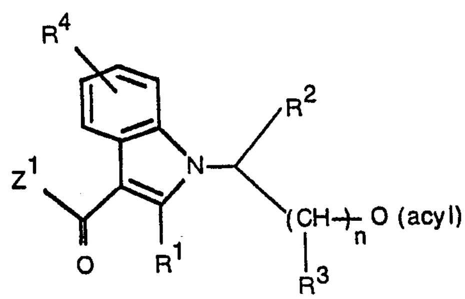

(37)

wherein $R^1$, $R^2$, $R^3$, $R^4$, n and $Z^1$ are as defined hereinabove.

This dehydrogenation reaction is carried out in an inert organic solvent usually at a temperature of 0°C to the refluxing temperature of the reaction system usually for 30 minutes to 72 hours using 2 to 10 moles of a dehydrogenating agent such as 2,3-dichloro-5,6-dicyano-p-benzoquinone (DDQ), 2,3,5,6-tetrachloro-p-benzoquinone (Chloranil) or 3,5,4,6-tetrachloro-o-benzoquinone per mole of the compound of formula *36*.

This dehydrogenation reaction is likewise carried out in an inert organic solvent at a temperature

of usually about 0°C to the refluxing temperature of the reaction system for a period of usually 30 minutes to 72 hours using 0.1 to 10 moles of a dehydrogenating catalyst such as selenium, palladium/carbon, platinum, or rhodium per mole of the compound of formula *36*.

Suitable inert organic solvents are aprotic inert organic solvents examples of which include ethers such as THF, dioxane, 1,2-dimethoxyethane, diethyleneglycol dimethyl ether, diethylene glycol diethyl ether, and diethylene glycol dibutyleter, and aromatic hydrocarbons such as benzene, toluene, xylene, chlorobenzene, nitrobenzene, naphthalene and quinoline.

Usually, the reaction can be monitored by thin-layer chromatography.

Isolation of the final product (*37*) from the reaction mixture and the purification can be performed usually by removing insoluble materials (e.g., 2,3-dichloro-5,6-dicyano-p-dihydroquinone, or the remaining inert palladium/carbon catalyst, etc.) from the reaction mixture by filtration, distilling off the reaction solvent under reduced pressure, and then subjecting the residue to the same treating operation as described in Reaction Scheme 1.

According to Reaction Scheme 3, hydrolysis of the 3-acyl-1-($\omega$-acyloxyalkyl)indole of formula *37* formed by the above acylation reaction and the subsequent dehydrogenation gives a 3-acyl-1-($\omega$-hydroxyalkyl)indole of the following formula *38*

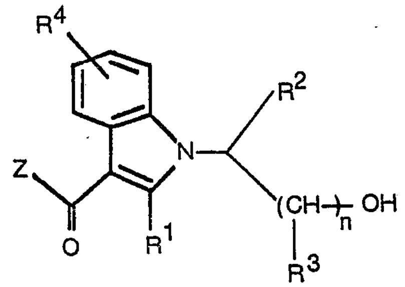

(38)

wherein all symbols are as defined hereinabove.

This hydrolysis reaction is carried out under the same conditions as described with regard to Reaction Scheme 1, and the final desired product can be obtained by the same isolating and purifying operation.

According to Reaction Scheme 3, there can be thus produced not only acylated indole derivatives of this invention included within formula [I] in which the $\alpha$-carbon atom and/or $\beta$-carbon atom in the substituent at the 1-position, but also those in which all carbon atoms including these $\alpha$- and $\beta$-carbon atoms are substituted by an alkyl group ($R^3$).

The 1-($\omega$-acyloxyalkyl)-tetrahydroindole (*35*) used in the acylation reaction according to Reaction Scheme 3 is prepared by condensing 1 mole of a 1,4-diketone of formula *33* (wherein $R^1$ and $R^{42}$ are defined hereinabove) with 0.5 to 5 moles of a primary amine of formula *34* (wherein $R^2$, $R^3$ and n are as defined hereinabove) in the presence of acetic acid and in the presence or absence of an inert organic solvent at a temperature of 0°C to the refluxing temperature of the reaction system.

Suitable inert organic solvents are, for example, benzene, toluene, xylene and dioxane.

(a)-4 Reaction Scheme 4

According to Reaction Scheme 4, the acylation reaction using an acid anhydride which constitutes one aspect of the present invention is carried out by condensing a 1-($\omega$-acyloxyalkyl)-tetrahydroindole of the following formula *46*

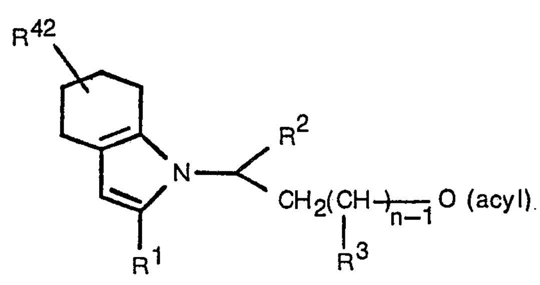

(46)

wherein all symbols are as defined hereabove,

with the acid anhydride of formula [III] in the presence of hydrogen iodide to give a 3-acyl-1-(ω-acyloxyalkyl)tetrahydroindole of the following formula *47*

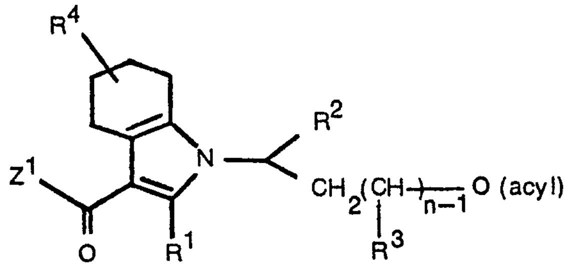

(47)

wherein all symbols are defined hereinabove.

This acylation reaction is carried out under the same conditions as described hereinabove with regard to Reaction Scheme 1, and the final desired product can be obtained by the same isolating and purifying operation.

According to Reaction Scheme 4, dehydrogenation of the product (*47*) obtained by the acylation reaction gives the compound (*21*) mentioned above, and hydrolysis of the compound (*21*) gives the compound (*22*) described above. The dehydrogenation and hydrolysis reactions are carried out in the same way as described with regard to Reaction Scheme 3.

The 1-(ω-acyloxyalkyl)-tetrahydroindole (46) used in the acylation reaction according to Reaction Scheme 4 can be prepared in the following manner.

One mole of the 1,4-diketone (*33*) is condensed with 1 to 2 moles of a mineral acid salt (such as a hydrochloride, hydrobromide or sulfate) of an α-amino acid ester of formula *39* (wherein $R^2$ and $R^5$ are as defined hereinabove) in the presence of 1 to 3 moles of an acid acceptor such as sodium acetate, potassium acetate, sodium carbonate, potassium carbonate, sodium hydrogen carbonate or potassium hydrogen carbonate in acetic acid or an inert organic solvent containing acetic acid at a temperature of 0°C to the refluxing temperature of the reaction system. Suitable inert organic solvents are, for example, benzene, toluene, xylene and dioxane.

The 1-(alkyloxycarbonylmethyl)tetrahydroindole of formula *40* (wherein $R^{42}$ is as defined hereinabove) thus obtained is reduced with lithium aluminum hydride to give a 1-(hydroxyethyl)tetrahydroindole of formula *41*, which is converted to a 1-(haloethyl)tetrahydroindole of formula *42* by halogenation. The compound of formula *42* is then subjected to a Grignard reaction to convert it to a 1-(ω-protected hydroxyalkyl)tetrahydroindole of formula *44* through or not through an intermediate of formula *43*. Deprotection of the compound of formula *44* gives a 1-(ω-hydroxyalkyl)tetrahydroindole of formula *45*.

The resulting 1 - (hydroxyethyl)tetrahydroindole of formula *41* and 1 - (ω - hydroxyalkyl)tetrahydroindole· of formula *44* are each condensed with a monocarboxylic acid anhydride in pyridine to give a 1-(acyloxyethyl)tetrahydroindole from the compound of formula *41*, and a 1-(ω-acyloxyalkyl)tetrahydroindole from the compound of formula *44*. These products are inclusively represented by formula *46*.

A series of reactions for production of the compound of formula *46* from the compound of formula *40* are carried out under the same conditions as in a series of the corresponding reactions in Reaction Scheme 1.

As can be understood from the above description, when an optically active α-amino acid is used as the α-amino acid in formula *39* in the reaction of Reaction Scheme 4, there can be obtained acylated indole derivatives of formula [I] expressed by formula *21* or *22* which are optically active at the α-carbon atom of the substituent at the 1-position.

According to Reaction Schemes 1 to 4, there can be advantageously produced 3-acyl-1-(ω-hydroxyalkyl)indole derivatives exemplified by compounds (100) to (137) given hereinabove, or 3-acyl-1-(ω-acyloxyalkyl)indole derivatives exemplified by compounds (200) to (219) given hereinabove.

(b) Production of compounds of general formula [I] wherein Y is an oxygen atom, A is an alkyl group or a phenyl group optionally substituted by $R^4$ in accordance with Reaction Schemes 5 to 8:—

(5)

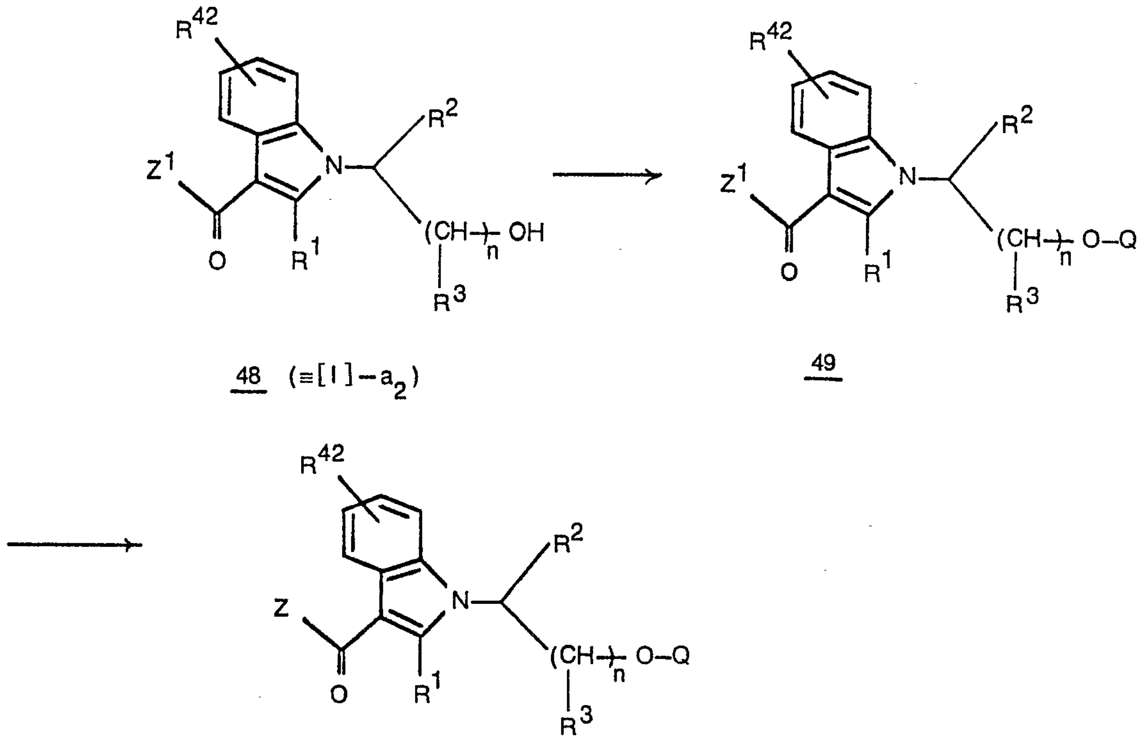

48 (≡[I]−$a_2$)

49

[I]−e

REACTION SCHEME 5

(6)

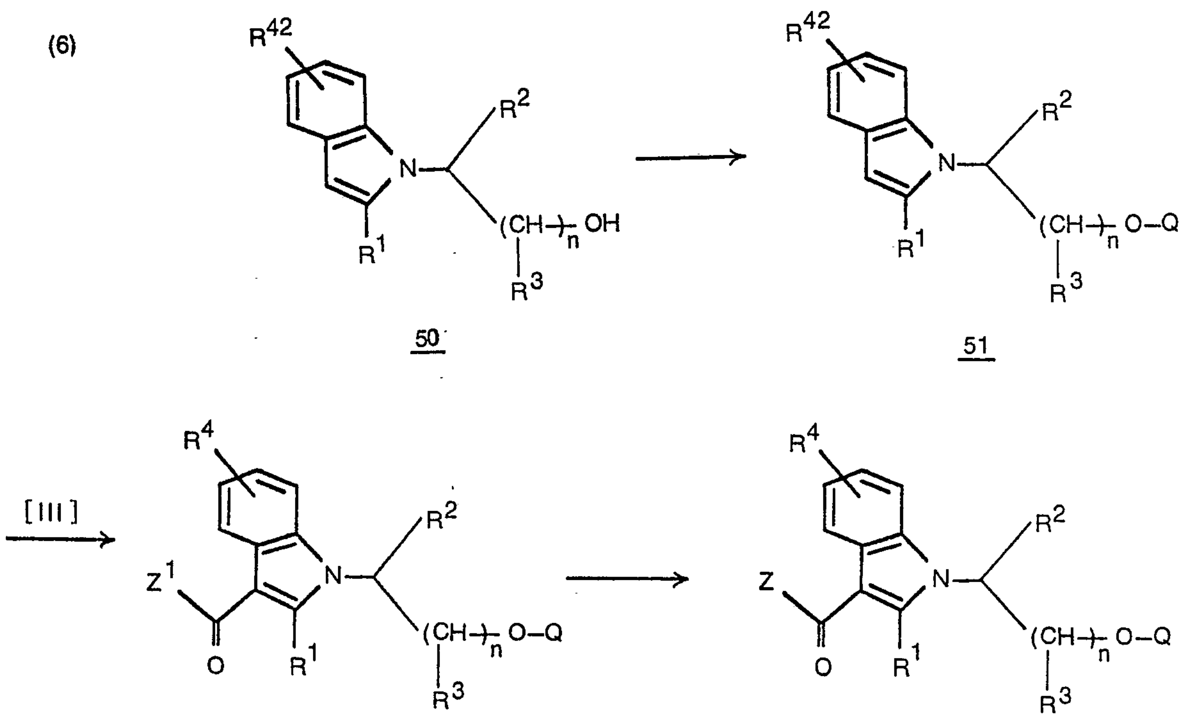


50

51

52

53

REACTION SCHEME 6

(7)

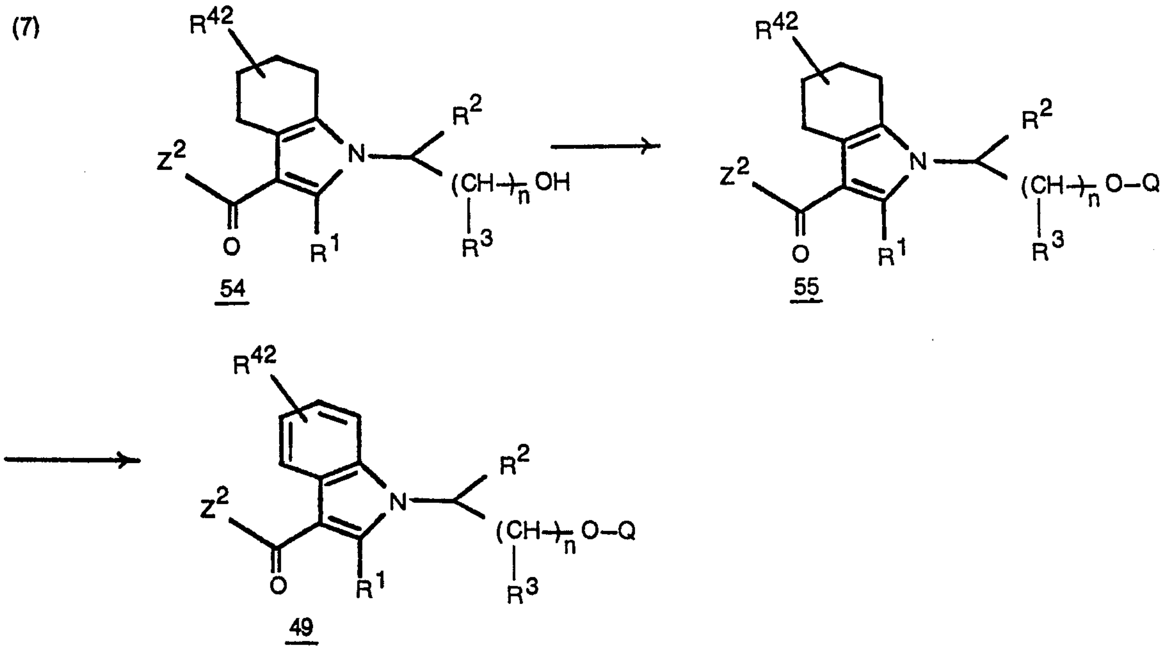

54 55 49

REACTION SCHEME 7

(8)

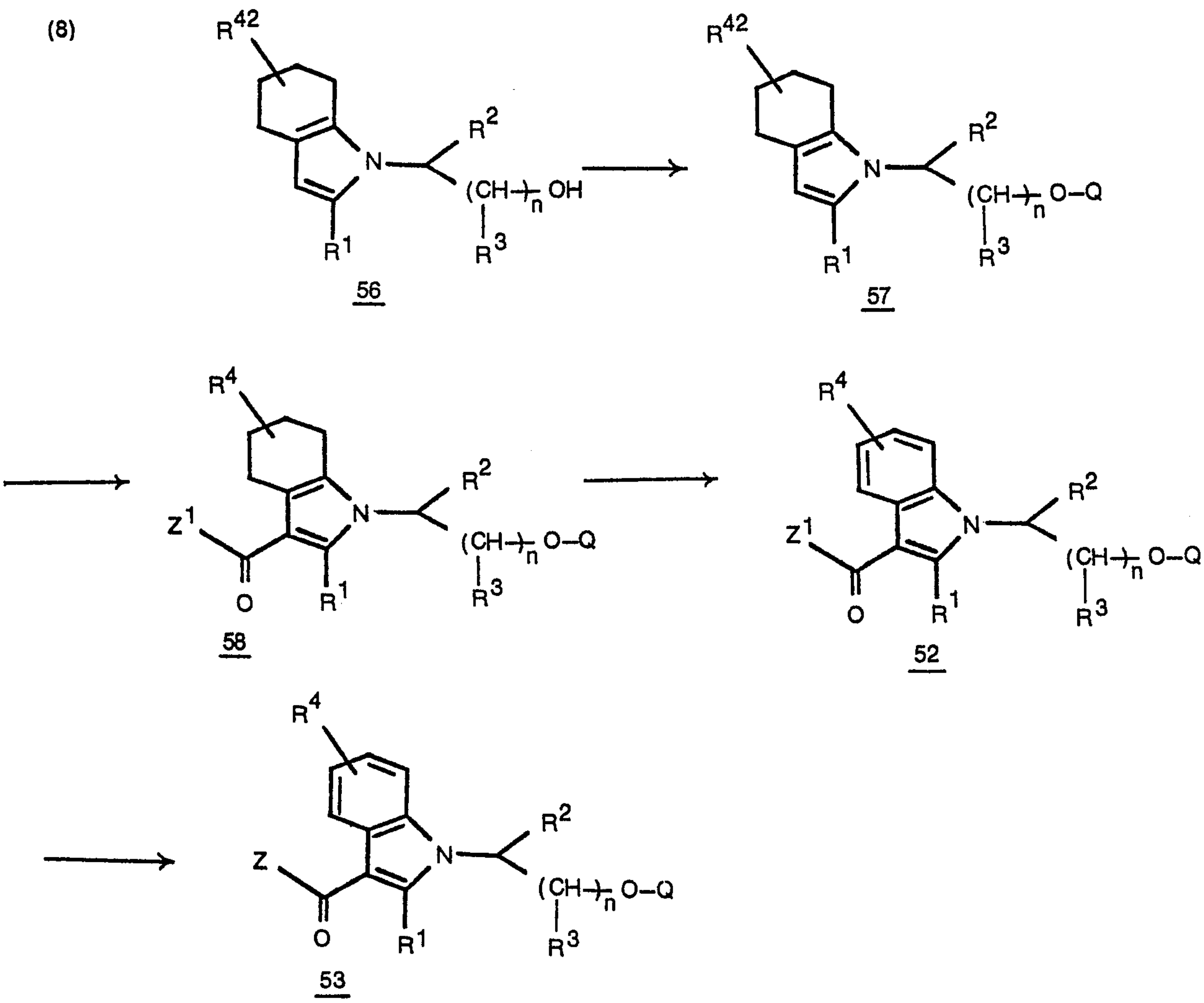

56 57 58 52 53

REACTION SCHEME 8

(b)-1 Reaction Scheme 5

According to Reaction Scheme 5, a 3-acyl-1-($\omega$-hydroxyalkyl)indole of the following formula *48*

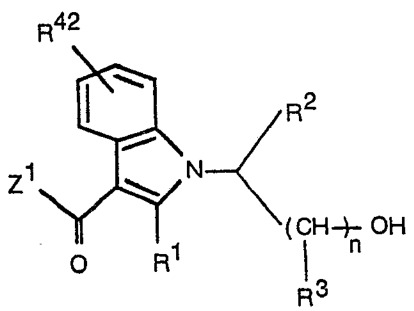

(48)

wherein $R^1$, $R^2$, $R^3$, $R^{42}$ and n are as defined hereinabove, and $Z^1$ represents a phenyl or thienyl group optionally substituted by $R^{42}$ or a protected hydroxyl group,

is condensed with a halogen compound of the following formula [V]

$$Q{-}X \qquad [V]$$

wherein Q represents an alkyl group, a phenylalkyl group, or a phenylalkyl group substituted by $R^{42}$, and X represents a halogen atom,

in the presence of a basic compound to give a 3-acyl-1-(oxaalkyl)indole of the following formula *49*

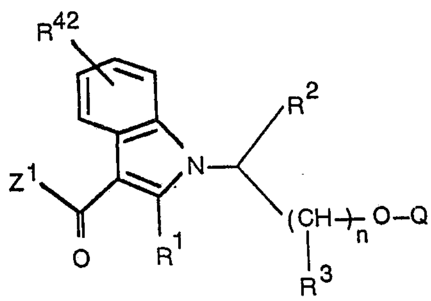

wherein all symbols are as defined above.

The condensation reaction (etherification reaction) is carried out by treating 1 mole of the compound of formula *48* with 1 to 10 moles of the halogen compound in an inert organic solvent in the presence of 1 to 5 moles of the basic compound at a temperature of −78°C to the refluxing temperature of the reaction system.

In formula [V], examples of the group Q are linear or branched alkyl groups having 1 to 6 carbon atoms such as methyl, ethyl, n-propyl, iso-propyl, n-butyl, sec-butyl, iso-butyl, n-pentyl and n-hexyl, phenylalkyl groups with the alkyl group having 1 or 2 carbon atoms, such as benzyl, $\alpha$-phenethyl and $\beta$-phenethyl and such phenylalkyl groups substituted by the group $R^4$. X is preferably a chlorine, bromine or iodine atom.

Iodides and bromides are desirable as the halogen compounds of formula [V] in view of their respectivity. Examples include methyl iodide, ethyl iodide, ethyl bromide, n-propyl iodide, iso-propyl bromide, n-butyl bromide, n-pentyl bromide, n-hexyl bromide, benzyl bromide, $\alpha$-phenethyl bromide, $\beta$-phenethyl bromide, benzyl chloride, $\alpha$-phenethyl chloride, p-methoxy-$\alpha$-phenethyl bromide, and p-chlorobenzyl chloride. These are merely exemplification of preferred compounds of formula [V].

Examples of preferred basic compounds are alkali metal hydrides or amides, such as sodium hydride, potassium hydride, sodium amide and lithium hydride.

Preferred reaction solvents are aprotic inert organic solvents such as diethyl ether, THF, dioxane, 1,2-dimethoxyethane, ethylene glycol dimethyl ether, benzene, toluene, xylene, DMF, DMSO and HMPA.

The compound of formula *49* is, if desired, subjected to hydrolysis to form the compound of formula [I]-e.

The compound of formula 48 overlap the compound of formula 38 prepared by Reaction Scheme 3. In other words, a compound of formula *38* in which $R^4$ is $R^{42}$ and Z is $Z^1$ is the compound of formula *48*.

(b)-2 Reaction Scheme 6

According to Reaction Scheme 6, a 3-acyl-1-(oxaalkyl)-indole of the following formula *52*

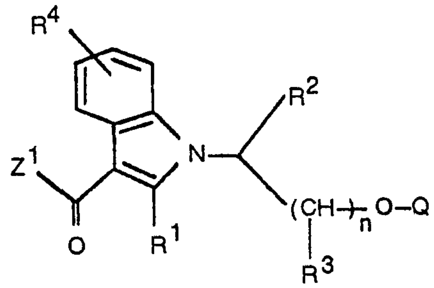

(52)

wherein all symbols are as defined hereinabove, is obtained by condensing a 1-(ω-hydroxyalkyl)-3-unsubstituted indole of the following formula *50*

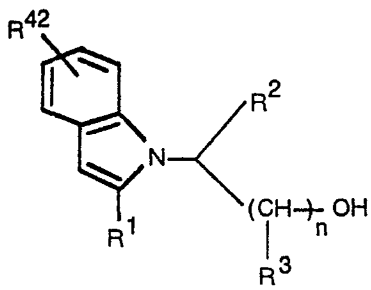

(50)

wherein all symbols are as defined hereinabove,

with the halogen compound of formula [V] in the presence of a basic compound, and then reacting the condensate further with the acid anhydride of formula [III] in the presence of hydrogen iodide.

The above etherification reaction can be performed under the same reaction conditions as described above with respect to Reaction Scheme 5. The above acylation reaction can be performed under the same reaction conditions as described with regard to the acylation reaction involving use of the acid anhydride of formula [III] in Reaction Scheme 1.

According to Reaction Scheme 6, hydrolysis of the compound of formula *52* under the same conditions as in Reaction Scheme 1 gives a compound of the following formula *53*

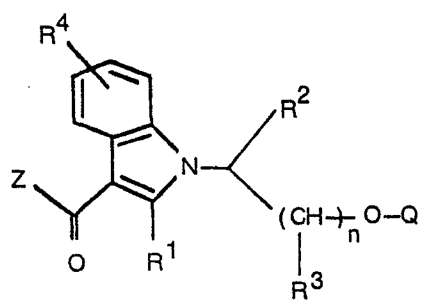

(53)

wherein $R^1$, $R^2$, $R^3$, $R^4$, n, Q and Z are as defined above.

The compound of formula *50* can be prepared by dehydrogenating the compound of formula *45* in Reaction Scheme 3 under the same dehydrogenation reaction conditions for the compound *36*, and then hydrolyzing the product.

(b)-3 Reaction Scheme 7

According to Reaction Scheme 7, the same compound of *49* as obtained in Reaction Scheme 7 can be obtained by condensing a 3-acyl-1-($\omega$-hydroxyalkyl) tetrahydroindole of the following formula *54*

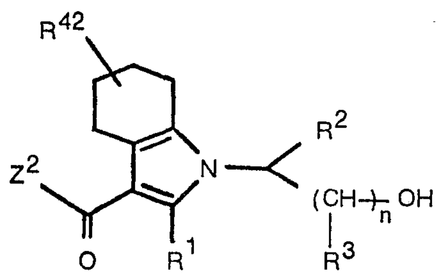

(54)

wherein all symbols are as defined hereinabove,

with the halogen compound of formula [V] in the presence of a basic compound, and then dehydrogenating the resulting 3-acyl-1-(oxaalkyl)tetrahydroindole of the following formula *55*

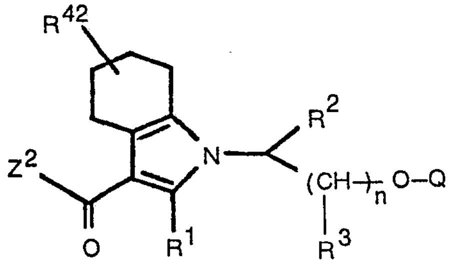

(55)

wherein all symbols are as defined hereinabove.

This etherification reaction can be performed under the same conditions as described hereinabove with regard to Reaction Scheme 5, and the dehydrogenation reaction can be performed under the same conditions as in the dehydrogenation reaction of the compound of formula *36* in Reaction Scheme 3.

The compound of formula *54* overlaps some of the compounds of formula *36* in Reaction Scheme 3.

(b)-4 Reaction Scheme 8

According to Reaction Scheme 8, a 1-($\omega$-hydroxyalkyl)-tetrahydroindole of the following formula *56*

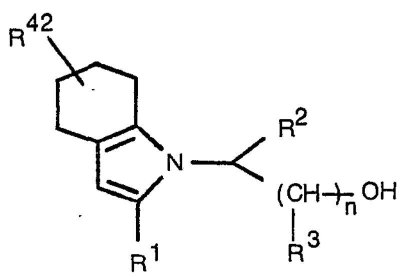

(56)

wherein all symbols are as defined hereinabove,

with the halogen compound of formula [V] in the presence of a basic compound to form a 1-(oxaalkyl)tetrahydroindole of the following formula *57*

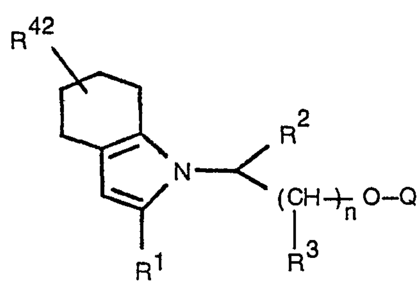

(57)

wherein all symbols are as defined hereinabove,

then acylating the compound of formula *57* with the acid anhydride of formul [III] in the presence of hydrogen iodide to form a 3-acyl-1-(oxaalkyl)tetrahydroindole of the following formula *58*

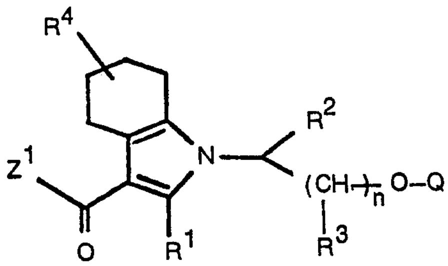

(58)

wherein all symbols are as defined hereinabove,
and dehydrogenating the compound of formula *58* to form the 3-acyl-1-(oxaalkyl)indole of formula *52*. Hydrolysis of the compound of formula *52* gives the compound of formula *53*.

The above etherification reaction and dehydrogenation reaction can be performed under the same conditions as in Reaction Scheme 7. The acylation reaction and hydrolysis reaction can be performed under the same conditions as in Reaction Scheme 6.

The compound of formula *56* is obtained by hydrolysis of the compound *35* in Reaction Scheme 3.

According to Reaction Schemes 5 to 8, there can be advantageously prepared 3-acyl-1-(ω-alkyl or substituted or unsubstituted phenylalkyl-oxyalkyl)indole derivatives exemplified by compounds (300) to (347) exemplified hereinabove.

(c) Production of compounds of general formula [I] wherein Y is an oxygen atom, and A is a phenyl pyridinyl, imidazolyl or thiadiazolyl group optionally substituted by the group $R^4$:—

(9)

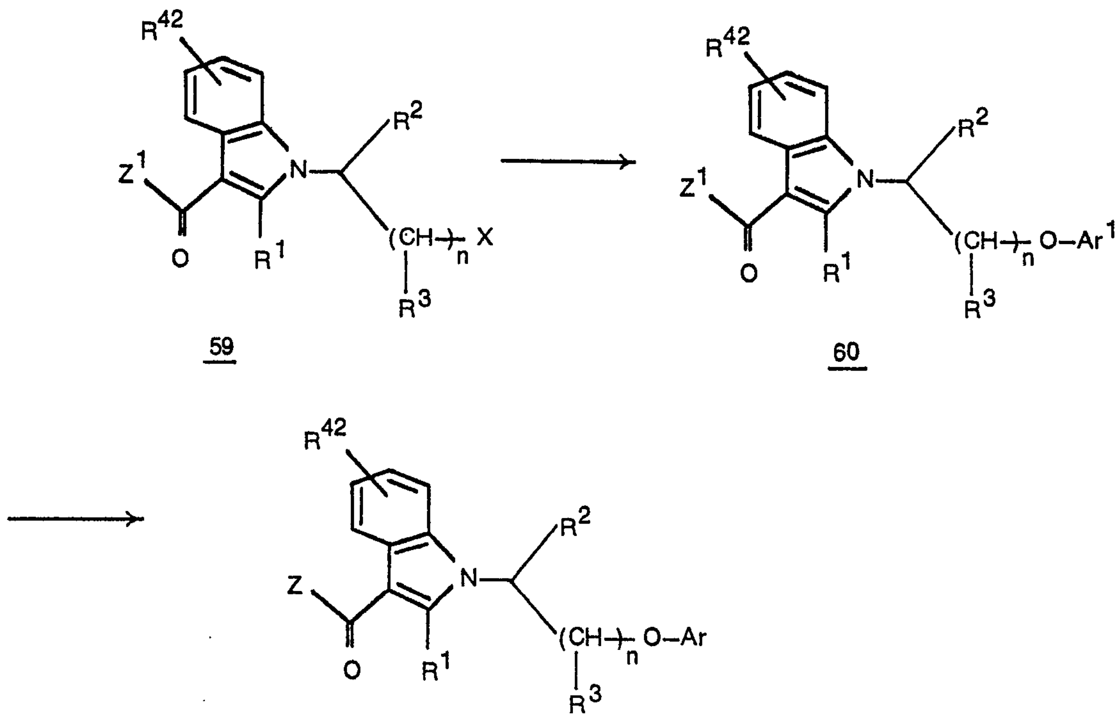

59

60

61

REACTION SCHEME 9

According to Reaction Scheme 9, a 3-acyl-1-(ω-aryloxyalkyl)indole of the following formula *60*

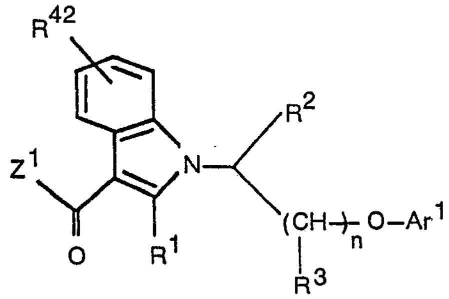

(60)

wherein $Ar^1$ represents a phenyl pyridinyl imidazolyl or thiadiazolyl group optionally substituted by $R^{42}$ or a protected hydroxyl group,
is prepared by condensing a 3-acyl-1-(ω-haloalkyl)indole expressed by the following formula *59*

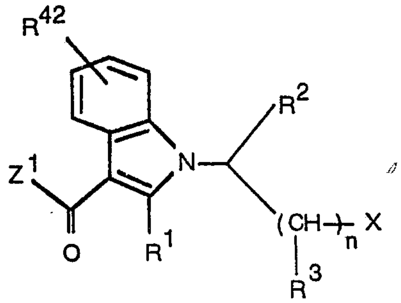

(59)

wherein $R^1$, $R^2$, $R^3$, $R^{42}$, n and $Z^1$ are as defined hereinabove, and X represents a halogen atom, with an aromatic hydroxy compound of the formula [VI]

$$Ar^1—OH$$

wherein $Ar^1$ is the same as defined hereinabove,
in the presence of a basic compound in an inert organic solvent.

This condensation reaction (etherification reaction) can be performed by using 1 mole of the halogen compound of formula *59* and 1 to 2 moles of the aromatic hydroxy compound of formula [VI] in the presence of 1 to 3 moles of the basic compound at a temperature of —78°C to the refluxing temperature of the reaction system.

Chrlorine, bromine and iodine atoms are preferred as the halogen atom X in formula *59*. The compound of formula *59* is prepared by halogenating some of the compounds of formula *38* in Reaction Scheme 3 (performed under the same conditions as in the halogenation reaction of preparing the compound of formula *14* from the compound of formula *13* in Reaction Scheme 1).

Examples of the aromatic hydroxy compounds of formula [VI] are phenol, p-acetoxyphenol, p-methoxyphenol, o-chlorophenol, m-cresol, p-isopropylphenol, 3-allyl alcohol, and 4-hydroxypyridine.

Preferred basic compounds include, for example, alkali metal hydrides or amides such as sodium hydride, potassium hydride, lithium hydride and sodium amide.

The same aprotic inert organic solvents as used in the etherification reaction in Reaction Scheme 5 are preferred as the reaction solvent in the above reaction.

According to Reaction Scheme 9, hydrolysis of the compound of formula *60* obtained by the above reaction under the same conditions as in the hydrolysis of the compound of formula *52* in Reaction Scheme 6 gives a compound of the following formula *61*

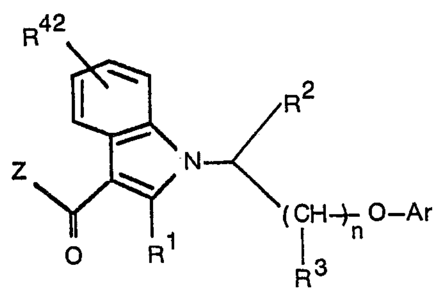

(61)

$R^1$, $R^2$, $R^3$, $R^{42}$, n and Z are as defined hereinabove, Ar represents a phenyl pyridinyl, imidazolyl or thiadiazolyl group optionally substituted by $R^{42}$ or a hydroxyl group.

According to Reaction Scheme 9, there can be prepared 3-acyl-1-(ω-substituted or unsubstituted phenyl or heterocyclicoxyalkyl)indole derivatives exemplified by compounds (348) to (352) given hereinabove which come within the definition of formula [I] of this invention.

[B] Compounds of formula [I] wherein Y is a bond and A is a hydrogen atom:—

These compounds can be produced in accordance with Reaction Schemes 10 to 13 below.

(10)

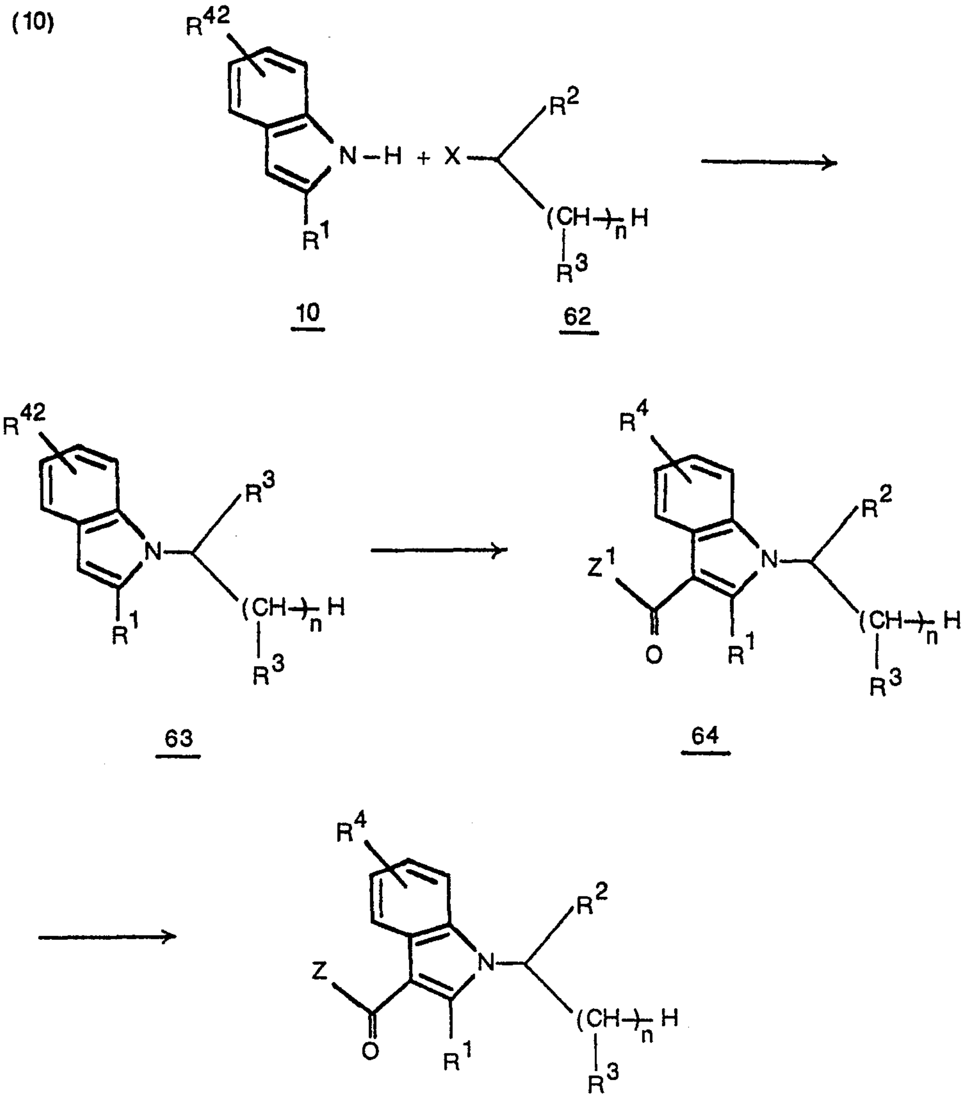

10 62

63 64

65

REACTION SCHEME 10

(11)

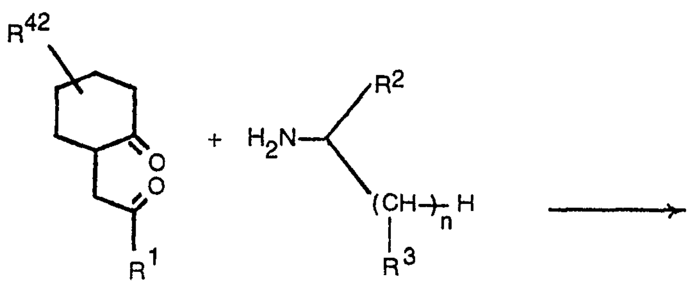

33 66

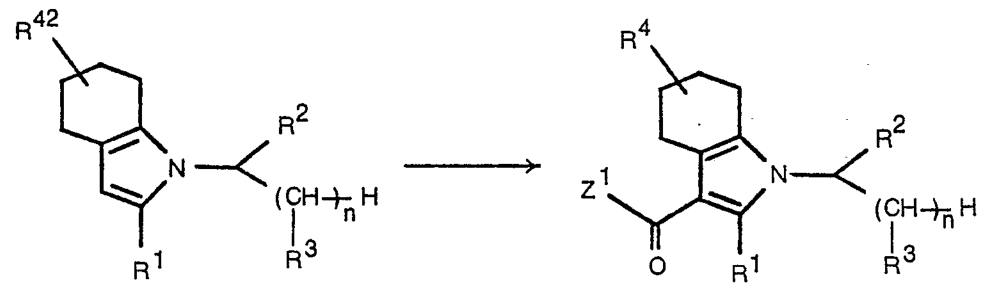

67 68

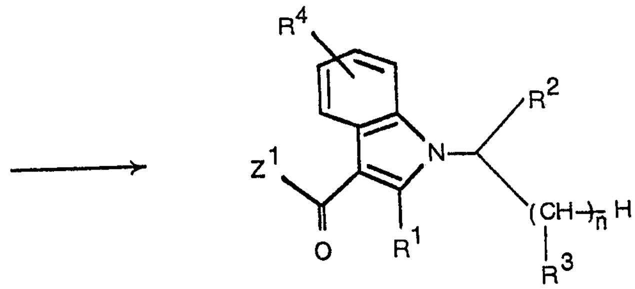

64

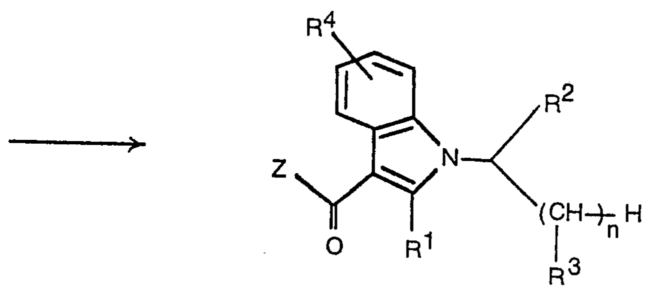

65

REACTION SCHEME 11

(12)

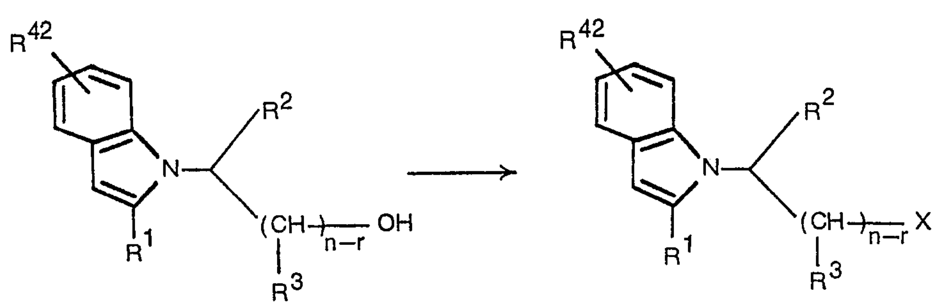

69 70

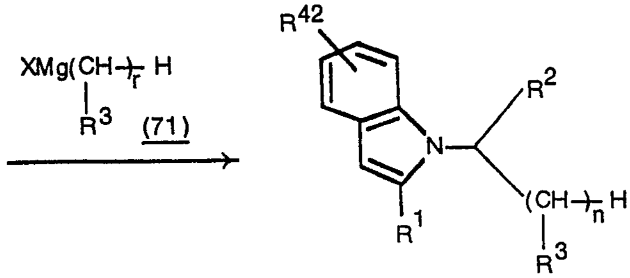

(71)

63 (≡[II]–b)

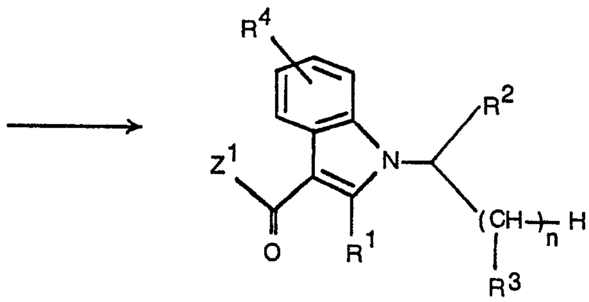

64

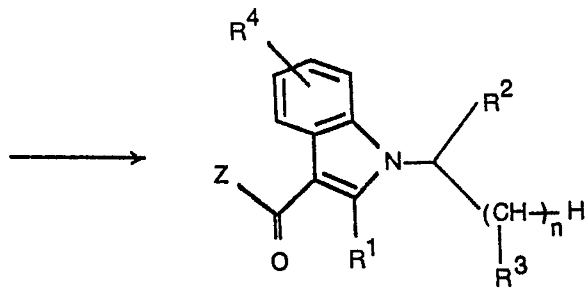

65 (≡[I]–b)

REACTION SCHEME 12

(13)

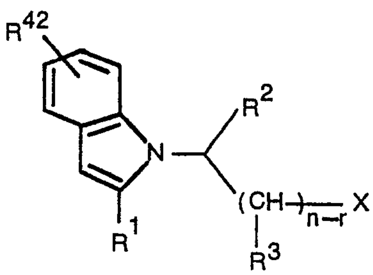

69

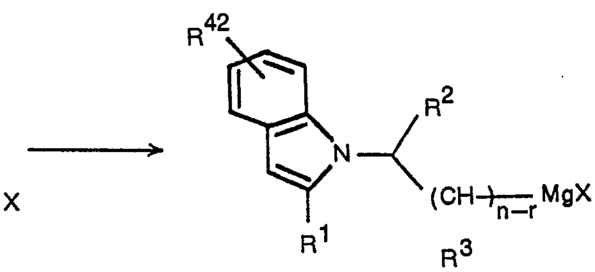

72

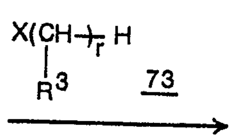

73

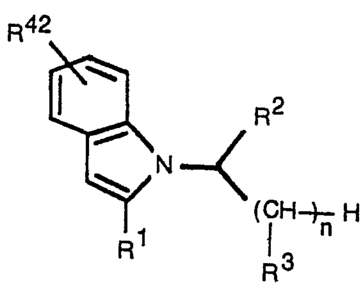

63

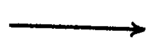

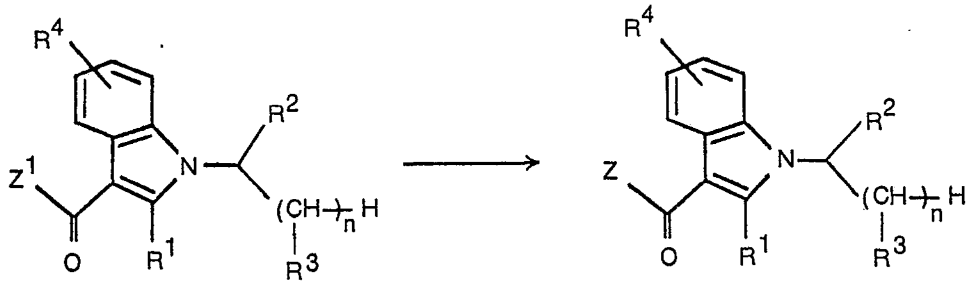

64

65

REACTION SCHEME 13

(14)

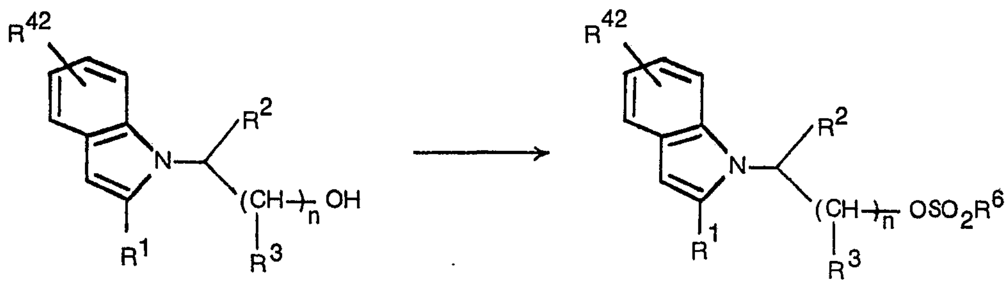

50

74

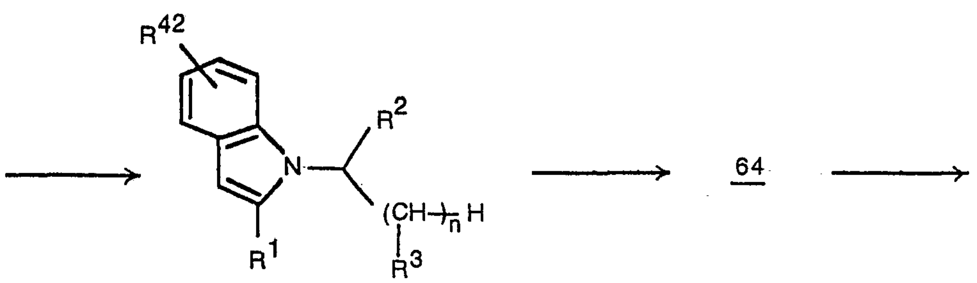

64 65

63

REACTION SCHEME 14

According to Reaction Schems 10 and 12 to 14, a 1-alkyl-3-unsubstituted indole having an alkyl group of the desired number of carbon atoms at the 1-position and represented by the following formula *63*

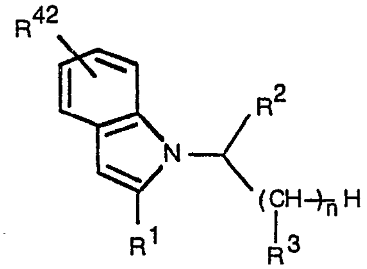

(63)

wherein $R^1$, $R^2$, $R^3$, $R^{42}$ and n are as defined hereinabove.

is condensed with the acid anhydride of formula [III] in the presence of hydrogen iodide to give a 3-cyano-1-alylindole of the following formula *64* having the alkyl group with the desired number of carbon atoms at the 1-position

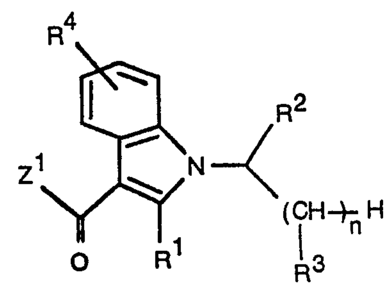

(64)

wherein all symbols are as defined above.

Hydrolysis of the compound of formula *64* gives a compound of the following formula *65* having the alkyl group of the desired number of carbon atoms at the 1-position

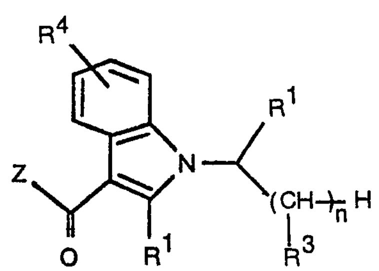

(65)

wherein all symbols are as defined hereinabove.

The above acylation reaction and hydrolysis reaction are carried out under the same conditions as described hereinabove with regard to Reaction Scheme 1.

The 1-alkyl-3-unsubstituted indole of formula *63* which gives the 3-acylindole derivatives of formulae *64* and *65* which fall within the definition of formula [I] can be produced

(i) in accordance with Reaction Scheme 10 by reaction 1 mole of the indole of formula *10* with 1 to 5 moles of an alkyl halide of formula *62* (wherein $R^2$, $R^3$, X and n are as defined hereinabove) in the presence of a basic catalyst in an inert organic solvent at a temperature of 0°C to the refluxing temperature of the reaction system;

(ii) in accordance with Reaction Scheme 12 by halogenating a 1-(ω-hydroxyalkyl)-3-unsubstituted indole of formula *69* (wherein $R^1$, $R^2$, $R^3$ and $R^{42}$ are as defined hereinabove, n is 1 to 10, r is 1 to 9, and n minus r is 1 to 9) under the same reaction conditions as in the production of the compound of formula *14* from the compound of formula *13* in Reaction Scheme 1, and condensing the resulting 1-(ω-haloalkyl)-3-unsubstituted indole of formula *70* (wherein $R^1$, $R^2$, $R^3$, $R^{42}$, n and r are as defined in formula *69*, and X represents a halogen atom such as chlorine or bromine) with a Grignard reagent of formula *71* (wherein $R^3$, X and r are as defined hereinabove) under the same conditions as in the production of the compound of formula *18* from the compounds of formulae *14* and *17* in Reaction Scheme 1;

(iii) in accordance with Reaction Scheme 13 by reacting the compound of formula *69* with metallic magnesium under the conditions as in the production of the compound of formula *15* from the compound of formula *14* in Reaction Scheme 1 to form a compound of formula *72* (wherein $R^1$, $R^2$, $R^3$, $R^{42}$, X, n and r are as defined above), and condensing the compound of formula *72* with an alkyl halide of formula *73* (wherein $R^3$, X and r are as defined hereinabove) under the same conditions as in the production of the compound of formula *18* from the compounds of formulae *15* and *16* in Reaction Scheme 1; or

(iv) in accordance with Reaction Scheme 14 by condensing 1 mole of 1-(ω-hydroxyalkyl)-3-unsubstituted indole of formula *50* with 1 to 3 moles of a sulfonyl halide of the formula

$$R^6SO_2X$$

wherein $R^6$ represents a methyl group or p-tolyl group and X represents a halogen atom such as chlorine or bromine,

in the presence of a basic compound such as triethylamine or pyridine at a temperature of 0°C to 50°C to form a 1-(ω-organic sulfonyloxyalkyl)-3-unsubstituted indole of formula *74* (wherein $R^1$, $R^2$, $R^3$, $R^{42}$, $R^6$ and n are as defined hereinabove), and treating the compound of formula *74* with lithium aluminum hydride in an inert organic solvent such as diethyl ether, tetrahydrofuran or dioxane at a temperature of 0°C to the refluxing temperature of the reaction system to remove the organic sulfonyloxy group.

According to Reaction Scheme 11, 3-acyl-1-alkylindoles of formulae *64* and *65* can be produced as follows:

The 1,4-diketone of formula *33* is condensed with the primary amine of formula *66* (wherein $R^2$, $R^3$ and n are as defined hereinabove) under the same conditions as in the production of the compound of formula *35* from the compounds of formulae *33* and *34* in Reaction Scheme 3 to afford a 1-alkyl-3-unsubstituted-tetrahydroindole of formula *67* (wherein $R^1$, $R^2$, $R^3$, $R^{42}$ and n are as defined as above) which is then converted to the compound of formula *64* through the compound of formula *68* under the same conditions as in the production of the compound *37* by dehydrogenation of the compound of formula *36* formed by acylation of the compound of formula *35* in Reaction Scheme 3 (the acylation reaction and dehydrogenation reaction).

According to Reaction Schemes 11 to 14, 3-acyl-1-alkylindole derivatives exemplified by compounds (700) to (709) exemplified hereinabove which come within the acylated indole derivatives [I] of this invention can be advantageously produced.

[C] Compounds of general formula [I] wherein Y is a sulfur atom and A is a hydrogen atom or acyl group:—

These compounds can be produced in accordance with Reaction Scheme 15 below.

(15)

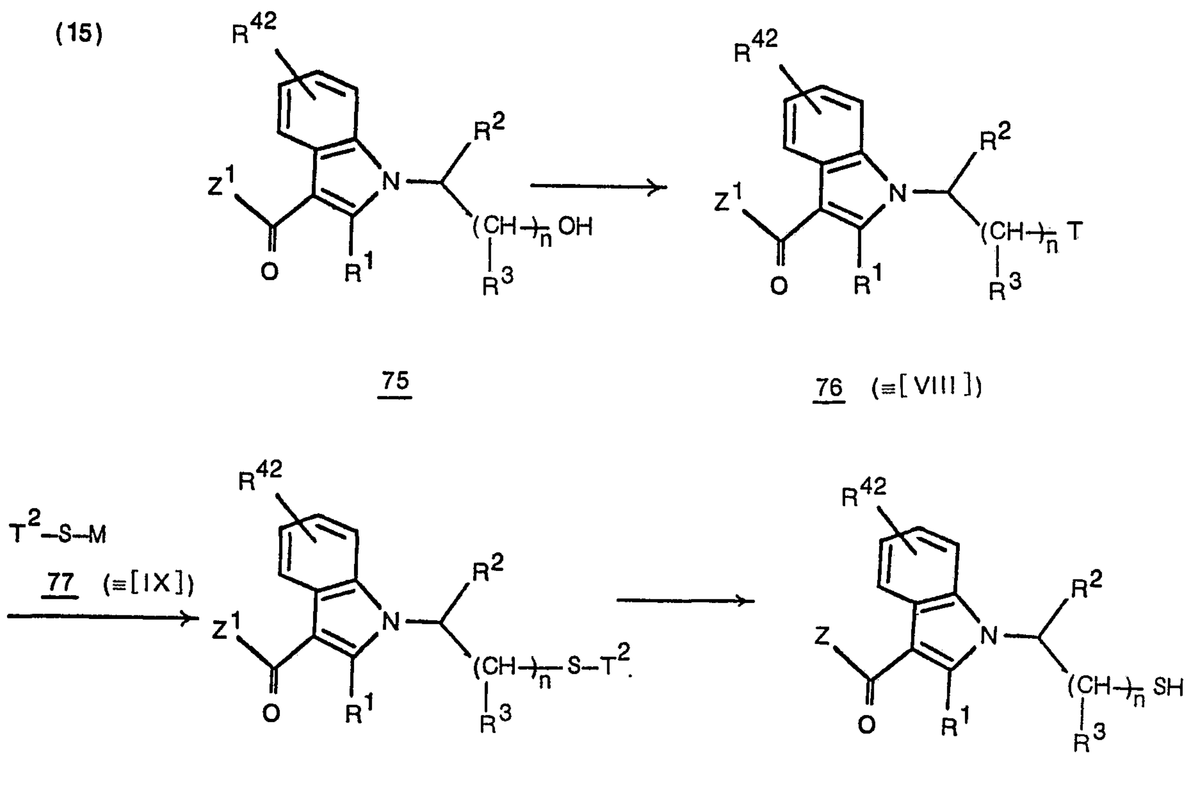


75

76 (≡[VIII])

78

79

REACTION SCHEME 15

According to Reaction Scheme 15, a 3-acyl-1-(ω-halogeno or sulfonyloxyalkyl)indole of the following formula *76*

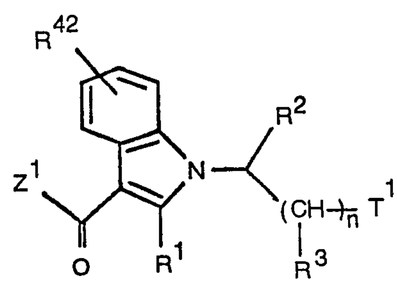

wherein $R^1$, $R^2$, $R^3$ $R^{42}$, n and $Z^1$ are as defined above, and $T^1$ represents a halogen atom such as a chlorine, bromine or iodine atom, $-OSO_2CH_3$ or $-OSO_2$ 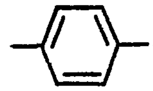 $-CH_3$,

is reacted with a thiocarboxylic acid or thiourea or an alkali metal salt having the following formula *77*

$$T^2—S—M \quad (77)$$

wherein $T^2$ represents an acyl group having 1 to 6 carbon atoms such as an acetyl, propionyl or amidino group, and M represents a hydrogen atom or an alkali metal such as sodium or potassium,

to form a 3-acyl-1-(ω-acylthio or amidinothioalkyl)indole of the following formula *78*

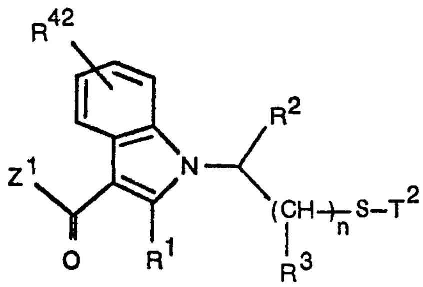

(78)

wherein $R^1$, $R^2$, $R^3$, $R^{42}$, n, $T^2$ and $Z^1$ ar the same as defined hereinabove.

If desired, the compound of formula *78* is hydrolyzed to give a 3-acyl-1-(ω-mercaptoalkyl)indole of the following formula *79*

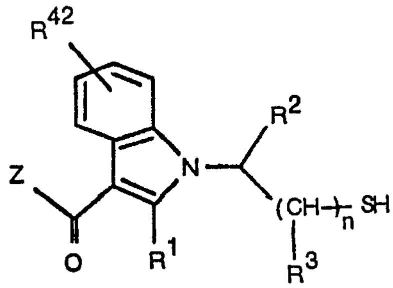

(79)

wherein $R^1$, $R^2$, $R^3$, $R^{42}$, n and Z are defined hereinabove.

The reaction between the compound of formula *76* and the compound of formula 77 is usually carried out in an inert organic solvent such as DMF, DMSO, HMPA, methanol, ethanol, acetone, ether, or THF at a temperature of about 0°C to the refluxing temperature of the reaction system for 30 minutes to 48 hours.

In this reaction, 1 to 10 moles of the compound of formula *77* can be used per mole of the compound of formula *76*.

A compound of formula *78* in which $T^2$ is an acyl group is embraced by formula [I] and constitutes part of the acyl indole derivatives of the present invention.

If required, the compound of formula *78* is hydrolyzed under the same conditions as described hereinabove to form the compound of formula *79* which is one of the acylated indole derivative of this invention within formula [I].

The 3-acyl-1-(ω-hydroxyalkyl)indole of formula *75*, a starting compound for production of the compound of formula *76*, is included within the compound *38* of Reaction Scheme 3.

A compound of formula *76* in which $T^1$ is a halogen atom can be prepared from the compound of formula *75* in the same way as in the production of the compound of formula *14* from the compound of formula *13* in accordance with Reaction Scheme 1. A compound of formula *76* in which $T^1$ is $-OSO_2CH_3$ or

$-OSO_2$ 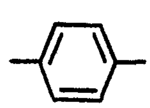 $CH_3$,

can be produced from the compound of formula *75* in the same way as in the production of the compound of formula *74* from the compound of formula *50* in Reaction Scheme 14.

According to Reaction Scheme 15, 3-acyl-1-(ω-mercapto- or acylthio-alkyl)indole derivatives

exemplified by compounds (416) and (422) to (424) given hereinabove, which fall into the acylated indole derivatives of formula [I] of this invention, can be advantageously produced.

[D] Compounds of formula [I] wherein Y is a sulfur atom, and A is an alkyl group, or a substituted or unsubstituted phenyl, phenylalkyl, pyridinyl, imidazolyl or thiadiazolyl group:—

These compounds can be produced in accordance with Reaction Schemes 16 to 18.

(16)

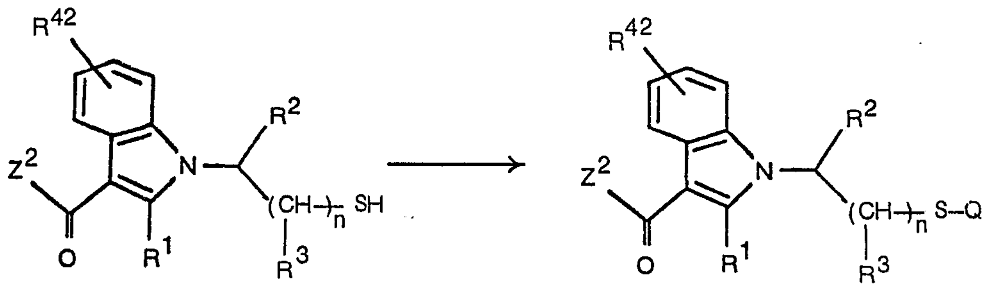

79 80

REACTION SCHEME 16

(17)

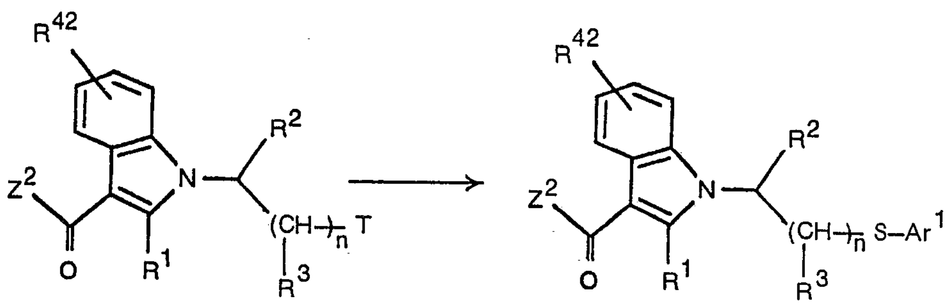

76 81

REACTION SCHEME 17

(18)

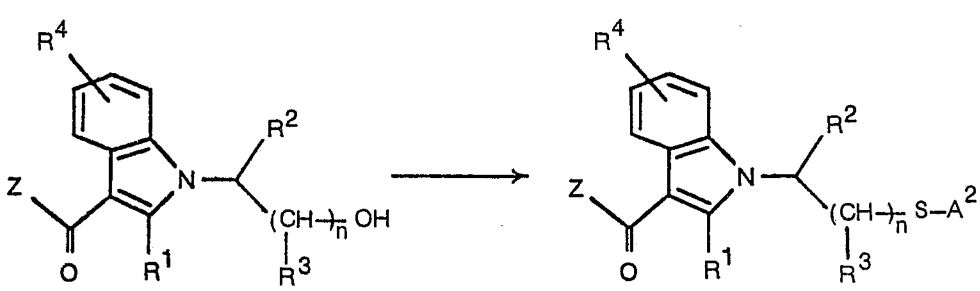

[I]–$a_1$ 82 (≡[I]–d

REACTION SCHEME 18

D—1. Reaction Scheme 16

According to Reaction Scheme 16, the 3-acyl-1-(ω-mercaptoalkyl)indole of formula *79* (obtained in accordance with Reaction Scheme 15) is condensed with a halogen compound of formula [V]

Q—X

wherein Q represents an alkyl group, a phenylalkyl group, or a phenylalkyl group substituted by $R^{42}$, or X represents a halogen atom,

in the presence of a basic compound to give a 3-acyl-1-(ω-thiaalkyl)indole of the following formula *80*

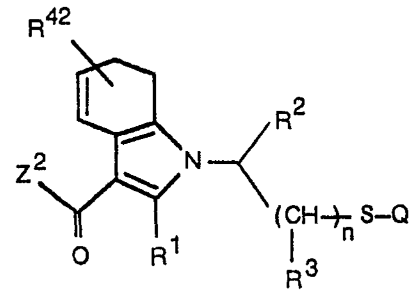

wherein $R^1$, $R^2$, $R^3$, $R^{42}$, n, $Z^1$ and Q are as defined hereinabove.

The above condensation reaction can be performed under the same conditions as described above with regard to Reaction Scheme 5.

According to Reaction Scheme 16, there can be produced acylated indole derivatives of this invention in which the 1-position is replaced by an ω-alkylthioalkyl group or an ω-substituted or unsubstituted phenylthioalkyl or phenylalkylthioalkyl group, as shown by formula *80*.

D—2. Reaction Scheme 17

According to Reaction Scheme 17, the 3-acyl-1-(ω-halogeno- or sulfonyloxy-alkyl)indole of formula *76* (obtained in Reaction Scheme 15) is condensed with the aromatic thiol compound of formula [VI]

$Ar^1$—SH [VI]

in the presence of a basic compound in an inert organic solvent to afford a 3-acyl-1-(ω-arylthiaalkyl)indole of the following formula *81*

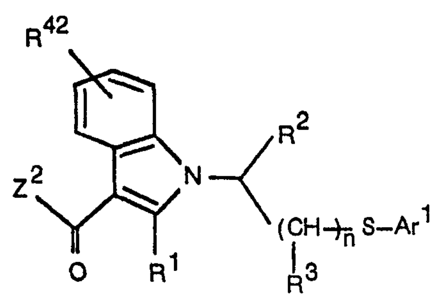

(81)

wherein all symbols are as defined hereinabove.

The condensing reaction can be performed under the same conditions as described with regard to Reaction Scheme 9. In this case, however, pyridine and sodium alkoxides such as sodium methoxide and sodium ethoxide can also be used as the basic compound.

According to Reaction Scheme 17, acylated indole derivatives of this invention in which the 1-position is substituted by an arylthiaalkyl group, as shown by formula *81*, can be obtained.

D—3. Reaction Scheme 18

According to Reaction Scheme 18, the 3-acyl-1-(ω-hydroxyalkyl)indole of formula [I]-$a_1$ (the compound of formula *38* obtained by Reaction Scheme 3) is condensed with a disulfide compound of the following formula [IV]

$A^2$—S—S—$A^2$ [IV]

wherein $A^2$ represents an alkyl group, or a phenyl, phenylalkyl pyridinyl, imidazolyl or thiadiazolyl group, optionally substituted by $R^{42}$,

in pyridine in the presence of a trialkylphosphine to give a 3-acyl-1-thiaalkylindole of the following formula *82*

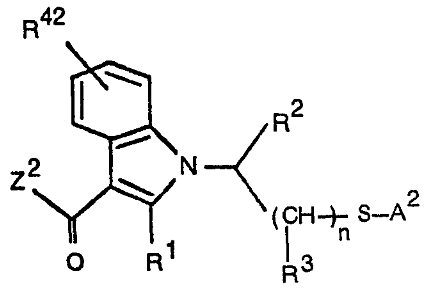

(82)

wherein all symbols are as defined hereinabove.

The condensation reaction is carried out by contacting 1 mole of the compound of formula [I]-a¹ with 1 to 10 moles of the disulfide compound in the presence of 1 to 10 moles of the trialkylphosphine at a temperature of 0°C to 80°C.

Examples of the disulfide compound include di-alkyl disulfides such as dimethyl disulfide, diethyl disulfide, di-n-propyl disulfide, diisopropyl disulfide, di-n-butyl disulfide, di-n-pentyl disulfide and di-n-hexyl disulfide; di(substituted or unsubstituted phenyl) disulfides such as diphenyl disulfide, bis(p-chlorophenyl) disulfide, bis (o-methoxyphenyl) disulfide and di(p-tolyl)disulfide; di(substituted or unsubstituted phenylalkyl) disulfides such as dibenzyl disulfide, bis(p-methoxybenzyl) disulfide, bis(o-chlorobenzyl) disulfide, bis(p-chlorobenzyl) disulfide, di(α-phenethyl) disulfide, bis(p-chloro-α-phenethyl) disulfide, di(β-phenethyl) disulfide and bis(p-methoxy-β-phenethyl) disulfide; and di(substituted or unsubstituted heterocyclic group) disulfides such as di(4-pyridyl) disulfide, di(2-pyridyl) disulfide and di(2-imidazolyl) disulfide.

Preferred trialkylphosphines are those having alkyl groups with 1 to 6 carbon atoms, such as trimethylpophoephoine, triethylphosphine, tri-n-butylphoephine and tri-n-hexylphosphine. Of these, tri-n-butylphosphine is especially preferred.

The reaction is carried out in pyridine as a solvent, and is completed usually in 1 hour to 72 hours.

If desired, the condensation product is hydrolyzed. Thus, compounds of formula *82* in which the group Z and the group $A^2$ are protected hydroxyl groups can be converted to the corresponding compounds in which these groups are converted to hydroxyl groups.

According to Reaction Scheme 18, acylated indole derivatives of this invention can be produced in which the 1-position is substituted by an ω-alkylthioalkyl group, an ω-substituted or unsubstituted phenylthioalkyl or phenylalkylthioalkyl group, or an ω-substituted or unsubstituted heterocyclic thioalkyl group, as shown by formula *82*.

According to Reaction Schemes 16 to 18, 3-acyl-1-thiaalkyl indole derivatives exemplified by compounds (400) to (428) exemplified hereinabove which come within the acylated indole derivatives of formula [I] can be advantageously produced.

[E] Compound of general formula [I] wherein Y represents —SO— or —$SO_2$— and A represents a alkyl group, a substituted or unsubstituted phenyl, phenylalkyl, pyridinyl, imidazolyl or thiadiazolyl group:—

These compounds can be produced in accordance with Reaction Schemes 19 and 20.

(19)

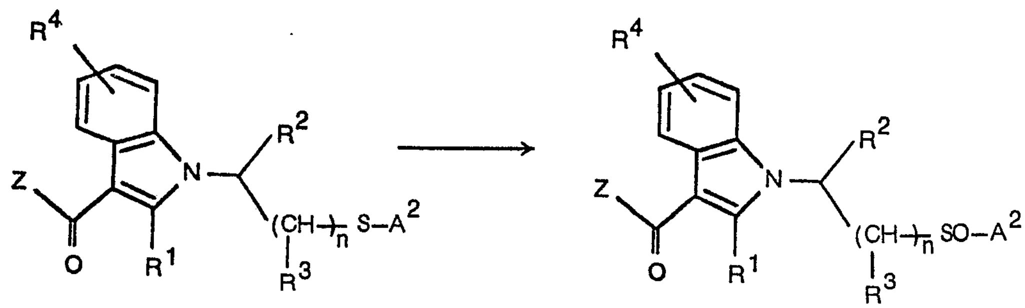

82 83

REACTION SCHEME 19

(20)

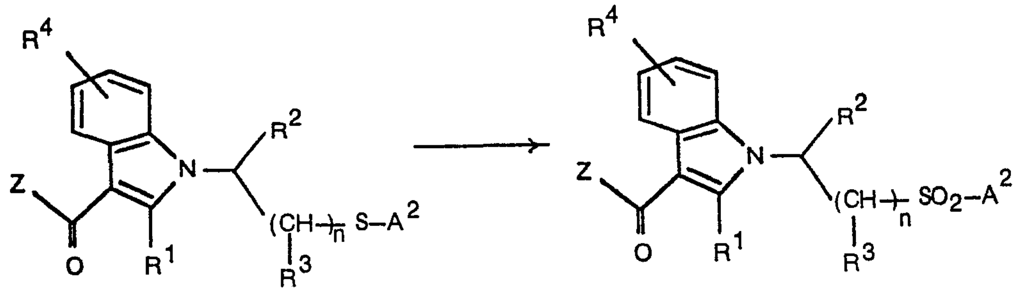

82 84

REACTION SCHEME 20

E—1. Reaction Scheme 19

According to Reaction Scheme 19, the 3-acyl-1-4-thiaalkylindole of formula *82* (obtained in accordance with Reaction Scheme 18) is reacted with sodium perchlorate or sodium periodate in a solvent such as water, methanol, ethanol, THF or dioxane at a temperature of −50°C to +50°C for a period of 30 minutes to 24 hours (first reaction), or by reacting 1 mole of the compound of formula *82* with about 1 mole 0.9 to 1.2 moles) of an oxidizing agent such as hydrogen peroxide, peracetic acid, performic acid, perbenzoic acid, m-chloroperbenzoic acid, isobutyl hydroperoxide, sec-butyl hydroperoxide, t-butyl hydroperoxide, N-bromosuccinimide (NBS) or N-chlorosuccinimide (NCS) in a solvent such as acetic acid, methylene chloride, chloroform, 1,2-dichloroethane or benzene at a temperature of −50°C to +50°C for 30 minutes to 24 hours (second reaction), to give a 3-acyl-1-oxythiaalkylindole of the following formula *83*

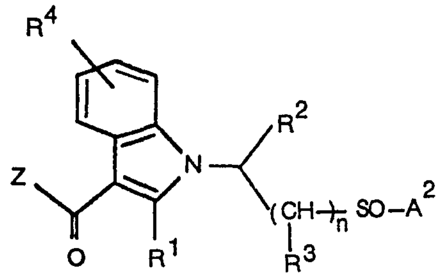

(83)

wherein all symbols are as defined hereinabove.

According to Reaction Scheme 19, 3-acyl-1-oxythiaalkylindole derivatives exemplified by compounds (500) to (514) given hereinabove, which come within the definition of the acylated indole derivatives of formula [I] of this invention, can be advantageously produced.

E—2. Reaction Scheme 20

According to Reaction Scheme 20, 1 mole of the 3-acyl-1-thialkylindole of formula 82 is reacted with at least 2 moles, preferably 2 to 3 moles of the oxidizing agent used in the first reaction of Reaction Scheme 19 under the same conditions as in the second reaction of Reaction Scheme 18 to afford a 3-acyl-1-dioxythiaalkylindole of the following formula *84*

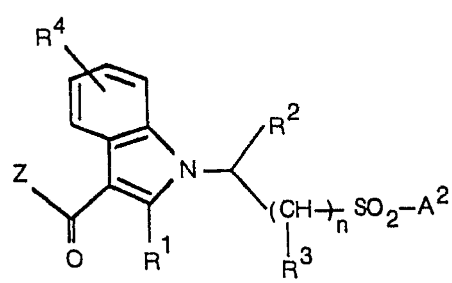

(84)

wherein all symbols are as defined hereinabove.

According to Reaction Scheme 20, 3-acyl-1-dioxythiaalkylindole derivatives exemplified by compounds (600) to (614) given hereinabove, which come within the acylated indole derivatives of formula [I] of this invention, can be advantageously produced.

[F] Compounds of general formula [I] wherein Y is a bond and A is an alkyl group.

These compounds can be produced in accordance with Reaction Schemes 21 to 23 below.

(21)

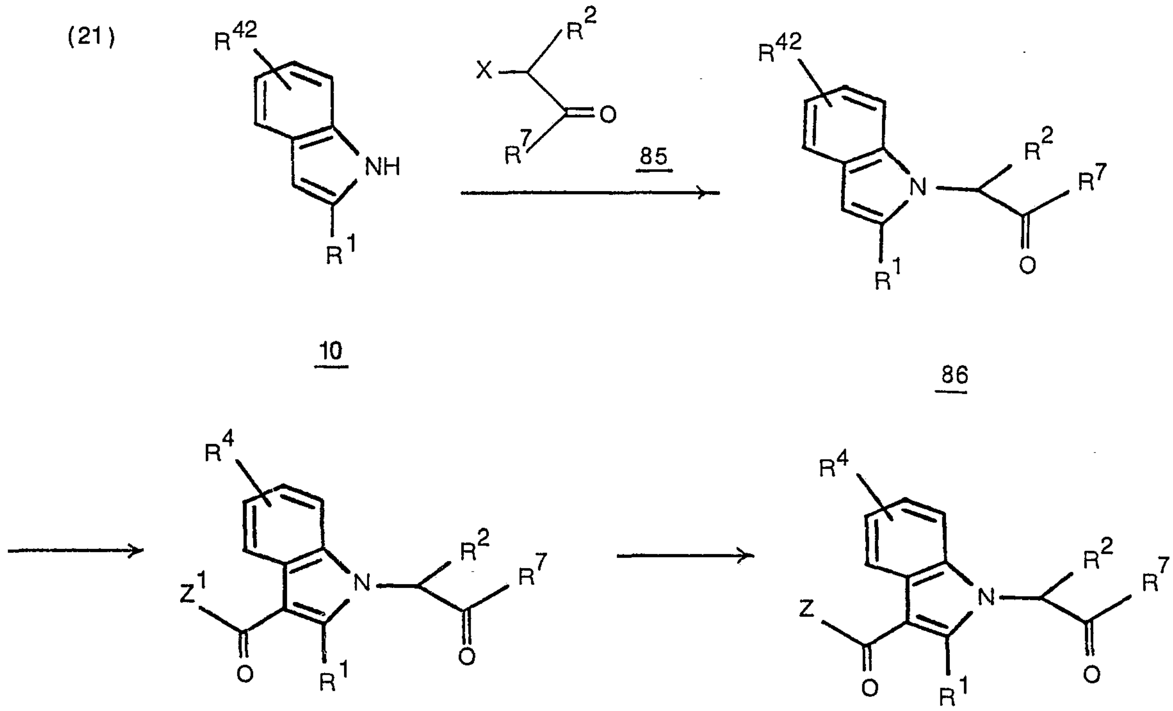

85

10

86

87

88

REACTION SCHEME 21

(22)

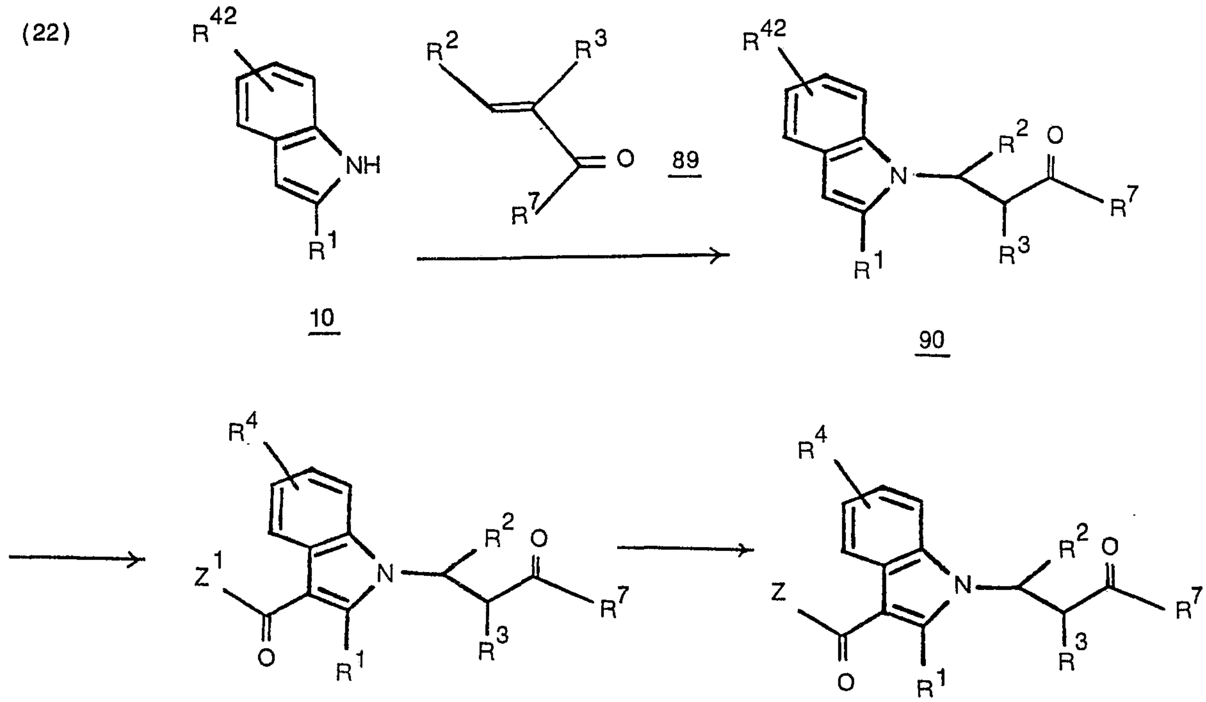

89

10

90

91

92

REACTION SCHEME 22

(23)

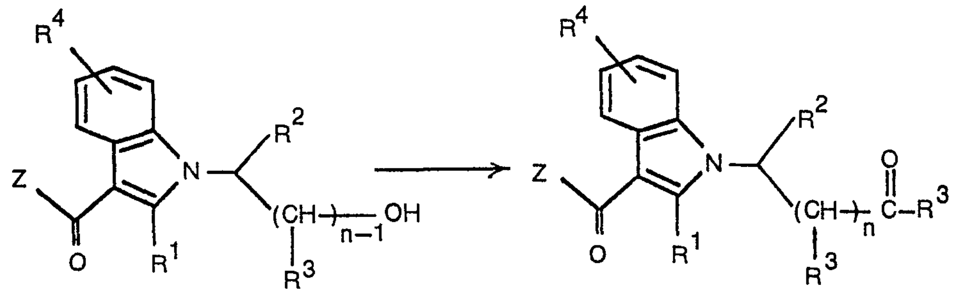

93

94

REACTION SCHEME 23

(24)

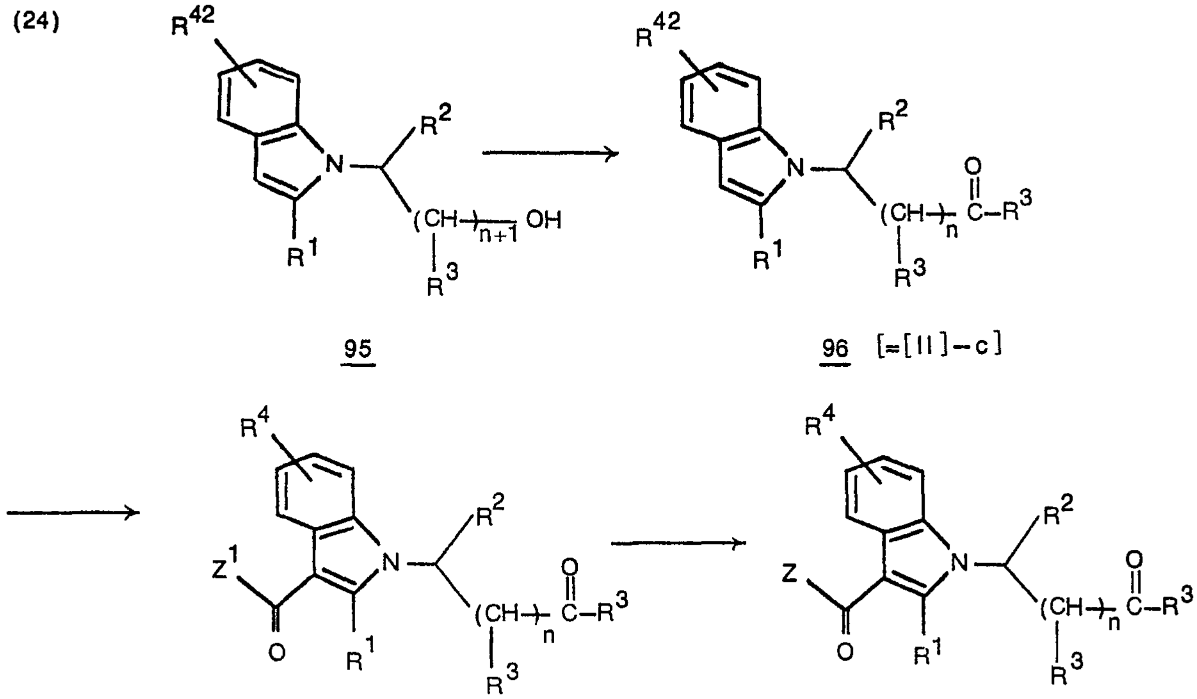

95

96 [≡[II]−c]

97

98 [≡[I]−c]

REACTION SCHEME 24

F—1. Reaction Scheme 21

According to Reaction Scheme 21. The indole of formula 10 is reacted with an α-haloketone of formula *85* (wherein $R^2$ is as defined hereinabove, and $R^7$ represents an alkyl group having 1 to 6 carbon atoms) under the same conditions as in the production of the compound of formula *12* from the compounds of formulae *10* and *11* in Reaction Scheme 1 to give a 1-lower acylmethylindole of the following formula *86*

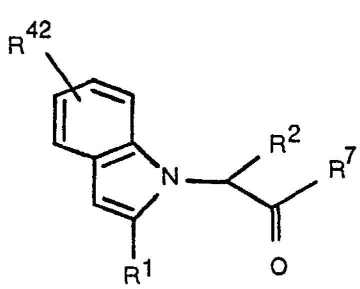

(86)

wherein all symbols are as defined hereinabove.

The compound of formula *86* is then acylated with the anhydride [III] in the same way as in Reaction Scheme 1, and if desired, hydrolyzed to afford a 3-acyl-1-acylmethylindole of the following formula *87*

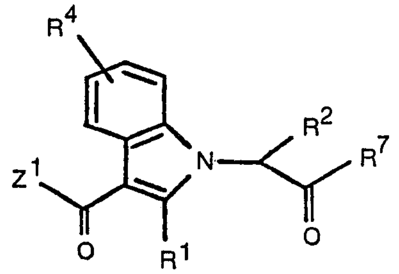

(87)

wherein all symbols are as defined hereinabove
or the hydrolysis product of the compound of formula *87* which is expressed by the following formula *88*

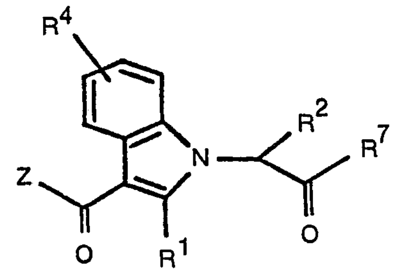

(88)

wherein all symbols are as defined hereinabove.

F—2. Reaction Scheme 22

According to Reaction Scheme 22, the indole of formula *10* is reacted with an $\alpha,\beta$-unsaturated ketone of formula *89* (wherein $R^2$, $R^3$ and $R^7$ are as defined hereinabove) under the same conditions as in the production of the compound of formula *24* from the compounds of formulae *10* and *23* in Reaction Scheme 2, to afford 1-acylethylindole of the following formula *90*

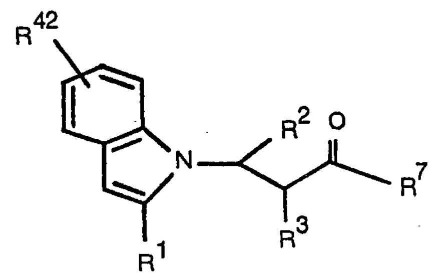

(90)

wherein all symbols are defined hereinabove.

The compound of formula 90 is then acylated with the acid anhydride [III] in the same way as in Reaction Scheme 2, and then hydrolyzed to give a 3-acyl-1-acylethylindole of the following formula *91*

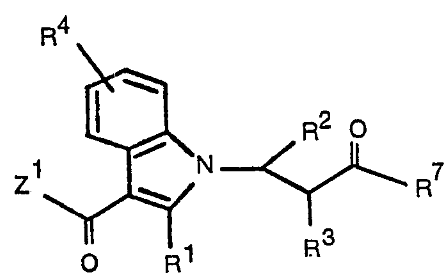

(91)

wherein all symbols are as defined above,

or the hydrolysis product of the compound of formula *91*, which is expressed by the following formula *92*

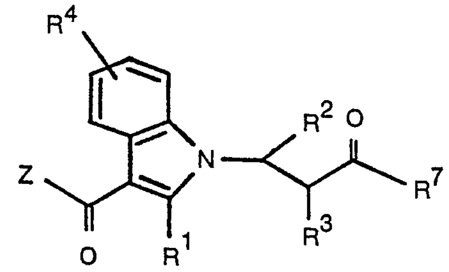

(92)

wherein all symbols are as defined hereinabove.

F—3. Reaction Scheme 23

According to Reaction Scheme 23, a 3-acyl-1-($\omega$-acylalkyl)indole of the following formula *94*

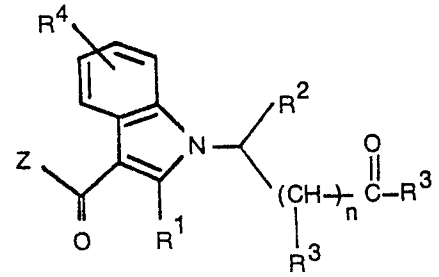

(94)

wherein all symbols are as defined hereinabove, can be produced either by oxidizing a 3-acyl-1-($\omega$-hydroxyalkyl)indole of the following formula *93*

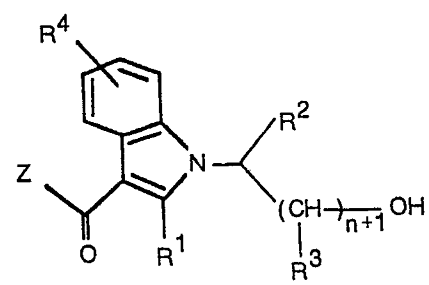

(93)

wherein all symbols are as defined hereinabove, with a chromic acid-type complex such as chromic acid-pyridine complex (Collin's reagent), pyridine-dichromate (PDC) or pyridinium chlorochromate (PDC) in an inert organic solvent such as ether, THF, methylene chloride, chloroform, carbon tetrachloride or acetone at a temperature of 0°C to 50°C, or by oxidizing the compound of formula *93* with dimethylsulfoxide in the presence of one of dicyclohexylcarbodiimide, oxalyl chloride, thionyl chloride and acetyl chloride and one primary amine such as pyridine, s-collidine and triethylamine at a temperature of −78°C to 20°C (Moffat Oxidation Reaction).

The compound of formula *93*, a starting material in Reaction Scheme 23, can be prepared in the same way as in Reaction Scheme 3 from a compound of formula *34* in which *n* is changed to *n* + 1.

F—4. Reaction Scheme 24

According to Reaction Scheme 24, a 1-($\omega$-hydroxyalkyl)-3-unsubstituted indole of the following formula *95*

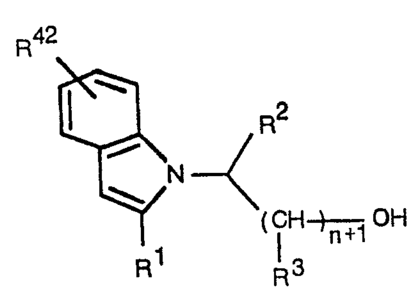

(95)

wherein all symbols are as defined hereinabove, is oxidized under the same conditions as in the

production of the compound of formula *94* from the compound of formula *93* in Reaction Scheme 23, to give a 1-($\omega$-acylalkyl)indole of the following formula *96*

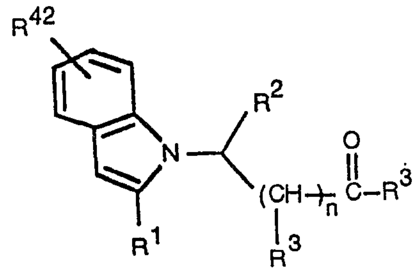

(96)

wherein all symbols are as defined hereinabove.

The compound of formula *96* is reacted with the acid anhydride of formula [III] in the presence of hydrogen iodide under the same conditions as in the production of the compound of formula *21* from the compound of formula *20* to give a compound of formula *97*. Hydrolysis of the compound *97* gives a compound of formula *98*.

According to Reaction Schemes 21 to 24, 3-acyl-1-($\omega$-acylalkyl)indole derivatives exemplified by compounds (800) to (808) given hereinabove, which come within the definition of the acylated indole derivatives of formula [I] of this invention, can be advantageously produced.

The acylated indole derivatives of formula [I] provided by the present invention have a characteristic pharmacoligical action in that they have excellent ability to inhibit platelet aggregation with reduced tendency toward inducing ulcer formation.

Thus, according to this invention, there is provided a composition having the ability to inhibit platelet aggregation comprising a pharmaceutically effective amount of a acylated indole derivative of the following formula [I]-a

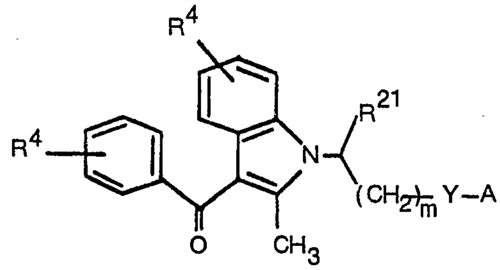

[I]-1

wherein $R^{21}$ represents an alkyl group having 1 to 3 carbon atoms, two groups $R^4$ are identical or different and each represents hydrogen, hydroxyl, halogen, alkyl or alkoxy, Y represents a bond, —O—, —S—, —SO— or —$SO_2$, A represents hydrogen, alkyl, acyl, phenyl, phenylalkyl, pyridinyl, imidazolyl or thiadiazolyl, or phenyl, phenylalkyl, pyridinyl, imidazolyl or thiadiazolyl substituted by $R^4$, and m is zero or an integer of 1 to 3 provided that when m is 0, Y represents a bond and A represents acyl

or an optically active isomer or salt thereof, and a pharmaceutically acceptable carrier, or said composition in unit dosage form.

In a preferred aspect, the present invention also provides a composition having the ability to inhibit platelet aggregation comprising a pharmaceutically effective amount of an acylated indole derivative of the following formula [I]-b

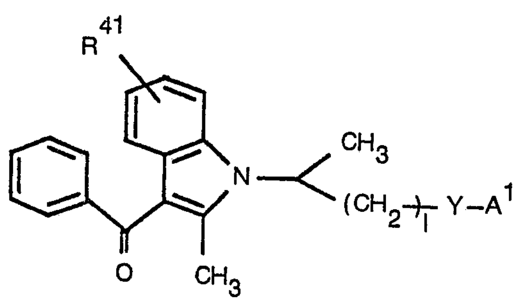

[I]-2

wherein $R^{41}$ represents hydrogen, hydroxyl, chlorine, methyl or methoxy, Y is as defined in formula (I)—1, $A^1$ represents hydrogen, alkyl of 1 to 4 carbon atoms, acyl, of 1 to 4 carbon atoms, phenyl, pyridinyl, imidazolyl or thiadiazolyl, and l is 0, 1 or 2 provided that when l is 0, Y represents a bond and A′ represents acyl.

or an optically active isomer or salt thereof, and a pharmaceutically acceptable carrier, or said composition in unit dosage form.

Usually, the acylated indole derivative of this invention is administered perorally or parenterally (e.g., intrarectally, subcutaneously, intramuscularly, etc.) as the aforesaid composition or a drug in unit dosage form composed of the aforesaid composition. Preferably, it is administered peroally or intrarectally, and this is convenient to patients.

For oral administration, the composition is formulated into a solid or liquid preparation. Examples of the solid preparation are tablets, pills, powders or granules. In such a solid preparation, at least one active substance (acylated indole derivative of the invention) is mixed with at least one inert diluent such as calcium carbonate, potato starch, alginic acid or lactic acid. The mixture is formulated in a customary manner. Additives other than diluents, such as lubricants (e.g., magnesium stearate), may also be incorporated.

Examples of the liquid preparation for oral administration are an emulsion, a solution, a suspension, a syrup and an alixir. These liquid preparations contain common inert diluents such as water or liquid paraffin.

These liquid preparations may also contain other auxiliary agents such as wetting agents, suspending aids, sweetenings, flavoring agents, aromas, and antiseptics.

The liquid preparations may be made into capsules using an absorbent substance such as gelatin.

A solid preparation for intrarectal administration may, for example, be suppository containing at least one active substance and prepared by a method known *per se.*

Preparations for parenteral administration are, for example, aseptic aqueous or non-aqueous solutions, suspensions, and emulsions. Vegetable oils such as olive oils, propylene glycol, polyethylene glycol, and injectable organic esters such as ethyl oleate may, for example, be used as a non-aqueous solvent or suspending agent. These preparations for parenteral administration may also contain auxiliary agents such as antiseptics, wetting agents, emulsifiers and dispersing agents. They can be sterilized by, for example, filtration on a bacterial-holding filter, incorporation of a germicides, or by irradiation. Or an aseptic solid preparation may be produced and immediately before use, may be dissolved in aseptic water or aseptic solvents for injection.

The acylated indole derivative of this invention can be converted into an inclusion compound with cyclodextrin. Such an inclusion compound has the advantage that a solid prepration containing such an inclusion compound, when administered orally to a warm-blooded animal, has increased dispersibility in the stomach and increased absorption, and that the inclusion compound is easy to form into a solid preparation.

The cyclodextrin includes $\alpha$-, $\beta$- and $\gamma$-cyclodextrins, and the $\beta$-cyclodextrin is preferred.

The inclusion compound of the acylated indole derivative of this invention with cyclodextrin can be prepared, or for example, by a co-precipitation method which comprises forming an aqueous solution of $\beta$-cyclodextrin, adding the acylated indole derivative, stirring the mixture, and if required cooling the mixture to precipitate an inclusion compound; a solvent evaporating method which comprises adding a solution of the acylated indole derivative in an organic solvent such as ethyl ether to an aqueous solution of $\alpha$-cyclodextrin, stirring the mixture, and then distilling off the organic solvent; or a kneading method which comprises forming an aqueous slurry of $\beta$-cyclodextrin, adding the acylated indole derivative or a solution of it in an organic solvent, and kneading the mixture by a ball mill, grinding machine, etc.

The resulting inclusion compound can be directly used as a powdery drug. Or it can be formulated into tablets, granules, capsules, syrups, etc. by adding required amounts of excipients, binders, disintegrating agents, luster-imparting agents, corrigents, smell modifiers, coloring agents, etc.

Since the acylated indole derivative of this invention have the excellent pharmacological activities, it can be administered to warm-blooded animals including man when it is desired to inhibit platelet aggregation or inhibit or prevent thrombus formation.

For example, the acylated indole derivatives of this invention are useful for prevention of infarction of a cardiovascular system, prevention of post-operative thrombosis, promotion of patency of a vascular prosthesis after surgical operation, and for prevention and treatment of atherosclerosis and arteriosclerosis.

They are also used to prevent onset of cerebral ischemia in patients of senile diseases, and to prevent or treat myocardial infarction and a poplexy for a long period of time after their seizure.

The present invention also provides a method for inhibiting platelet aggregation of a warm-blooded animal, which comprises administering an amount effective for inhibition of platelet aggregation of the acylated indole derivative of formula [I]-a or an optionally active isomer or salt thereof to said warm-blooded animals.

The dosage of the acylated indole derivative of this invention is 0.0005 to 20 mg, preferably 0.01 to 10 mg, per kg of body weight per day. The dosage, however, is affected by the condition, body weight and age of a patient and the route of administration.

The acylated indole derivative of this invention may also be used as an additive for fluids used in artificial circulation and perfusion outside the body of the blood, blood products, substitute blood, or those in separated parts of a body (for example, limbs or organs either adhering to the parent body,

stored after separation, or provided for transplantation, or adhering to a new body). Platelets clotted in such a circulating or perfusing fluids tend to clog vessels and circulating organs. This clogging can be avoided by the presence of the acylated indole derivative of this invention. For this purpose, the acylated indole derivative may be added gradually or at a time or in several portions to the circulating blood, the blood of a donor animal, parts of the body under perfusion (separated from or adhering to the recipient), or to two or all of these.

The following Examples illustrate the present invention more specifically.

Example 1

Synthesis of 1-(1-acetoxy-2-propyl)-3-benzoyl-2-methylindole (No. 200):—

(1) 2-Acetonylcyclohexanone (4.62 g; 30 millimoles), 4.60 g (30 millimoles) of alanine ethyl ether hydrochloride and 2.46 g (30 millimoles) of sodium acetate were suspended in 20 ml of acetic acid, and the suspension was heated under reflux for 3 hours. After the reaction, the acetic acid was distilled off under reduced pressure. The resulting reaction product was dissolved in 200 ml of ethyl acetate, and washed with 100 ml of a saturated aqueous solution of sodium bicarbonate twice, 100 ml of water and then 100 ml of a saturated aqueous solution of sodium chloride. The organic layer was dried over anhydrous sodium sulfate, and the solvent was distilled off under reduced pressure. The resulting oily product was chromatographed on a silica gel column using benzene as an eluent to afford 3.0 g (yield 42%) of ethyl 2-(4,5,6,7-tetrahydro-2-methylindol-1-yl)propionate.

NMR($CDCl_3$, $\delta$ (ppm);:

1.19 (3H, t, J=7 Hz), 1.55 (3H, d, J=7 Hz), 1.6 (4H, m), 2.13 (3H, s), 2.4 (4H, m), 4.17 (2H, q, J=7 Hz), 4.73 (1H, q, J=7Hz), 5.65 (1H, s).

(2) A solution of 3.50 g (14.9 millimoles) of the ethyl 2-(4,5,6,7-tetrahydro-2-methylindol-1-yl)-propionate obtained in (1) above in 20 ml of anhydrous ether was added dropwise to a stirred suspension of 566 mg (14.9 millimoles) of lithium aluminum hydride in 30 ml of anhydrous ether. The mixture was stirred at room temperature for 16 hours, and a saturated aqueous solution of anhydrous sodium sulfate was added to decompose the excess of lithium aluminum hydride and the resulting aluminum compound. The ethereal layer was washed with a saturated aqueous solution of sodium chloride, and dried over anhydrous sodium sulfate. The solvent was distilled off under reduced pressure. The resulting oily product was dissolved in 10 ml of acetic anhydride and 10 ml of pyridine, and the mixture was stirred at room temperature for 18 hours. The pyridine and acetic anhydride were distilled off under reduced pressure, and the reaction product was dissolved in 400 ml of ether. The ethereal layer was washed successively with 100 ml of 1N hydrochloric acid, 100 ml of a saturated aqueous solution of sodium bicarbonate, 100 ml of water, and 100 ml of a saturated aqueous solution of sodium chloride. On distilling off the solvent under reduced pressure, 1 - (1 - acetoxy - 2 - propyl) - 4,5,6,7 - tetrahydro - 2 - methylindole (yield 91%) was obtained.

NMR($CDCl_3$, $\delta$ (ppm)):

1.48 (2H, d, J=7Hz), 1.75 (4H, m), 1.97 (3H, s), 2.20 (3H, s), 2.55 (4H, m), 4.3 (3H, m), 5.62 (1H, s).

(3) A mixture of 23.2 g (0.099 mole) of the 1 - (1 - acetoxy - 2 - propyl) - 4,5,6,7 - tetrahydro - 2 - methylindole obtained in (2) above, 44.8 g (0.198 mole) of benzoic anhydride and 1 ml of 52% hydriodic acid was stirred at 145°C for 2 hours in a stream of nitrogen with stirring. The reaction mixture was allowed to cool to room temperature, and dissolved in 300 ml of ethyl acetate. The solution was washed successively with 100 ml of 1N aqueous sodium hydroxide solution, 100 ml of water and 100 ml of a saturated aqueous solution of sodium chloride. The organic layer was dried over anhydrous sodium sulfate, and and the solvent was distilled off under reduced pressure. The resulting blackish brown oily product was chromatographed on a silica gel column using benzene/ethyl acetate (19/1) as an eluent to afford 29.6 g (yield 88%) of 1 - (1 - acetoxy - 2 - propyl) - 3 - benzoyl - 4,5,6,7 - tetrahydro - 2 - methylindole as an oil.

NMR($CDCl_3$, $\delta$ (ppm)):

1.45 (3H, d, J=8Hz), 1.6 (4H, m), 1.94 (3H, s), 2.2 (2H, m), 2.24 (3H, s), 2.6 (2H, m), 4.2—4.7 (3H, m), 7.3—7.6 (3H, m), 7.6—7.8 (2H, m).

(4) 2.69 g (7.9 millimoles) of the 1 - (1 - acetoxy - 2 - propyl) - 3 - benzoyl - 4,5,6,7 - tetrahydro - 2 - methylindole obtained in (3) above, and 4.32 g (18.9 millimoles) of 2,3 - dichloro- - 5,6 - dicyanobenzo - 1,4 - quinone were dissolved in 50 ml of dioxane, and the solution was heated under reflux for 4 hours. The dioxane was distilled off under reduced pressure, and 400 ml of ethyl acetate was added to the residue to dissolve the reaction product. The solution was filtered. The filtrate was washed with 200 ml of water, 100 ml of 1N aqueous sodium hydroxide solution, 200 ml of water and 100 ml of a saturated aqueous solution of sodium chloride, followed by drying over anhydrous magnesium sulfate. The solvent was distilled off under reduced pressure. The resulting blackish brown

oily product was chromatographed on a column of silica gel using benzene/acetic acid (20/1) as an eluent to afford 1.44 g (yield 52%) of the desired compound (No. 200) as an oil.

$IR(CHCl_3)cm^{-1}$:

1736, 1625, 1521, 1461, 1613, 1382, 1230, 1174, 1050.

$NMR(CDCl_3m\ \delta$ (ppm)):

1.63 (3H, d, J=8Hz), 1.87 (3H, s), 2.52 (3H, s), 4.3—4.6 (2H, m), 4.6—5.2 (1H, m), 6.8—7.8 (9H, m).

Example 2

Synthesis of 3-benzoyl-1-(1-hydroxy-2-propyl)-2-methylindole (No. 100):—

In a mixture of 10 ml of methanol and 5 ml of a 1N aqueous solution of sodium hydroxide was dissolved 597 mg (1.78 millimoles) of the compound (No. 200) obtained in Example 1. The solution was stirred at room temperature for 3 hours. Methanol was distilled off under reduced pressure, and the residue was dissolved in 50 ml of ethyl acetate. The solution was washed with 50 ml of water and 50 ml of a saturated aqueous solution of sodium chloride. The product was dried over anhydrous sodium sulfate, and the solvent was distilled off under reduced pressure to afford 520 mg (yield 100%) of the desired compound (No. 100) as an oil.

$IR(CHCl_3)cm^{-1}$:

3400, 1620, 1576, 1513, 1462, 1412, 1390, 1273, 1230, 1163, 1056, 1131, 899.

$NMR(CDCl_3,\ \delta$ (ppm)):

1.60 (3H, d, J=8Hz), 2.47 (3H, s), 2.60 (1H, br), 3.6—4.2 (2H, m), 4.2—4.8 (1H, m), 6.9—7.8 (9H, m).

Example 3

Synthesis of 3-benzoyl-1-(1-ethoxy-2-propyl)-2-methylindole (No. 302):—

447 mg of the compound (No. 100) obtained in Example 2 was dissolved in 20 ml of dimethoxyethane. While the solution was cooled with ice with stirring, 90 mg (1.2 equivalents) of sodium hydride was added, followed by addition of 0.5 ml of ethyl bromide. The mixture was stirred overnight. The dimethoxyethane was distilled off under reduced pressure, and the reaction product was dissolved in 50 ml of ethyl acetate. The solution was washed first with 50 ml of water and then with 50 ml of a saturated aqueous solution of sodium chloride, followed by drying over anhydrous sodium sulfate. The solvent was distilled under reduced pressure, and the resulting oily product was chromatographed on a silica gel column using benzene/ethyl acetate (20/1) as an eluent to afford 270 mg (yield 56%) of the desired compound (No. 302) as an oil.

$IR(CHCl_3)cm^{-1}$:

2960, 2850, 1620, 1572, 1516, 1459, 1445, 1407, 1381, 1254, 1268, 1210—1230, 1168, 1100, 1070, 1025, 896, 696.

$NMR(CDCl_3,\ \delta$ (ppm)):

1.03 (3H, t, J=6.5), 1.60 (3H, d, J=7), 253 (3H, s), 3.1—3.6 (2H, m), 3.7—4.2 (2H, m), 4.67 (1H, sextet), 6.9—7.9 (9H, m).

Example 4

Synthesis of 3-benzoyl-1-(1-methoxy-2-propyl)-2-methylindole (No. 300):—

The compound (No. 100) obtained in Example 2 was reacted with methyl iodide in the same way as in Example 3 to afford the desired compound (No. 300).

$IR(CHCl_3)cm^{-1}$:

2950, 2900, 1620, 1572, 1515, 1450, 1403, 1374, 1267, 1225, 1200, 1165, 1108, 1036, 895, 744.

$NMR(CDCl_3,\ \delta$ (ppm)):

1.60 (3H, d, J=7 Hz), 2.55 (3H, s), 3.23 (3H, s), 3.6—4.1 (2H, m), 4.5—5.1 (1H, m), 6.9—7.9 (9H, m).

Example 5

Synthesis of 3-benzoyl-1-(1-isopropoxy-2-propyl)-2-methylindole (No. 305):—

The compound (No. 100) obtained in Example 2 was reacted with 2-bromopropane in the same way as in Example 3 to afford the desired compound (No. 305) in a yield of 90%.

$NMR(CDCl_3,\ \delta$ (ppm)):

0.92 (3H, d, J=6 Hz), 1.04 (3H, d, J=6 Hz), 1.61 (3H, d, J=6 Hz), 2.51 (3H, s), 3.37 (1H, sextet, J=6 Hz), 3.82 (2H, bd, J=6 Hz), 4.68 (1H, sextet, J=6 Hz), 6.8—7.6 (7H, m), 7.6—7.9 (2H, m).

$IR(CHCl_3)cm^{-1}$:

2960, 1620, 1515, 1450, 1405, 1220, 1160, 1070, 1025, 920, 890, 740.

Example 6

Synthesis of 3-benzoyl-1-(1-heptyloxy-2-propyl)-2-methylindole (No. 325):—

The compound (No. 100) obtained in Example 2 was reacted with n-heptyl bromide in the same way as in Example 3 to afford the desired compound (No. 325) in a yield of 42%.

NMR($CDCl_3$, $\delta$ (ppm)):

0.83 (br, t), 1.0—1.5 (10H, br, s), 1.67 (3H, d, J=7 Hz), 2.58 (3H, s), 3.2—3.5 (2H, m), 3.8—4.1 (2H, m), 4.75 (1H, sextet, J=7 Hz), 7.0—7.95 (9H, m).

IR($CHCl_3$)$cm^{-1}$:

2950, 2900, 1620, 1405, 900.

Example 7

Synthesis of 1-[1-acetoxy-2(R)-propyl]-3-benzoyl-2-methylindole (No. 217):—

The same reaction as in Example 1 was performed using D-alanine ethyl ester hydrochloride ($[\alpha]_D^{25}$=—3.1°, c=1.00; methanol) to afford the desired compound (No. 217).

$[\alpha]_D^{19.2}$=—9.6°, c=0.91; methanol.

Example 8

Synthesis of 1-[1-acetoxy-2-(S)-propyl]-3-benzoyl-2-methylindole (No. 218):—

The same reaction as in Example 1 was performed using L-alanine ethyl ester hydrochloride ($[\alpha]_D^{25}$=3.8°, c=1.05; methanol) to afford the desired compound (No. 218).

$[\alpha]_D^{19.2}$=10.7°, c=0.80; methanol.

Example 9

Synthesis of 3-benzoyl-1-[1-hydroxy-2(R)-propyl]-2-methylindole (No. 135):—

The compound (No. 217) obtained in Example 7 was hydrolyzed to afford the desired compound (No. 135).

$[\alpha]_D^{19.2}$=—20.0°, c=0.73; methanol.

IR(KBr)$cm^{-1}$:

3420, 1598, 1585, 1563, 1548, 1415, 1167, 1062, 867.

NMR($CDCl_3$, $\delta$ (ppm)):

1.60 (3H, d, J=7Hz), 2.51 (3H, s), 3.6—4.3 (2H, m), 4.3—4.9 (1H, m), 6.9—7.8 (9H, m).

Example 10

Synthesis of 3-benzoyl-1-[1-hydroxy-2(S)-propyl]-2-methylindole (No. 136):—

The compound (No. 218) obtained in Example 8 was hydrolyzed to afford the desired compound (No. 136).

$[\alpha]_D^{19.2}$=+19.6°, c=0.63; methanol.

IR(KBr)$cm^{-1}$:

3425, 1600, 1584, 1563, 1530, 1416, 1168, 1063, 863.

NMR($CDCl_3$, $\delta$ (ppm)):

1.60 (3H, d, J=7 Hz), 2.51 (3H, s), 3.6—4.3 (2H, m), 4.3—4.9 (1H, m), 6.9—7.8 (9H, m).

Example 11

Synthesis of 3-benzoyl-1-[1-methoxy-2(R)-propyl]-2-methylindole (No. 346):—

The compound (No. 135) obtained in Example 9 was reacted with methyl iodide to afford the desired compound (No. 346).

$[\alpha]_D^{19.2}$=+2.5°, c=0.84; methanol.

IR(liquid film)$cm^{-1}$:

3000, 1620, 1520, 1460, 1405, 1170, 1105, 900.

NMR($CDCl_3$ $\delta$ (ppm)):

1.62 (3H, d, J=7 Hz), 2.51 (3H, s), 3.21 (3H, s), 3.3—4.0 (2H, m), 4.5—5.0 (1H, m), 6.8—7.9 (9H, m).

Example 12

Synthesis of 3-benzoyl-1-[1-methoxy-2-(S)-propyl]-2-methylindole (No. 347):—

The compound (No. 136) obtained in Example 10 was reacted with methyl iodide to afford the desired compound (No. 347).

$[\alpha]_D^{19.2}$=—1.6°, c=0.73; methanol.

IR($CHCl_3$)$cm^{-1}$:

3000, 1620, 1520, 1460, 1405, 1105, 900.

NMR($CDCl_3$, $\delta$ (ppm)):

1.60 (3H, d, J=6 Hz), 2.51 (3H, s), 3.21 (3H, s), 3.3—3.9 (2H, m), 4.5—5.0 (1H, m), 6.9—7.9 (9H, m).

Example 13

Synthesis of 3-benzoyl-1-(1-hydroxy-2-propyl)indole (no. 111), and 1-(1-acetoxy-2-propyl)-3-benzoylindole (No. 202):—

(1) 0.6 g of sodium hydride (50%, mineral oil) was suspended in 5 ml of dimethylformamide. A solution of 1.17 g (10 millimoles) of indole in 10 ml of dimethylformamide was added dropwise to the suspension. After generation of hydrogen ceased, 1.6 ml of ethyl $\alpha$-bromopropionate was added dropwise, and reacted at room temperature for 5 hours. After the reaction, 100 ml of water was added to the reaction mixture, and the mixture was extracted with 100 ml of ethyl acetate. The organic layer was washed three times with 100 ml of water, and dried over anhydrous sodium sulfate. The solvent was distilled off under reduced pressure to afford an oily product.

NMR($CDCl_3$, $\delta$ (ppm)):

1.12 (3H, t, J=7 Hz), 1.72 (3H, d, J=7 Hz), 4.13 (2H, q, J=7 Hz), 5.05 (1H, q, J=7 Hz), 4.87 (1H, d, J=3 Hz), 6.95—7.40 (4H, m), 7.55—7.75 (1H, m).

(2) The resulting product ethyl 2-(indol-1-yl)propionate was reduced with 0.4 g of lithium aluminum hydride in anhydrous ether. The product was acetylated with 5 ml of acetic anhydride and 10 ml of pyridine to afford 1.69 g (yield 77%) of 1-(1-acetoxy-2-propyl)indole.

NMR($CDCl_3$, $\delta$ (ppm)):

1.50 (3H, d, J=7 Hz), 1.85 (3H, s), 4.08 (2H, m), 4.77 (1H, sextet, J=7 Hz), 6.52 (1H, d, J=3 Hz), 6.9—7.4 (4H, m), 7.5—7.75 (1H, m).

(3) A mixture of 1.51 (7 millimoles) of the product obtained in (2) above, 3.14 g (14 millimoles) of benzoic anhydride and 0.2 ml of 52% hydriodic acid was stirred at 140°C for 1.5 hours. After the reaction, the reaction mixture was dissolved in 100 ml of ethyl acetate, and successively washed with 100 ml of a saturated aqueous solution of sodium bicarbonate, 100 ml of water and 100 ml of a saturated aqueous solution of sodium chloride. The product was dried over anhydrous sodium sulfate, and the solvent was distilled off under reduced pressure. The resulting oily product was chromatographed on a silica gel column using benzene/ethyl acetate (25/1) as an eluent to afford 0.8 g (yield 37%) of 1-(1-acetoxy-2-propyl)-3-benzoylindole (No. 202).

NMR($CDCl_3$, $\delta$ (ppm)):

1.50 (3H, d, J=7 Hz), 1.83 (3H, s), 4.0—4.4 (2H, m), 4.4—5.1 (1H, m), 7.2—7.8 (9H, m), 8.35—8.6 (1H, m).

(4) 0.8 g (2.5 millimoles) of the product obtained in (3) above was hydrolyzed with 5 ml of a 1N aqueous solution of sodium hydroxide in 10 ml of methanol to afford the desired compound (No. 111) in an amount of 595 mg (yield 85%).

IR($CHCl_3$)$cm^{-1}$:

3360, 2970, 1605, 1570, 1481, 1460, 1407, 1382, 1306, 1230—1210, 1177, 1049, 875.

NMR($CDCl_3$, $\delta$ (ppm)):

1.43 (3H, d, J=7Hz), 3.2 (1H, brs), 3.73 (2H, br, s), 4.3—4.7 (1H, m), 7.1—7.8 (9H, m), 8.1—8.4 (1H, m).

Example 14

Synthesis of 3-benzoyl-5-chloro-1-(1-hydroxy-2-propyl)-2-methylindole (No. 113):—

The same reaction as in Example 13 was performed using 5-chloro-3-methylindole and ethyl $\alpha$-bromopropionate to afford the desired compound (No. 113).

Melting point: 173—175°C (ethanol)

NMR($CDCl_3$, $\delta$ (ppm)):

1.60 (3H, d, J=7 Hz), 2.44 (3H, s), 3.7—4.2 (2H, m), 4.5—4.95 (2H, m), 7.10 (1H, dd, J=2, 9 Hz), 7.4—7.9 (7H, m).

IR(KBr)$cm^{-1}$:

3455, 1602, 1572, 1520, 1450, 1410, 1358, 1230, 1216, 1164, 1078, 1048, 1008, 890, 878, 802, 798, 752, 722, 702.

Example 15

Synthesis of 3-benzoyl-5-chloro-1-(1-methoxy-2-propyl)-2-methylindole (No. 304):—

The compound (No. 113) obtained in Example 14 was reacted with methyl iodide to afford the desired compound (No. 304).

NMR($CDCl_3$, $\delta$ (ppm)):

1.59 (3H, d, J=7 Hz), 2.47 (3H, s), 3.21 (3H, s), 3.5—4.1 (2H, m), 4.67 (1H, m), 7.05 (1H, dd, J=2, 9 Hz), 7.25—7.9 (7H, m).

IR($CHCl_3$)$cm^{-1}$:

1626, 1600, 1580, 1522, 1452, 1412, 1168, 1114, 1068, 1014, 940, 898.

Example 16

Synthesis of 3-benzoyl-1-(1-hydroxy-2-propyl)-2,5-dimethylindole (No. 114):—

The same reaction as in Example 13 was carried out using 2,5-dimethylindole and ethyl $\alpha$-bromopropionate to afford the desired compound (No. 114).

NMR($CDCl_3$, $\delta$ (ppm)):

1.38 (3H, d, J=6.5 Hz), 1.88 (1H, dd, J=4.8 Hz, disappeared in $D_2O$), 2.27 (3H, s), 2.35 (3H, s), 3.2—4.55 (3H, m), 6.02 (1H, s), 6.82 (1H, dd, J=1.9 Hz), 7.06—7.34 (2H, m).

IR(KBr)$cm^{-1}$:

3370, 1600, 1582, 1562, 1516, 1402, 1236, 1180, 1158, 1052, 1016, 912, 888, 808, 794, 762, 732.

Example 17

Synthesis of 3-benzoyl-1-(1-ethoxy-2-propyl)-2,5-dimethylindole (No. 348):—

The compound (No. 114) obtained in Example 16 was reacted with ethyl iodide to afford the desired compound (No. 348).

Melting point: 84—85°C (cyclohexane).

NMR($CDCl_3$, $\delta$ (ppm)):

1.06 (3H, t, J=7 Hz), 1.61 (3H, d, J=7 Hz), 2.27 (3H, s), 2.47 (3H, s), 3.1—3.6 (2H, m), 3.82 (2H, dd, J=2, 7 Hz), 4.4—4.93 (1H, m), 6.82—7.9 (8H, m).

IR(KBr)$cm^{-1}$:

1628, 1600, 1580, 1520, 1486, 1470, 1444, 1410, 1386, 1376, 1360, 1274, 1238, 1194, 1176, 1110, 1078, 1050, 900, 878, 792, 736, 708.

Example 18

Synthesis of 1-(1-methoxy-2-propyl)-2-methyl-3-thenoylindole (No. 315):—

The same reaction as in Example 1 was carried out using 2-methylindole and methyl $\alpha$-bromopropionate to afford 1-(1-acetoxy-2-propyl)-2-methylindole. The product was hydrolyzed with sodium hydroxide, and then reacted with methyl iodide to form 1-(1-methoxy-2-propyl)-2-methylindole. The product was then reacted with thiophenecarboxylic anhydride in 52% hydriodic acid to afford the desired compound (No. 315).

IR($CHCl_3$)$cm^{-1}$:

1605, 1514, 1460, 1413, 1390, 1351, 1267, 1220, 1165, 1106, 1082, 834.

NMR($CDCl_3$, $\delta$ (ppm)):

1.60 (3H, d, J=6 Hz), 2.55 (3H, s), 3,18 (3H, s), 3.5—4.1 (2H, m), 4.70 (1H, sextet, J=6 Hz), 6.9—7.75 (7H, m).

Example 19

Synthesis of 1-(1-hydroxy-2-propyl)-2-methyl-3-o-tolyl)indole (No. 126):—

The 1-(1-acetoxy-2-propyl)-2-methylindole obtained in Example 18 was acylated with o-methylbenzoic anhydride, and then hydrolyzed to form the desired compound (No. 126).

NMR($CDCl_3$, $\delta$ (ppm)):

1.46 (3H, d, J=7 Hz), 2.21 (3H, s), 2.40 (3H, s), 3.33 (1H, bs), 3.45—4.20 (2H, m).

IR($CHCl_3$)$cm^{-1}$:

3400, 3040, 2920, 1600, 1565, 1510, 1480, 1455, 1400, 1260, 1225, 1165, 1050, 920, 895, 760, 740.

Example 20

Synthesis of 1-(1-acetoxy-2-propyl)-3-benzoyl-5-methoxy-2-methylindole (No. 219), 3-benzoyl-1-(1-hydroxy-2-propyl)-5-methoxy-2-methylindole (No. 116), and 3-benzoyl-5-hydroxy-1-(1-hydroxy-2-propyl)-2-methylindole (No. 117):—

5-Methoxy-2-methylindole was reacted with ethyl $\alpha$-bromopropionate in the same way as in Example 13 to form 1 - (1 - acetoxy - 2 - propyl) - 5 - methoxy - 2 - methylindole. The resulting product was reacted with benzoic anhydride to obtain 1 - (1 - acetoxy - 2 - propyl) - 3 - benzoyl - 5 - methoxy - 2 - methylindole (No. 219) and (1 - acetoxy - 2 - propyl) - 3 - benzoyl - 5 - hydroxy - 2 - methylindole which were then hydrolyzed with sodium hydroxide to form the desired compounds (No. 116) and (No. 117), respectively.

Spectral data of the compound (No. 219)

IR($CHCl_3$)$cm^{-1}$:

2940, 1734, 1600, 1460, 1250—1210, 860.

NMR($CDCl_3$, $\delta$ (ppm)):

1.60 (3H, d, J=7 Hz), 1.89 (3H, s), 2.47 (3H, s), 3.59 (3H, s), 4.3—5.0 (3H, m), 6.63—6.87 (2H, m), 7.25—7.80 (6H, m).

Spectral data of the compound (No. 116)

$IR(CHCl_3)cm^{-1}$:

3320, 2930, 2200, 1600, 1470, 1400, 1270, 1150, 900, 685.

$NMR(CDCl_3, \delta$ (ppm)):

1.57 (3H, d, J=7 Hz), 2.41 (3H, s), 2.7 (1H, br, s), 3.57 (3H, s), 3.7—4.8 (3H, m), 6.57—6.83 2H, m), 7.20—7.75 (6H, m).

Spectral data of the compound (No. 117)

$IR(KBr)cm^{-1}$:

3300, 3150, 1620, 1600, 1590, 1565, 1520, 1475, 1425, 1255, 1055, 880, 760.

$NMR(DMSO-d_6, \delta$ (ppm):

1.63 (3H, d, J=7 Hz), 2.52 (3H, s), 3.9 (3H, m), 4.5—4.9 (1H, m), 6.6—6.9 (2H, m), 7.4—7.8 (6H, m), 8.90 (1H, br, s).

## Example 21

Synthesis of 3-benzoyl-5-methoxy-1-(1-methoxy-2-propyl)-2-methylindole (No. 318):—

A mixture of 0.85 g (3.2 millimoles) of 5 - methoxy - 1 - (1 - methoxy - 2 - propyl) - 2 - methylindole, 1.47 g (6.4 millimoles) of benzoic anhydride and 0.2 ml of 52% hydriodic acid was heated at 140°C for 2 hours in a stream of nitrogen with stirring. The reaction mixture was worked up to separate 0.46 g (yield 43%) of the desired compound (No. 318).

$IR(CHCl_3)cm^{-1}$:

2960, 2900, 1610, 1572, 1506, 1474, 1440, 1410, 1388, 1275, 1160, 1144, 1106, 1032, 905.

$NMR(CDCl_3, \delta$ (ppm)):

1.57 (3H, d, J=7Hz), 2.47 (3H, s), 3.20 (3H, s), 3.59 (3H, s), 3.60 (2H, m), 4.63 (1H, sextet, J=7Hz), 6.6—6.9 (2H, m), 7.2—7.8 (6H, m).

## Example 22

Synthesis of 3-benzoyl-2-isopropyl-1-(1-methoxy-2-propyl)indole (No. 303):—

A mixture of 1.15 g (5 millimoles) of 2 - isopropyl - 1 - (1 - methoxy - 2 - propyl)indole, 2.26 g (10 millimoles) of benzoic anhydride and 0.3 ml of 52% hydriodic acid was heated at 140°C for 2 hours in a stream of nitrogen with stirring. The reaction mixture was worked up to separate 1.03 g (yield 62%) of the desired compound (No. 303).

$IR(CHCl_3)CM^{-1}$:

3000, 2940, 1623, 1508, 1460, 1410, 1240—1205, 1103.

$NMR(CDCl_3, \delta$ (ppm)):

1.40 (3H, d, J=7 Hz), 1.43 (3H, d, J=7 Hz), 1.60 (3H, d, J=7 Hz), 3.23 (3H, s), 3.67 (1H, m), 3.80 (2H, d, J=7 Hz), 4.83 (1H, sextet, J=7 Hz), 6.8—7.9 (9H, m).

## Example 23

Synthesis of 3-(p-chlorobenzoyl-1-(1-methoxy-2-propyl)-2-methylindole (No. 317):—

A mixture of 0.8 g (4 millimoles) of 1-(1-methoxy-2-propyl)-2-methylindole, 3.48 g (11.8 millimoles) of p-chlorobenzoic anhydride and 0.25 ml of 52% hydriodic acid was heated at 140°C for 2.5 hours in a stream of nitrogen with stirring. The reaction mixture was worked up to separate 810 mg (yield 66%) of the desired compound (No. 317).

$IR(liquid film)cm^{-1}$:

2850, 1618, 1593, 1460, 1408, 1105, 1089, 898, 837.

$NMR(CDCl_3, \delta$ (ppm)):

1.62 (3H, d, J=7Hz), 2.58 (3H, s), 3.26 (3H, s), 3.8 (2H, m), 4.76 (1H, sextet, J=7Hz), 6.9—7.8 (8H, m).

## Example 24

Synthesis of 3-benzoyl-1-(1-hydroxy-3-pentyl)-2-methylindole (No. 106):—

The same reaction as in Example 13 was carried out using methyl 2-pentenoate and 2-methylindole to afford the desired compound (No. 106).

$IR(CHCl_3)cm^{-1}$:

3610, 3550, 3000, 2400, 1600, 1400, 1040, 900, 870.

$NMR(CDCl_3, \delta$ (ppm)):

0.75 (3H, t, J=7Hz), 1.7—2.8 (4H, m), 2.52 (3H, s), 2.9—3.8 (2H, m), 4.3—4.9 (1H, m), 6.8—8.0 (9H, m).

## Example 25

Synthesis of 3-benzoyl-1-(1-methoxy-3-pentyl)-2-methylindole (No. 319):—

The compound (No. 106) obtained in Example 24 was reacted with methyl iodide to afford the desired compound (No. 319).

IR(KBr)$cm^{-1}$:

2945, 1617, 1520, 1410, 1233, 1105, 1077, 1052, 895.

NMR($CDCl_3$, $\delta$ (ppm)):

0.77 (3H, t, J=7Hz), 1.6—3.5 (6H, m), 2.51 (3H, s), 3.20 (3H, s), 4.2—4.8 (1H, m) 6.9—7.9 (9H, m).

## Example 26

Synthesis of 3-benzoyl-1-(1-ethoxy-3-pentyl)-2-methylindole (No. 320):—

The compound (No. 106) obtained in Example 24 was reacted with ethyl iodide to afford the desired compound (No. 320) in a yield of 58%.

IR(KBr)$cm^{-1}$:

2980, 2880, 1618, 1526, 1417, 1166, 1121, 861, 750.

NMR($CDCl_3$, $\delta$ (ppm)):

0.76 (3H, t, J=7Hz), 1.13 (3H, t, J=7Hz), 1.6—3.1 (6H, m), 2.53 (3H, s), 3.1—3.6 (2H, m), 4.3—4.9 (1H, m), 6.9—7.9 (9H, m).

## Example 27

Synthesis of 1-(1-acetoxy-3-pentyl)-3-benzoyl-2-methylindole (No. 204):—

The compound (No. 106) obtained in Example 24 was acetylated to afford the desired compound (No. 204) in a yield of 76%.

IR($CHCl_3$)$cm^{-1}$:

3480, 3000, 2900, 2400, 1900, 1740, 1620, 1580, 1520, 1460, 1410, 1170, 1100, 1040, 900, 670.

NMR($CDCl_3$, $\delta$ (ppm)):

0.77 (3H, t, J=7Hz), 1.6—2.8 (4H, m), 1.90 (3H, s), 2.52 (3H, s), 3.4—4.7 (3H, m), 6.8—7.9 (9H, m).

## Example 28

Synthesis of 3-benzoyyl-1-(1-hydroxy-2-butyl)-2-methylindole (No. 104), and 1-(1-acetoxy-2-butyl)-3-benzoyl-2-methylindole (No. 205):—

The same reaction as in Example 13 was carried out using ethyl $\alpha$-bromobutyrate to afford the desired compounds (No. 104) and (No. 205).

Spectral data of the compound (No. 104)

IR($CHCl_3$)$cm^{-1}$:

3370, 1613m 1573, 1512, 1460, 1448, 1410, 1390, 1230—1195, 1053.

NMR($CDCl_3$, $\delta$ (ppm)):

0.72 (3H, t, J=7 Hz), 1.7—2.5 (2H, m), 2.38 (3H, s), 3.0 (3H, br), 3.6—4.5 (3H, m), 7.8—8.75 (9H, m).

Spectral data of the compound (No. 205)

IR($CHCl_3$)$cm^{-1}$:

1733, 1620, 1517, 1458, 1447, 1410, 1387, 1365, 1250, 1200, 1040.

NMR($CDCl_3$, $\delta$ (ppm)):

0.80 (3H, t, J=7Hz), 1.88 (3H, s), 1.8—2.3 (2H, m), 2.55 (3H, s), 4.35—4.7 (3H, m), 6.8—7.9 (9H, m).

## Example 29

Synthesis of 3-benzoyl-1-(1-methoxy-2-butyl)-2-methylindole (No. 311):—

The compound (No. 104) obtained in Example 28 was reacted with methyl iodide to afford the desired compound (No. 311).

IR($CHCl_3$)$cm^{-1}$:

1613, 1576, 1510, 1460, 1450, 1409, 1390, 1358, 1230—1210, 1168, 1005.

NMR($CDCl_3$, $\delta$ (ppm));

0.77 (3H, t, J=7Hz), 2.32 (quintet, J=7Hz), 2.53 (3H, s), 3.18 (3H, s), 3.55—4.1 (2H, m), 4.15—4.75 (1H, m), 6.9—7.9 (9H, m).

Example 30

Synthesis of 3-benzoyl-1-(1-ethoxy-2-butyl)-2-methylindole (No. 312):—

The compound (No. 104) obtained in Example 28 was reacted with ethyl iodide to afford the desired compound (No. 312).

$IR(CHCl_3)cm^{-1}$:

1616, 1513, 1460, 1448, 1410, 1230—1200, 1122, 1106.

NMR($CDCl_3$, $\delta$ (ppm)):

0.77 (3H, t, J=7Hz) 1.02 (3H, t, J=7 Hz), 1.8—2.4 (2H, m), 2.52 (3H, s), 3.05—3.7 (2H, m), 3.7—4.1 (2H, m), 4.2—4.7 (1H, m), 6.85—7.8 (9H, m).

Example 31

Synthesis of 3-benzoyl-1-(1-hydroxy-2-hexyl)-2-methylindole (No. 102):—

2-Methylindole (6.5 g; 0.05 millimole) was dissolved in 30 ml of dimethylformamide, and with stirring under ice cooling, 2.5 g of sodium hydride (50%) was added. After generation of hydrogen ceased, 10.5 g of methyl $\alpha$-bromocaproate was added dropwise, and reacted at room temperature for 3 days. Water (200 ml) was added to the reaction mixture to stop the reaction. The mixture was then extracted with 200 ml of ethyl acetate, washed with water and an aqueous solution of sodium chloride, and then dried over anhydrous sodium sulfate. The dried product was concentrated under reduced pressure. The resulting oily product was chromatographed on a silica gel column using hexane/ethyl acetate (15/1) as an eluent to afford 7.64 g (yield 59%) of methyl 2-(2methylindol-1-yl)caproate.

The resulting ester (7.64 g) was dissolved in 40 ml of ether. With stirring under ice cooling, 1.0 g of lithium aluminum hydride was added. With ice cooling, the mixture was stirred at room temperature for 30 minutes, and treated with a saturated aqueous solution of sodium sulfate. The resulting oily product was chromatographed on a silica gel column using benzene as an eluent to afford 3.0g of 1-(1-hydroxy-2-hexyl)-2-methylindole.

The resulting product (3.0 g) was reacted with 5 ml of acetic anhydride and 5 ml of pyridine to afford 3.25 g of 1-(1-acetoxy-2-hexyl)-2-methylindole.

The resulting acetate (3.25 g; 11.9 millimoles) was benzoylated using 5.40 g (24 millimoles) of benzoic anhydride and then hydrolyzed to afford 2.05 g (yield 51% based on the acetate) of the desired compound (No. 102).

NMR($CDCl_3$, $\delta$ (ppm)):

0.78 (3H, s), 0.9—1.4 (4H, m), 1.6—2.1 (2H, m), 2.43 (3H, s), 3.3 (1H, br), 3.5—4.4 (3H, br, m), 7.0—7.85 (9H, m).

$IR(CHCl_3)cm^{-1}$:

3430, 2950, 2880, 1620, 1570, 1520, 1460, 1400, 1260, 1160, 890.

Example 32

Synthesis of 1-(1-acetoxy-2-hexyl)-3-benzoyl-2-methylindole (No. 206):—

500 mg of the compound (No. 102) obtained in Example 31 was reacted with 2 ml of acetic anhydride and 2 ml of pyridine to afford 560 mg (yield 100%) of the desired compound (No. 206).

NMR($CDCl_3$, $\delta$ (ppm)):

0.79 (3H, brt, J=6 Hz), 1.0—1.5 (4H, m), 1.88 (3H, s), 1.55—2.0 (2H, m), 2.58 (3H, s), 4.4—4.8 (3H, m), 7.0—7.95 (9H, m).

$IR(CHCl_3)cm^{-1}$:

2930, 1740, 1620, 1404, 890.

Example 33

Synthesis of 3-benzoyl-1-(1-methoxy-2-hexyl)-2-methylindole (No. 334):—

853 mg (2.5 millimoles) of the compound (No. 102) obtained in Example 31 was dissolved in 5 ml of dimethylformamide and with stirring under ice cooling, 124 mg (1.1 equivalents) of sodium hydride (52.9%) was added to form the corresponding sodium salt. Then, 1 ml of methyl iodide was added, and the mixture was maintained at room temperature for 12 hours. The reaction mixture was worked up to separate the desired compound (No. 334) in a yield of 85%.

NMR($CDCl_3$, $\delta$ (ppm)):

0.82 (3H, brt, J=6 Hz), 1.0—1.5 (4H, m), 1.8—2.4 (2H, m), 2.56 (3H, s), 3.22 (3H, s), 3.6—4.15 (2H, m, ABX), 4.25—4.9 (1H, m), 7.0—8.95 (9H, m).

$IR(CHCl_3)cm^{-1}$:

2950, 1628, 1533, 1418, 1385, 1110, 895, 755.

Example 34

Synthesis of 3-benzoyl-1-(1-hydroxy-2-octyl)-2-methylindole (No. 103):—

The same reaction as in Example 31 was carried out using 2-methylindole and ethyl $\alpha$-bromocaprylate to afford the desired compound (No. 103).

NMR($CDCl_3$, $\delta$ (ppm)):

0.81 (3H, brt, J=6 Hz), 1.0—1.4 (8H, m), 1.7—2.1 (2H, m), 1.05 (3H, t, J=7 Hz), 3.32 (2H, q,

J=7Hz), 2.45 (3H, s), 3.80 (2H, m), 4.2—4.7 (1H, m), 7.0—7.9 (9H, m).

IR(KBr)$cm^{-1}$:

3300, 2950, 1630, 1527, 1462, 1418, 1215, 1065, 748.

Example 35

Synthesis of 1-(1-acetoxy-2-octyl)-3-benzoyl-2-methylindole (No. 207):—

The compound (No. 103) obtained in Example 34 was acetylated to afford the desired compound (No. 207) in a yield of 98%.

IR (liquid film)$cm^{-1}$:

3030, 2950, 2900, 1740, 1630, 1580, 1520, 1460, 1420, 1170, 1040, 900.

NMR($CDCl_3$, $\delta$ (ppm)):

0.5—2.5 (13H, m), 1.91 (3H, s), 2.52 (3H, s), 4.3—4.8 (3H, m), 6.9—8.0 (9H, m).

Example 36

Synthesis of 3-benzoyl-1-(1-methoxy-2-octyl)-2-methylindole (No. 335):—

The compound (No. 103) obtained in Example 34 was reacted with methyl iodide to afford the desired compound (No. 335).

IR(KBr)$cm^{-1}$:

2950, 2900, 2870, 1630, 1600, 1530, 1460, 1420, 1220, 1120, 1080, 900, 800, 740.

NMR($CDCl_3$, $\delta$ (ppm)):

0.6—2.3 (13H, m), 2.53 (3H, s), 3.55 (3H, s), 3.5—4.0 (2H, m), 4.2—4.8 (1H, m), 6.9—7.9 (9H, m).

Example 37

Synthesis of 3-benzoyl-1-(1-ethoxy-2-octyl)-2-methylindole (No. 336):—

The compound (No. 103) obtained in Example 34 was reacted with ethyl iodide to afford the desired compound (No. 336) in a yield of 80%.

NMR($CDCl_3$, $\delta$ (ppm)):

0.81 (3H, brt, J=6 Hz), 1.0—1.4 (8H, m), 1.7—2.1 (2H, m), 1.05 (3H, t, J=7 Hz), 3.32 (2H, q, J=7 Hz), 2.45 (3H, s), 3.80 (2H, m), 4.2—4.7 (1H, m), 7.0—7.9 (9H, m).

IR($CHCl_3$)$cm^{-1}$:

2930, 2850, 1620, 1408, 1110, 890.

Example 38

Synthesis of 3-benzoyl-1-(4-hydroxy-2-butyl)-2-methylindole (No. 105):—

(1) 2-Methylindole (13.1 g; 0.1 millimole) was reacted with 23.6 g (0.236 mole) of methyl crotonate in 80 ml of dimethyl formamide in the presence of 0.6 g (0.012 mole) of sodium hydride at room temperature for 20 hours. After the reaction, water was added, and the reaction mixture was extracted with ethyl acetate. The organic layer was dried over anhydrous magnesium acetate, and concentrated to afford 34 g of a crude product. The crude product was reduced with 9.5 g (0.25 mole) of lithium aluminum hydride in 250 ml of ether at room temperature for 18 hours with stirring. After the reaction, the reaction mixture was worked up with a saturated aqueous solution of sodium sulfate, washed with ether, dried over magnesium sulfate, and concentrated to afford 22.8 g of a crude product.

The product was chromatographed on a silica gel column using hexane/ethyl acetate (2/1) as an eluent to afford 8.00 g (39.4 millimoles; yield 39.4%) of 1 - (1 - hydroxy - 3 - butyl) - 2 - methylindole.

NMR($CDCl_3$, $\delta$ (ppm)):

1.30 (br, 1H, OH), 1.57 (d, 3H, J=7Hz, NCHC$H_3$), 1.7—2.3 (m, 2H, C$H_2$$CH_2$OH), 2.40 (s, 3H, $CH_3$), 2.8—3.7 (m, 2H, C$H_2$OH), 4.4—5.0 (m, 1H, NCH), 6.16 (s, 1H, proton at the 3-position of indole) 6.8—7.6 (m, 4H, proton on the indole ring).

IR(liquid film)$cm^{-1}$:

3300, 3000, 2900, 2850, 1600, 1540, 1450, 1405, 1355, 1335, 1300, 1230, 1160, 1140, 1100, 1035, 985, 950, 915, 840, 760, 740, 730.

(2) 8.00 g of 1-(1-hydroxy-3-butyl)-2-methylindole was reacted with 30 ml of acetic anhydride and 25 ml of pyridine for 20 hours to afford 9.0 g (36.7 millimoles; yield 93.2%) of 1-(1-acetoxy-3-butyl)-2-methylindole.

NMR($CDCl_3$, $\delta$ (ppm)):

1.57 (d, 3H, J=7 Hz, NCHC$H_3$), 1.92 (s, 3H, $OCOCH_3$), 1.9—2.6 (m, 2H, NCHC$H_2$), 2.38 (s, 3H, $CH_3$), 3.6—4.2 (m, 2H, $CH_2$OCO), 4.3—4.9 (m, 1H, NCH), 6.26 (s, 1H, proton at the 3-position of indole), 7.0—7.7 (m, 4H, proton on the indole ring).

(3) 9.0 g of 1-(1-acetoxy-3-butyl)-2-methylindole was reacted with 20.7 g (91.8 millimoles) of benzoic anhydride and 0.5 ml of hydriodic acid in an atmosphere of nitrogen at 140°C for 1.5 hours. After cooling, ethanol, water and sodium hydroxide were added to the reaction mixture. The mixture

was stirred at room temperature for 3 hours to hydrolyze the reaction product. The product was concentrated under reduced pressure, extracted with ethyl acetate, washed with an aqueous solution of sodium hydrogen carbonate, dried and concentrated. The crude product was chromatographed on a silica gel column using hexane/ethyl acetate (1/1) as an eluent to afford 6.69 g (21.8 millimoles; yield 59.4%) of the desired compound (No. 105).

NMR($CDCl_3$, $\delta$ (ppm)):

1.63 (d, 3H, J=6.5Hz, $NCHCH_3$), 1.9—2.5 (m, 2H, $NCHCH_2$), 2.53 (s, 3H, $CH_3$), 2.75 (bs, 1H, OH, 3.1—3.7 (m, 2H, $CH_2OH$), 4.5—5.2 (m, 1H, NCH), 7.0—7.95 (m, 9H, protons on the indole and benzene rings).

IR(KBr)$cm^{-1}$:

3350, 3000, 2880, 2830, 1600, 1565, 1505, 1450, 1435, 1400, 1350, 1295, 1260, 1220, 1160, 1150, 1075, 1050, 1030, 920, 890, 860, 790, 735, 705, 685.

Example 39

Synthesis of 1-(4-acetoxy-2-butyl)-3-benzoyl-2-methylindole (No. 201):—

Acetic anhydride (20 ml) and 15 ml of pyridine were added to 2.26 g (7.36 millimoles) of the compound (No. 105) obtained in Example 38, and the mixture was stirred at room temperature for 1 hour to decompose the acetic anhydride. The product was concentrated under reduced pressure. The crude product was dissolved in ethyl acetate and was washed successively with an aqueous solution of potassium hydrogen sulfate, an aqueous solution of sodium chloride and an aqueous solution of sodium hydrogen carbonate, dried over magnesium sulfate and concentrated to afford 2.70 g of the crude product. The crude product was chromatographed on a silica gel column using hexane/ethyl acetate (2/1) as an eluent to afford 2.29 g (6.56 millimoles; yield 89.2%) of the desired compound (No. 201).

NMR($CDCl_3$, $\delta$ (ppm)):

1.67 (d, 3H, J=7Hz, $NCHCH_3$), 1.93 (s, 3H, $OCOCH_3$), 2.1—2.6 (m, 2H, $NCHCH_2$), 2.57 (s, 3H, $CH_3$), 3.8—4.4 (m, 2H, $CH_2OCO$), 4.5—5.1 (m, 1H, NCH), 7.03—8.0 (m, 4H, protons on the indole and benzene rings).

IR (liquid film)$cm^{-1}$:

3010, 2949, 1730, 1615, 1570, 1510, 1450, 1405, 1380, 1360, 1300, 1230, 1165, 1130, 1100, 1050, 1030, 920, 890, 870, 795, 740, 715, 690.

Example 40

Synthesis of 3-benzoyl-1-(4-methoxy-2-butyl)-2-methylindole (No. 306):—

The compound (No. 105) obtained in Example 38 (1.50 g; 4.89 millimoles) was reacted with 367 mg (7.35 millimoles) of sodium hydride and 5 ml of methyl iodide in 8 ml of dimethoxyethane at room temperature for 20 hours. The reaction mixture was worked up to afford 1.514 g of a crude product. The crude product was chromatographed on a silica gel column using hexane/ethyl acetate (3/1) as an eluent to afford 1.301 g (4.05 millimoles; yield 82.9%) of the desired compound (No. 306).

Melting point: 91—93°C

NMR($CDCl_3$, $\delta$ (ppm)):

1.65 (d, 3H, J=7Hz, $NCHCH_3$), 1.8—2.5 (m, 2H, $NCHCH_2$), 2.56 (s, 3H, $CH_3$), 3.23 (s, 3H, $OCH_3$), 3.30 (t, 2H, J=6Hz, $CH_2O$), 4.6—5.1 (m, 1H, NCH), 7.0—8.0 (m, 9H, protons on the indole and benzene rings).

IR(KBr)$cm^{-1}$:

3020, 2940, 2890, 2840, 1620, 1600, 1570, 1515, 1455, 1440, 1410, 1380, 1350, 1310, 1295, 1265, 1230, 1185, 1165, 1110, 1090, 1040, 1020, 920, 890, 865, 795, 740, 715, 690.

Example 41

Synthesis of 3-benzoyl-1-(4-ethoxy-2-butyl)-2-methylindole (No. 307):—

The compound (No. 105) obtained in Example 38 (1.50 g; 4.89 millimoles) was reacted with 367 mg (7.34 millimoles) of sodium hydride and 5 ml of ethyl iodide in 18 ml of dimethoxyethane at room temperature for 20 hours. The reaction mixture was worked up, and the crude product was chromatographed on a silica gel column using hexane/ethyl acetate (3/1) as an eluent to afford 1.359 g (4.06 millimoles; 83.0%) of the desired compound (No. 307).

Melting point: 74 to 76°C

NMR($CDCl_3$, $\delta$ (ppm)):

1.10 (t, 3H, J=7Hz, $OCH_2CH_3$), 1.60 (d, 3H, J=7Hz, $NCHCH_3$), 1.9—2.8 (m, 2H, $NCHCH_2$), 2.57 (s, 3H, $CH_3$), 3.0—3.55 (m, 4H, $CH_2OCH_2$), 4.6—5.1 (m, 1H, NCH), 7.0—8.0 (m, 4H, protons on the indole and benzene rings).

IR(KBr)$cm^{-1}$:

3020, 2950, 2900, 2840, 1610, 1600, 1570, 1520, 1475, 1450, 1440, 1410, 1380, 1350, 1310, 1300, 1260, 1230, 1165, 1135, 1115, 1095, 1070, 1045, 1020, 1010, 985, 960, 920, 895, 870, 855, 815, 795, 760, 740, 720, 690.

Example 42

Synthesis of 3-(o-fluorobenzoyl)-1-(4-hydroxy-2-butyl)-2-methylindole (No. 125):—

The 1-(1-acetoxy-3-butyl)-2-methylindole obtained in Example 38 was reacted with o-fluorobenzoic anhydride, and the reaction product was hydrolyzed to afford the desired compound (No. 125).

NMR($CDCl_3$, $\delta$ (ppm)):

1.55 (3H, d, J=6Hz), 1.9—2.4 (2H, m), 3.03 (1H, bs), 3.1—3.6 (2H, m), 4.5—5.1 (1H, m).

IR($CHCl_3$)$cm^{-1}$:

3450, 3050, 2970, 2930, 2870, 1605, 1570, 1510, 1480, 1460, 1450, 1410, 1360, 1215, 1100, 930, 755, 630.

Example 43

Synthesis of 3-benzoyl-1-(4-hydroxy-2-butyl)-2,5-dimethylindole (No. 137):—

The same reaction as in Example 38 was carried out using 2,5-dimethylindole and methyl crotonate to afford the desired compound (No. 137).

NMR($CDCl_3$, $\delta$ (ppm)):

1.61 (3H, d, J=7 Hz), 1.8—2.8 (3H, m), 2.25 (3H, s), 2.48 (3H, s), 3.1—3.8 (2H, m), 4.5—5.15 (1H, m), 6.8—7.9 (8H, m).

IR(KBr)$cm^{-1}$:

3370, 1584, 1562, 1516, 1404, 1234, 1180, 1158, 1052, 1016, 912, 888, 808, 794, 762, 730, 702, 696.

Example 44

Synthesis of 3-benzoyl-1-(4-methoxy-2-butyl)-2,5-dimethylindole (No. 310):—

The compound (No. 137) obtained in Example 43 was reacted with methyl iodide to afford the desired compound (No. 310).

NMR($CDCl_3$, $\delta$ (ppm)):

1.63 (3H, d, J=7Hz), 2.29 (3H, s), 2.49 (3H, s), 3.23 (3H, s), 1.8—3.5 (4H, m), 4.45—5.1 (1H, m), 6.85—8.0 (8H, m).

IR(KBr)$cm^{-1}$:

1620, 1600, 1580, 1522, 1418.

Example 45

Synthesis of 3-benzoyl-1-(7-hydroxy-2-heptyl)-2-methylindole (No. 108):—

(1) A solution (10 ml) of 2.5 g of 1-(4-bromo-2-butyl)-2-methylindole in tetrahydrofuran was added to a mixture of 228 mg (9.4 millimoles) of metallic magnesium and a trace of iodine, and the mixture was stirred at room temperature for 2 hours. Then, 100 mg of cuprous iodide and 5 ml of a tetrahydrofuran solution of 3.14 g (14.1 millimoles) of a tetrahydrofuran protected product of 3-bromo-1-propanol were added. The mixture was stirred at room temperature for 2 hours. After the reaction, the reaction mixture was extracted with ether, washed with a saturated aqueous solution of sodium hydrogen carbonate and a saturated aqueous solution of sodium chloride, dried over sodium sulfate, and concentrated under reduced pressure to afford 4.2 g of an oily product.

The oily product was deprotected with 37% $HClO_4$ in 40 ml of methanol to afford 1.404 g of 1-(7-hydroxy-2-heptyl)-2-methylindole.

NMR($CDCl_3$, $\delta$ (ppm)):

1.51 (3H, d, J=7Hz), 2.34 (3H, s), 3.43 (3H, t, J=6Hz), 6.14 (1H, bs), 6.8—7.6 (4H, m).

(2) 1.372 g of the 1-(7-hydroxy-2-heptyl)-2-methylindole obtained in (1) above was acetylated with acetic anhydride and pyridine to afford 1.448 g of the corresponding acetyl compound.

(3) 1.448 g of the acetyl compound was reacted with 1.88 ml (10 millimoles) of benzoic anhydride and then hydrolyzed to afford the desired compound (No. 108).

IR($CHCl_3$)$cm^{-1}$:

3700—3200, 2950, 1620, 1522, 1415, 1235, 1168, 545.

Example 46

Synthesis of 3-benzoyl-1-(7-ethoxy-2-heptyl)-2-methylindole (No. 328):—

The compound (No. 108) obtained in Example 45 was reacted with ethyl iodide to afford the desired compound (No. 328) in a yield of 16.9%.

NMR($CDCl_3$, $\delta$ (ppm)):

0.83—2.4 (8H, m), 1.11 (3H, t, J=7 Hz), 1.63 (3H, d, J=7 Hz), 2.57 (3H, s), 3.30 (2H, q, J=7Hz), 3.31 (2H, t, J=7 Hz), 4.28—4.8 (1H, m), 6.93—7.93 (9H, m).

IR($CHCl_3$)$cm^{-1}$:

3000, 2950, 2900, 1620, 1520, 1460, 1410, 1110, 900, 860.

Example 47

Synthesis of 3-benzoyl-(11-hydroxy-2-undecyl)-2-methylindole (No. 110):—

The same reaction as in Example 45 was carried out using 1-(4-bromo-2-butyl)-2-methylindole and 7-bromoheptanol tetrahydropyranyl ether to afford the desired compound (No. 110).

IR(liquid film)$cm^{-1}$:

3700—3200, 2950, 1625, 1418, 548.

NMR($CDCl_3$, $\delta$ (ppm)):

0.9—1.5 (16H, br), 1.59 (3H, d, J=7 Hz), 2.53 (3H, s), 3.53 (2H, t, J=6 Hz), 4.52 (1H, br. sextet, J=7 Hz), 6.8—7.8 (9H, m).

IR($CHCl_3$)$cm^{-1}$:

3025, 2950, 2880, 1620, 1520, 1460, 1410, 1270, 1170, 1020, 900, 870.

Example 48

Synthesis of 3-benzoyl-1-(11-ethoxy-2-undecyl)-2-methylindole (No. 330):—

The compound (No. 110) obtained in Example 48 was reacted with ethyl iodide to afford the compound (No. 330).

NMR($CDCl_3$, $\delta$ (ppm)):

0.9—2.33 (19H, m), 1.62 (3H, d, J=7 Hz), 2.56 (3H, s), 3.36 (2H, t, J=7 Hz), 3.39 (2H, q, J=7 Hz), 4.3—4.8 (1H, m), 6.9—7.9 (9H, m).

IR($CHCl_3$)$cm^{-1}$:

3000, 2950, 2900, 1620, 1520, 1460, 1410, 1270, 1170, 1110, 900.

Example 49

Synthesis of 3-benzoyl-1-(1-ethylthio-2-propyl)-2-methylindole (No. 400):—

586 mg (2 mmoles) of the compound (No. 100) obtained in Example 2, 732 mg (6 millimoles) of diethyl sulfide and 1.5 ml (6 millimoles) of tributylphosphine were dissolved in 1 ml of anhydrous pyridine. The solution was stirred at room temperature for 16 hours and then at 50°C for 8 hours. After the reaction, the reaction mixture was dissolved in 50 ml of ethyl acetate, and washed successively with 25 ml of 1N HCl, 20 ml of a saturated aqueous solution of sodium bicarbonate, 20 ml of water and 20 ml of a saturated aqueous solution of sodium chloride. The ethyl acetate layer was dried over magnesium sulfate, and the ethyl acetate was distilled off under reduced pressure to afford an oily product. The oily product was chromatographed on a silica gel column using benzene as an eluent to afford 519 mg (yield 77%) of the desired compound (No. 400).

NMR($CDCl_3$, $\delta$ (ppm)):

1.08 (3H, t, J=7 Hz), 1.71 (3H, d, J=7 Hz), 2.28 (2H, q, J=7 Hz), 2.60 (3H, s), 3.18 (2H, m), 4.75 (1H, m), 7.0—8.0 (9H, m).

IR($CHCl_3$)$cm^{-1}$:

3000, 2945, 1620, 1522, 1460, 1415, 1267, 1166, 897.

Example 50

Synthesis of 3-benzoyl-2-methyl-1-(1-phenylthio-2-propyl)indole (No. 401):—

The same reaction as in Example 49 was carried out at room temperature for 16 hours using diphenyl disulfide to afford the desired compound (No. 401).

NMR($CDCl_3$, $\delta$ (ppm)):

1.67 (3H, d, J=7Hz), 2.32 (3H, s), 3.50 (2H, m), 4.67 (1H, m), 7.0—8.0 (14H, m).

IR($CHCl_3$)$cm^{-1}$:

3050, 1618, 1520, 1457, 1405, 1223, 1163, 735.

Example 51

Synthesis of 3-benzoyl-1-(1-ethylthio-2-butyl)-2-methylindole (No. 417):—

The same reaction as in Example 49 was carried out using the compound (No. 104) obtained in Example 28 and diethyl sulfide to afford the desired compound (No. 417) in a yield of 75%.

NMR($CDCl_3$, $\delta$ (ppm)):

0.79 (3H, t, J=7 Hz), 1.07 (3H, t, J=7 Hz), 1.85—2.50 (4H, m), 2.60 (3H, s), 3.18 (2H, m), 4.16—4.60 (1H, m), 6.86—8.00 (9H, m).

IR($CHCl_3$)$cm^{-1}$:

2950, 2895, 1620, 1520, 1458, 1405, 1270, 1167, 908.

Example 52

Synthesis of 3-benzoyl-2-methyl-1-(1-phenylthio-2-butyl)indole (No. 418):—

The same reaction as in Example 49 was carried out at room temperature for 16 hours using the compound (No. 104) obtained in Example 28 and diphenyl disulfide to afford the desired compound (No. 418) in a yield of 69%.

NMR($CDCl_3$, $\delta$ (ppm)):

0.77 (3H, t, J=7 Hz), 1.85—2.70 (2H, m), 2.33 (3H, s), 3.45—3.75 (2H, m), 4.15—4.60 (1H, m), 7.0—8.0 (14H, m).

IR($CHCl_3$)$cm^{-1}$:

1620, 1458, 1408, 902.

Example 53

Synthesis of 3-benzoyl-2-methyl-1-(1-methylthio-2-propyl)indole (No. 402):—

The same reaction as in Example 49 was carried out using dimethyl sulfide to afford the desired compound (No. 402) in a yield of 54%.

NMR($CDCl_3$, $\delta$ (ppm)):

1.62 (3H, d, J=7 Hz), 1.68 (3H, s), 2.53 (3H, s), 2.83—3.23 (2H, m), 4.4—4.87 (1H, m), 6.8—7.9 (9H, m).

IR($CHCl_3$)$cm^{-1}$:

3020, 2945, 1622, 1525, 1460, 1409, 1270, 1168, 1057, 900.

Example 54

Synthesis of 3-benzoyl-2-methyl-1-[1-(2-pyridylthio)-2-propyl]indole (No. 405):—

The same reaction as in Example 49 was carried out at room temperature for 16 hours using 2,2′-dithiodipyridine to afford the desired compound (No. 405) in a yield of 99%.

NMR($CDCl_3$, $\delta$ (ppm)):

1.77 (3H, d, J=7 Hz), 2.47 (3H, s), 3.82 (2H, d, J=7 Hz), 4.95 (1H, sextet, J=7 Hz) 6.9—8.0 (12H, m), 8.4—8.6 (1H, m).

IR($CHCl_3$)$cm^{-1}$:

3000, 1618, 1577, 1455, 1408, 1163, 1122, 895.

Example 55

Synthesis of 3-benzoyl-1-(4-ethylthio-2-butyl)-2-methylindole (No. 409):—

The same reaction as in Example 49 was carried out using the compound (No. 105) obtained in Example 38 and diethyl sulfide to afford the desired compound (No. 409) in a yield of 74%.

NMR($CDCl_3$, $\delta$ (ppm)):

1.12 (3H, t, J=7 Hz), 1.60 (3H, d, J=7Hz), 2.2—2.6 (6H, m), 2.60 (3H, s), 4.8 (1H, m), 7.0—8.0 (9H, m).

IR($CHCl_3$)$cm^{-1}$:

2980, 2945, 1619, 1520, 1458, 1407, 1262, 1167, 895.

Example 56

Synthesis of 3-benzoyl-2-methyl-1-[1-(2-pyridylthio)-2-butyl]indole (No. 421):—

The same reaction as in Example 51 was carried out using 2,2′-dithiodipyridine at room temperature for 16 hours to afford the desired compound (No. 421) in a yield of 68%.

NMR($CDCl_3$, $\delta$ (ppm)):

0.79 (3H, t, J=7 Hz), 2.40 (3H, s), 1.90—2.40 (2H, m), 3.86 (2H, d, J=8 Hz), 4.50—5.00 (1H, m), 6.80—8.57 (13H, m).

IR($CHCl_3$)$cm^{-1}$:

2980, 1620, 1580, 1520, 1455, 1407, 1126.

Example 57

Synthesis of 3-benzyl-2-methyl-1-[1-(4-pyridylthio)-2-propyl]indole (No. 404):—

The same reaction as in Example 49 was carried out at room temperature for 16 hours using 4,4′-dithiodipyridine to afford the desired compound (No. 404) in a yield of 58%.

NMR($CDCl_3$, $\delta$ (ppm)):

1.77 (3H, d, J=7 Hz), 2.41 (3H, s), 3.4—3.8 (2H, m), 4.4—5.1 (1H, m), 6.8—7.9 (11H, m), 8.0—8.33 (2H, m).

IR($CHCl_3$)$cm^{-1}$:

3005, 1625, 1577, 1525, 1460, 1408, 1268, 1167, 898.

Example 58

Synthesis of 3-benzoyl-2-methyl-1-[4-(4-pyridylthio)-2-butyl]indole (No. 413):—

The same reaction as in Example 49 was carried out at room temperature for 16 hours using the compound (No. 105) obtained in Example 38 and 4,4′-dithiodipyridine to afford the desired compound (No. 413) in a yield of 75%.

IR($CHCl_3$)$cm^{-1}$:

3000, 2450, 2400, 1620, 1580, 1400, 1260, 1020, 910.

Example 59

Synthesis of 3-benzoyl-2-methyl-1-[1-(4-pyridylthio)-2-butyl]indole (No. 420):—

The same reaction as in Example 51 was carried out using 4,4′-dithiodipyridine to afford the desired compound (No. 420) in a yield of 78%.

NMR($CDCl_3$, $\delta$ (ppm)):

0.80 (3H, t, J=7Hz), 2.40 (3H, s), 1.9—2.9 (2H, m), 3.2—4.1 (2H, m), 4.2—4.8 (1H, m), 6.8—7.9 (11H, m), 8.1—8.5 (2H, m)

IR($CHCl_3$)$cm^{-1}$:

2990, 1710, 1625, 1580, 1530, 1460, 1410, 1270, 1165, 905.

Example 60

Synthesis of 3-benzoyl-2-methyl-1-[4-(2-pyridylthio)-2-butyl]indole (No. 414):—

The same reaction as in Example 8 was carried out using 2,2′-dithiodipyridine to afford the desired compound (No. 414) in a yield of 74%.

IR($CHCl_3$)$cm^{-1}$:

3000, 1620, 1580, 1520, 1460, 1405, 1260, 1120, 900.

NMR($CDCl_3$, $\delta$ (ppm)):

1.65 (3H, d, J=7Hz), 2.0—3.0 (4H, m), 2.53 (3H, s), 4.5—5.1 (1H, m), 6.7—7.9 (11H, m), 8.1—8.5 (2H, m).

Example 61

Synthesis of 3-benzoyl-2-methyl-1-(4-phenylthio-2-butyl)indole (No. 410):—

The same reaction as in Example 1 was carried out using the compound (No. 105) obtained in Example 38 and diphenyl disulfide to afford the compound (No. 410) in a yield of 79%.

NMR($CDCl_3$, $\delta$ (ppm)):

1.60 (3H, d, J=7Hz), 2.1—2.9 (4H, m), 2.58 (3H, s), 4.8 (1H, m), 7.0—8.0 (14H, m).

IR($CHCl_3$)$cm^{-1}$:

3025, 1620, 1580, 1520, 1460, 1410, 1270, 1170, 1030, 900.

Example 62

Synthesis of 3-benzoyl-2-methyl-1-[1-(5-methyl-1,3,5-thiadiazol-2-ylthio)propyl]indole (No. 407):—

2.62 g (0.02 mole) of 2-mercapto-5-methyl-1,3,4-thiadiazole was dissolved in 50 ml of methylene chloride. While the solution was cooled with ice with stirring, a solution of 4.46 g (1.1 equivalents) of 85% m-chloroperbenzoic acid in 20 ml of methylene chloride was added dropwise and reacted for 1 hour. After the reaction, the reaction mixture was washed with a saturated aqueous solution of sodium bicarbonate, water and an aqueous solution of sodium chloride, dried over magnesium sulfate, and then distilled under reduced pressure to afford 2.3 g (8.8 millimoles) of a disulfide compound. Using the resulting disulfide and the compound (No. 100) obtained in Example 2, the same reaction as in Example 49 was performed to afford the desired compound (No. 407) in a yield of 57%.

NMR($CDCl_3$, $\delta$ (ppm)):

1.77 (3H, d, J=6Hz), 2.43 (3H, s), 2.63 (3H, s), 3.90 (2H, d, J=6Hz), 5.20 (1H, sextet, J=6Hz), 7.0—7.9 (9H, m).

IR($CHCl_3$)$cm^{-1}$:

3000, 1620, 1520, 1460, 1410, 1380, 1270, 1165, 1070, 900.

Example 63

Synthesis of 1-(1-benzenesulfinyl-2-propyl)-3-benzoyl-2-methylindole (No. 501):—

570 mg (1.54 millimoles) of the compound (No. 401) obtained in Example 50 was dissolved in 50 ml of methanol, and an aqueous solution of 414 mg (1.93 millimoles) of sodium metaperiodate in 30 ml of water was added. The mixture was stirred at room temperature for 2 hours in a stream of nitrogen. After the reaction, the reaction mixture was dissolved in 50 ml of ethyl acetate, and washed with 30 ml of water. The ethyl acetate layer was dried over sodium sulfate, and the ethyl acetate was distilled off under reduced pressure to form an oily product. The oily product was chromatographed on a silica gel column using a mixture composed of 67% of benzene and 33% of hexane as an eluent to afford 280 mg (yield 47%) of the desired compound (No. 501).

NMR($CDCl_3$, $\delta$ (ppm)):

1.70 (1.5H, d, J=7Hz), 1.80 (1.5H, d, J=7Hz), 2.42 (1.5H, s), 2.65 (1.5H, s), 2.9—3.8 (2H, m), 4.9—5.5 (1H, m), 6.6—8.3 (14H, m).

IR(KBr)$cm^{-1}$:

1622, 1525, 1410, 1030, 742.

Example 64

Synthesis of 3-benzoyl-1-(1-methanesulfinyl-2-propyl)-2-methylindole (No. 502):—

The same reaction as in Example 63 was carried out using the compound (No. 402) obtained in

Example 53 to afford the desired compound (No. 502) in a yield of 71%.

NMR($CDCl_3$, $\delta$ (ppm)):

1.77 (3H, d, J=7Hz), 2.45 (3H, s), 2.57 (3H, s), 2.8—4.0 (2H, m), 4.8—5.5 (1H, m), 6.9—7.9 (9H, m).

IR(KBr)$cm^{-1}$:

1621, 1520, 1462, 1405, 1273, 1232, 1165, 1058, 1035, 900, 745.

Example 65

Synthesis of 3-benzoyl-1-(4-mercapto-2-butyl)-2-methylindole (No. 423):—

1.00 g (3.26 millimoles) of the compound (No. 105) obtained in Example 38 and 1.75 g (6.52 millimoles) of triphenylphosphine were dissolved in 30 ml of ether, and 2.21 g (6.52 millimoles) of tetrabromomethane was added. The reaction was performed at room temperature for 12 hours. Then, the reaction mixture was worked up in a customary manner to afford 1.30 g of a crude product. The crude product was chromatographed on a silica gel column using benzene as an eluent to afford 1.17 g (yield 97%) of 3 - benzoyl - 1 - (4 - bromo - 2 - butyl) - 2 - methylindole. 1.17 g of the resulting bromo-substituted product and 241 mg (3.16 millimoles) of thiourea were dissolved in 5 ml of dimethylsulfoxide and reacted at room temperature for 12 hours. After the reaction, the reaction mixture was poured into 20 ml of a 10% aqueous solution of sodium hydroxide. Then, a saturated aqueous solution of ammonium sulfate was added, and the mixture was extracted with ethyl acetate. The organic layer was washed, dried and concentrated to afford 873 mg of a crude product. The crude product was subjected to thin-layer chromatography (benzene:ethyl acetate=97:3) to afford 350 mg (yield 34%) of the desired compound (No. 423).

NMR($CDCl_3$, $\delta$ (ppm)): 1.58 (3H, d, J=7Hz), 2.57 (3H, s), 4.8 (1H, m), 6.95—7.9 (9H).

IR($CHCl_3$)$cm^{-1}$:

2550.

Example 66

Synthesis of 1-(4-benzenesulfinyl-2-butyl)-3-benzoyl-2-methylindole (No. 507):—

The same reaction as in Example 63 was carried out using the compound (No. 410) obtained in Example 61 to afford the desired compound (No. 507) in a yield of 79%.

NMR($CDCl_3$, $\delta$ (ppm)):

1.62 (3H, s), 2.47 (1.5H, s), 2.50 (1.5H, s), 1.9—3.3 (4H, m), 4.3—5.2 (1H, m), 6.6—8.15 (14H, m).

IR(KBr)$cm^{-1}$:

3060, 2990, 2950, 1623, 1523, 1460, 1443, 1410, 1040, 745.

Example 67

Synthesis of 1-(1-benzenesulfonyl-2-propyl)-3-benzoyl-2-methylindole (No. 601):—

529 mg (1.54 millimoles) of the compound (No. 401) obtained in Example 50 was dissolved in 25 ml of methylene chloride, and 520 mg (3.00 millimoles) of metachlorobenzoic acid was added. The mixture was stirred at room temperature for 16 hours. After the reaction, the reaction mixture was washed successively once with 30 ml of a saturated aqueous solution of sodium bicarbonate and three times with 30 ml of water. The methylene chloride layer was dried over magnesium sulfate, and the methylene chloride was distilled off under reduced pressure to afford an oily product. The oily product was chromatographed on a silica gel column using a mixture of 95% of benzene and 5% of ethyl acetate as an eluent to afford 382 mg (yield 60%) of the desired compound (No. 601).

NMR($CDCl_3$, $\delta$ (ppm)):

1.70 (3H, d, J=7Hz), 2.51 (3H, s), 3.3—4.2 (2H, m), 4.9—5.4 (1H, m), 6.7—7.9 (14H, m).

IR(KBr)$cm^{-1}$:

3060, 2990, 2940, 1623, 1525, 1460, 1445, 1413, 1302, 1146, 742.

Example 68

Synthesis of 1-(4-benzenesulfonyl-2-butyl)-3-benzoyl-2-methylindole (No. 607):—

The same reaction as in Example 67 was carried out using the compound (No. 410) obtained in Example 61 to afford the desired compound (No. 607) in a yield of 40%.

NMR($CDCl_3$, $\delta$ (ppm)):

1.62 (3H, d, J=7Hz), 2.48 (3H, s), 2.2—3.1 (4H, m), 4.3—5.0 (1H, m), 6.8—7.9 (14H, m).

IR(KBr)$cm^{-1}$:

1622, 1523, 1468, 1442, 1410, 1302, 1143, 740.

Example 69

Synthesis of 3-benzoyl-1-(1-methanesulfonyl)-2-butyl)-2-methylindole (No. 614):—

The same reaction as in Example 67 was carried out using the compound (No. 402) obtained in Example 53 to afford the desired compound (No. 614) in a yield of 26%.

NMR($CDCl_3$, $\delta$ (ppm)):

1.75 (3H, d, J=7Hz), 2.20 (3H, s), 2.55 (3H, s), 3.1—4.4 (2H, m), 5.0—5.5 (1H, m), 6.9—7.8 (9H, m).

IR(Kbr)$cm^{-1}$:

3000, 2930, 1620, 1524, 1458, 1407, 1295, 1133, 865, 742.

Example 70

Synthesis of 3-benzoyl-1-(2-hexyl)-2-methylindole (No. 705):—

(1) 39.4 g (0.30 mole) of 2-methylindole, 19.9 g (0.30 mole) of potassium hydroxide and 2.5 g (0.01 mole) of 18-crown-6 were dissolved in 200 ml of benzene, and the solution was refluxed for 2 hours. Then, 80 ml of benzene having dissolved therein 48.0 g (0.29 mole) of 2-hexylbromide was added, and the mixture was refluxed for 7.5 hours. Materials insoluble in benzene were removed by filtration. Ethyl acetate was added to the filtrate, and the mixture was washed with water. The product was dried over magnesium sulfate, and concentrated to form an oily product. The oily product was purified by column chromatography to afford 5.90 g (yield 9.5%) of 1-(2-hexyl)-2-methylindole.

(2) The 1-(2-hexyl)-2-methylindole (5.90 g; 27.4 millimoles), 13.0 g (57.6 millimoles of benzoic anhydride and 0.3 ml of a 52% aqueous solution of hydrogen iodide were reacted at 145°C for 1.5 hours in a stream of nitrogen. After the reaction, ethanol and an aqueous solution of sodium hydroxide were added. The mixture was stirred for 2 hours, extracted with ethyl acetate, washed with water, dried and concentrated to afford an oily product. The oily product was chromatographed on a silica gel column using hexane/benzene (1/1) as an eluent to afford 6.56 g (yield 75.0%) of the desired product (No. 705).

NMR($CDCl_3$, $\delta$ (ppm)):

0.83 (3H, br, t), 1.0—1.4 (4H, m), 1.60 (3H, d, J=7Hz), 1.8—2.3 (2H, m), 2.57 (3H, s), 4.53 (1H, sextet, J=7Hz), 7.0—7.96 (9H, m).

IR($CHCl_3$)$cm^{-1}$:

3070, 2950, 2900, 1620, 1580, 1520, 1460, 1270, 1170, 1105, 1060, 1020, 910, 900.

Example 71

Synthesis of 3-benzoyl-1-(2-heptyl)-2-methylindole (No. 706):—

The same reaction as in Example 70 was carried out using 2-bromoheptane to afford the desired compound (No. 706) in a yield of 72.6%.

NMR($CDCl_3$, $\delta$ (ppm)):

0.83 (3H, br, t), 1.0—1.45 (6H, m), 1.60 (3H, d, J=7Hz), 1.6—2.1 (2H, m), 2.57 (3H, s), 4.50 (1H, m), 7.0—7.95 (9H, m).

IR(liquid film)$cm^{-1}$:

3070, 3020, 2950, 2900, 1620, 1580, 1520, 1460, 1410, 1380, 1320, 1270, 1170, 1020, 900.

Example 72

Synthesis of 3-benzoyl-1-(2-butyl)-2-methylindole (No. 701):—

2.25 g (11.08 millimoles) of 1-(4-hydroxy-2-butyl)-2-methylindole, 10 ml of pyridine and 0.86 ml of methane-sulfonyl chloride were reacted at 0°C for 2 hours. After the reaction, the reaction mixture was extracted with ether, washed, dried, and chromatographed on a silica gel column using ethyl acetate/benzene (1/9) as an eluent to obtain a mesylated compound. The mesylated compound was reduced with 0.5 g of lithium aluminum hydride, and chromatographed on a silica gel column using hexane/benzene (3/1) as an eluent to afford 1.74 g of 1-(2-butyl)-2-methylindole. The compound was acylated using 4.21 g (18.6 millimoles) of benzoic anhydride and 0.15 ml of 52% hydriodic acid. After the reaction, the reaction mixture was chromatographed on a silica gel column using benzene/ethyl acetate (25/1) as an eluent to afford the desired compound (No. 701).

IR($CHCl_3$)$cm^{-1}$:

3000, 2950, 2900, 1620, 1570, 1520, 1460, 1410, 1380, 1360, 1270, 1100, 1020, 900.

NMR($CDCl_3$, $\delta$ (ppm)):

0.76 (3H, t, J=7Hz), 1.58 (3H, d, J=7Hz), 1.66—2.4 (2H, m), 2.50 (3H, s), 4.4 (1H, m), 6.73—7.8 (9H, m).

Example 73

Synthesis of 3-benzoyl-1-(2-pentyl)-2-methylindole (No. 703):—

1.09 g (4.63 millimoles) of 3-benzoyl-2-methylindole was dissolved in 5 ml of hexamethylphosphoramide, and while cooling the solution, 278 mg (5.79 millimoles) of sodium hydride (50%) was added. Under ice cooling, the mixture was stirred for 30 minutes. Then, 1.13 ml (9.26 millimoles) of 2-bromopentane was added, and the mixture was stirred at 90°C for 20 hours. After the reaction, 75 ml of water was added, and the mixture was extracted with ethyl acetate, washed with water, dried and concentrated under reduced pressure to afford an oily product. The oily product was chromatographed on a silica gel column using ethyl acetate/benzene (1/50) as an eluent to afford 118 mg (yield 8%) of the desired compound (No. 703).

NMR($CDCl_3$, δ (ppm)):
1.60 (3H, d, J=7Hz), 2.57 (3H, s), 4.55 (1H, br. sextet, J=7Hz), 6.8—7.6 (7H, m), 7.6—7.9 (2H, m).
IR($CHCl_3$)$cm^{-1}$:
2960, 1622, 1515, 1458, 1408, 1227, 1168, 745.

Example 74

Synthesis of 3-benzoyl-1-(α-phenethyl)-2-methylindole (No. 709):—

The same reaction as in Example 73 was carried out using α-phenethyl bromide to afford the compound (No. 709) in a yield of 85%.
IR(KBr)$cm^{-1}$:
3080, 3000, 1610, 1600, 1540, 1460, 1400, 1350, 1230, 1160, 1060, 1020, 920, 880, 750.
NMR($CDCl_3$, δ (ppm)):
2.00 (3H, d, J=7Hz), 2.60 (3H, s), 5.90 (1H, q, J=7Hz), 6.85—8.0 (14H, m).

Example 75

Synthesis of 3-benzoyl-1-(formyl-2-propyl)-2-methylindole (No. 806):—

402 mg (1.3 millimoles) of the compound (No. 100) obtained in Example 2 was dissolved in 10 ml of methylene chloride, and 328 mg (2.6 millimoles) of pyridine dichromate was added. The reaction was performed at room temperature for 18 hours. Ether (50 ml) was added to the reaction mixture, and insoluble materials were removed by filtration. The filtrate was concentrated under reduced pressure to afford an oily product. The oily product was chromatographed on a silica gel column using benzene/ethyl acetate (9/1) as an eluent to afford 402 mg (yield 100%) of the desired compound (No. 806).
NMR($CDCl_3$, δ (ppm)):
1.60 (3H, d, J=7Hz), 2.60 (3H, s), 2.8—3.73 (2H, ABX), 3.26 (1H, sextet, J=7Hz), 7.0—8.0 (9H, m), 9.76 (1H, s).
IR($CHCl_3$)$cm^{-1}$:
3000, 1730, 1620, 1410, 1170, 900.

Example 76

Synthesis of 3-benzoyl-2-methyl-1-(3-oxo-2-butyl)-indole (No. 800):—

400 mg (1.7 millimoles) of 3-benzoyl-2-methylindole was dissolved in 10 ml of hexamethylphosphoramide, and with stirring under ice cooling, 50 mg (0.6 equivalent) of sodium hydride was added. After generation of hydrogen ceased, 0.318 g (2.9 millimoles) of 3-chloro-2-butanone was added dropwise, and the mixture was stirred at room temperature for 1 hour. Ethyl acetate (50 ml) was added, and the mixture was washed twice with 50 ml of water, and dried over anhydrous sodium sulfate. The solvent was distilled off under reduced pressure. The resulting oily product was chromatographed on a silica gel column using ethyl acetate/benzene (1/20) as an eluent to afford 120 mg (yield 25%) of the desired compound (No. 800).
IR($CHCl_3$)$cm^{-1}$.
1720, 1623, 1600, 1510, 1458, 1400, 1366, 1260, 1220, 1169, 1060, 1021, 893, 696.
NMR($CDCl_3$, δ (ppm)):
1.66 (3H, d, J=7Hz), 1.93 (3H, s), 2.60 (3H, s). 5.00 (1H, q, J=7Hz), 7.0—7.8 (9H, m).

Example 77

Synthesis of 3-benzoyl-2-methyl-1-(4-oxo-2-pentyl)indole (No. 801):—

The same reaction as in Example 38 was performed using 3-penten-2-one to afford the desired compound (No. 801) in a yield of 23%.
NMR($CDCl_3$, δ (ppm)):
1.63 (3H, d, J=7Hz), 2.07 (3H, s), 2.64 (3H, s), 3.2—3.4 (2H, m), 5.1—5.5 (1H, m), 7.0—8.0 (9H, m).
IR($CHCl_3$)$cm^{-1}$:
3010, 1720, 1620, 1522, 1460, 1408, 1167, 1074, 900.

Example 78

*in vitro* platelet aggregation inhibiting activity:—

The platelet aggregation inhibiting activities *in vitro* of test drugs including the acylated indole derivatives of this invention were assayed using guinea pigs PRP.

The results are shown in terms of the concentration of a test drug which provided a 50% inhibition of platelet aggregation. As an aggregating agent, sodium arachidonate having a final concentration of 0.1 millimole was used. The results are shown in Table 1.

Preparation of PRP, drug, and aggregating agent

(1) Preparation of platelet-enriched plasma

Citrated blood (consisting of 1 part by volume of 3.8% sodium citrate solution and 9 parts by volume of blood) was taken by cardiac puncture from male Hartley guinea pigs weighing 350 to 600 g.

The citrated blood was centrifuged at 1000 rpm for 10 minutes at room temperature, and the supernatant liqud (PRP) was separated.

The resulting PRP was stored at room temperature, and used as early as possible. PRP stored for more than 4 hours was not used.

(2) Preparation of drugs

Each test drug was dissolved in dimethlsulfoxide generally in a concentration of 10 mg/ml. The solution was diluted with physiological saline to prepare 1 ml each of solutions having a concentration of 2500 g/ml, 1000 μg/ml, 750 μg/ml, 5000 μg/ml, 250 μg/ml, and 100 μg/ml, respectively. A compound having a free carboxylic acid as a residue, such as Indomethacin and Ibprofen, was dissolved in 0.1M $NaHCO_3$ to provide a sodium salt solution having a concentration of 10 mg/ml. The solution was similarly diluted with physiological saline to prepare a solution of a test drug.

When as a result of the platelet aggregation inhibiting test, the drug completely inhibited platelet aggregation even when the concentration was 100 μg/ml (10 μg/ml calculated as the final concentration), the drug solution was further diluted with physiological saline (75 μg/ml, 50 μg/ml, 25 μg/ml and 10 μg/ml), and the platelet aggregation inhibition test was performed further on these dilutions.

(3) Preparation of and aggregating agent

*Sodium arachidonate*

Arachidonic acid (99% pure) made by Sigma Company was dissolved in 0.1M $NaHCO_3$ to prepare a 3.3mM solution of sodium arachidonate. The solution was diluted with physiological saline to form a 1mM solution which was used in the test. The solution having a concentration of 3.3mM was stored as a base solution in a refrigerator, and the 1mM solution was newly prepared from this base solution every time it was used for the test.

*Collagen floating solution*

The collagent floating solution made by Hormone Company of West Germany was dilusted with physiological saline to a concentration of 100 μg/ml for use in the test.

Testing method for inhibition of platelet aggregation

(1) Degree of platelet aggregation in a blank

50 μl of physiological saline and 50 μl of the aggregating agent solution were added to 400 μl of PRP which had been warmed in cuvette at 37°C in an aggregometer to aggregate the platelet. The aggregation curved was recorded for 3 minutes by an aggregometer (a product of Bryston Company). The maximum degree of aggregation in this platelet aggregation was defined as the maximum degree of aggregation in the blank.

(2) Adjustment of the intensity of aggregation by sodium arachidonate (by measurement of the rate of platelet aggregation inhibition of a control compound)

50 μl of an Ibprofen solution (250 μg/ml or 100 μg/ml) was added to 400 μl of PRP, and the mixture was pre-incuvated in a cuvette of the aggregometer with stirring for 2 minutes in the same way as in (1) above. Then, 50 μl of a 1mM solution of sodium arachidonate was added, and the platelet aggregation curve was recorded in the same way as in (1) above. From the curve, the maximum degree of aggregation was calculated, and the inhibiting rate was calculated in accordance with the following equation.

$$\text{Inhibiting rate (\%)} = 100 - \frac{\text{Maximum degree of aggregation in the Ibprofen-added system}}{\text{Maximum degree of aggregation in the blank}} \times 100$$

At this time, it is necessary to confirm that the inhibiting rate of a system having added thereto 250 μg/ml of Ibprofen is at least 50%, and the inhibiting rate of a system having added thereto 100 μg/ml of Ibprofen is not more than 50%. If these inhibiting rates are not attained, the concentration of the solution of sodium arachidonate is not suitable, and therefore, its concentration should be readjusted, and the test shall be performed again.

(3) Test for inhibition of platelet aggregation

50 μl of the test compound solution was added to 400 μl of PRP, and the mixture was preincubated in the same way as in (2) above. Then, 50 μl of the solution of sodium arachidonate having its concentration adjusted in (2) was added, and the aggregation curve was recorded for 3 minutes. The maximum degree of aggregation of platelet within this period was measured, and the rate of inhibition

was calculated from the following equation. The minimum concentration (expressed as final concentration) of the drug whose inhibiting rate exceeded 50% was shown as an $IC_{50}$ value.

$$\text{Inhibiting rate (\%)} = 100 - \frac{\text{Maximum degree of aggregation in the drug-added system}}{\text{Maximum degree of aggregation in the blank}} \times 100$$

For details of the *in vitro* platelet aggretation inhibiting test, see H. M. Davis, J. Phyllis, K. Paul and W. Terry: Thromb. Res. *11*, 217—226 (1977) and R. E. Anderson and J. G. Foulks: Thromb. Haemos. *36*, 343—359 (1976).

TABLE 1

| Experiment No. | Acylated indole derivative | $IC_{50}$ ($\mu g/ml$) |
|---|---|---|
| 1 | (101) | 0.1 |
| 2 | (105) | 0.075 |
| 3 | (116) | 0.5 |
| 4 | (117) | 0.25 |
| 5 | (135) | 0.025 |
| 6 | (136) | 0.05 |
| 7 | (200) | 0.075 |
| 8 | (201) | 0.075 |
| 9 | (205) | 0.1 |
| 10 | (300) | 0.055 |
| 11 | (302) | 0.055 |
| 12 | (306) | 0.25 |
| 13 | (307) | 0.25 |
| 14 | (311) | 0.25 |
| 15 | (312) | 0.25 |
| 16 | (315) | 0.01–0.1 |
| 17 | (318) | 0.75 |
| 18 | (346) | 0.05 |
| 19 | (347) | 0.075 |
| 20 | (400) | 0.5 |
| 21 | (401) | 0.25 |
| 22 | (402) | 0.075 |
| 23 | (405) | 0.5 |
| 24 | (409) | 0.25 |
| 25 | (800) | 0.05–0.1 |
| Comparison 1 | 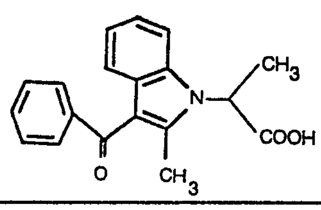 | 50 |

TABLE 1 (Continued)

| Experiment No. | Acylated indole derivative | $IC_{50}$ ($\mu g$/ml) |
|---|---|---|
| Comparison 2 | 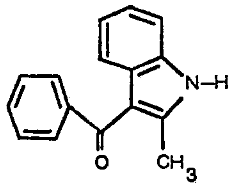 | 5 |
| Comparison 3 | 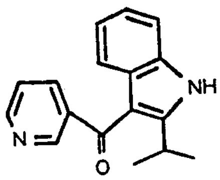 | 0.075 |

Example 79

*extra vivo* platelet aggregation inhibiting activity:—

The platelet aggregation inhibiting activities *extra vivo* of drugs sampled at random were examined using guinea pigs.

The drugs were used dissolved in propylene glycol to a concentration of 1 mg/ml (when the dosage was 1 mg/kg), 10 mg/ml (when the dosage was 10 mg/kg), and 30 mg/ml (when the dosage was 30 mg/kg). The drugs were administered orally to male Hartley guinea pigs (three in a group) having a body weight of 300 to 350 g. Propylene glycol was administered to a control group.

One hour, or 4 hours after the administration, citrated blood was taken out from the subject by cardiac puncture.

The guinea pigs were caused to fast overnight before use.

The inhibiting rate of the test drugs was measured as follows:

450 $\mu l$ of PRP was put into a cuvette of an aggregometer, and then 50 $\mu l$ of the aggregating agent was added. The aggregation curve was recorded for 3 minutes, and the maximum degree of aggregation was measured. As a blank, the maximum degree of aggregation of PRP of the group of guinea pigs to which propylene glycol had been administered was measured. The rate of inhibition was calculated from the following equation.

$$\text{Inhibition rate (\%)} = 100 - \frac{\text{Maximum degree of aggregation of PRP in the test drug-administered group}}{\text{Maximum degree of aggregation of the blank PRP}} \times 100$$

As the aggregating agent, 0.03 mM (final concentration sodium arachidonate and 5 $\mu$g/ml (final concentration) collagen floating solution were used.

The results are shown in Table 2.

For details of the *extra vivo* platelet aggregation inhibiting test, see Davis. H. M., Phyllis, J., Paul, K and Terry, W.: Thromb. Res. *11*, 217—226 (1977) and Anderson R. E. and Foulks J. G. : Thromb, Haemos. *36*, 343—359 (1976).

TABLE 2

| Experiment No. | Acylated indole derivative | Dosage (mg /kg) | Rate of inhibition of platelet aggregation (%) | | | |
|---|---|---|---|---|---|---|
| | | | Sodium arachidonate | | Collagen | |
| | | | After 1 hour | After 4 hours | After 1 hour | After 4 hours |
| 1 | (101) | 10 | 99 | 99 | 100 | 100 |
| 2 | (105) | 10 | 100 | 100 | 100 | 100 |
| 3 | (116) | 10 | 100 | 0.7 | 100 | 0 |
| 4 | (117) | 10 | 100 | 8.6 | 72.1 | 2.2 |
| 5 | (200) | 1 | 90.0 | 91.8 | – | – |
| 6 | (201) | 10 | 100 | 100 | 100 | 100 |
| 7 | (205) | 10 | 100 | 85.3 | 100 | 93.8 |
| 8 | (300) | 1 | 51.3 | 67.5 | 100 | 71.4 |
| 9 | (302) | 10 | 100 | 100 | – | – |
| 10 | (306) | 10 | 100 | 100 | 100 | 100 |
| 11 | (307) | 10 | 100 | 100 | 98.3 | 100 |
| 12 | (311) | 10 | 68.8 | 100 | 100 | 81.1 |

TABLE 2 (Continued)

| Experiment No. | Acylated indole derivative | Dosage (mg/kg) | Rate of inhibition of platelet aggregation (%) | | | |
|---|---|---|---|---|---|---|
| | | | Sodium arachidonate | | Collagen | |
| | | | After 1 hour | After 4 hours | After 1 hour | After 4 hours |
| 13 | (312) | 10 | 100 | 100 | 100 | 100 |
| 14 | (318) | 10 | 100 | 14.0 | 97.7 | 0.5 |
| 15 | (400) | 10 | 100 | 67.4 | 100 | 100 |
| 16 | (401) | 10 | 100 | 100 | 100 | 100 |
| 17 | (405) | 10 | 100 | 100 | 100 | 100 |
| 18 | (409) | 10 | 100 | 48.8 | 100 | 100 |
| 19 | (414) | 10 | 100 | 100 | – | – |
| 20 | (417) | 10 | 100 | 37 | 100 | 100 |
| 21 | (423) | 10 | 100 | 100 | 100 | 100 |
| 22 | (501) | 10 | 100 | 100 | 100 | 100 |
| 23 | (701) | 10 | 100 | 100 | – | – |
| 24 | (800) | 10 | 100 | 66.7 | 100 | 48.4 |

TABLE 2 (Continued)

| Comparison No. | Acylated indole derivative | Dosage (mg/kg) | Rate of inhibition of platelet aggregation (%) | | | |
|---|---|---|---|---|---|---|
| | | | Sodium arachidonate | | Collagen | |
| | | | After 1 hour | After 4 hours | After 1 hour | After 4 hours |
| 4 | 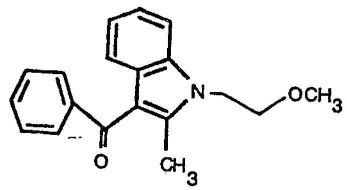 | 10 | 45.5 | 7.1 | 38.0 | 7.6 |
| 5 | 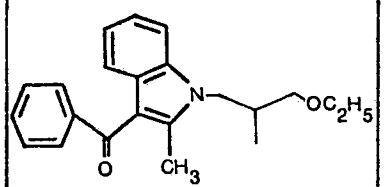 | 10 | 56.3 | 37.3 | 66.7 | 66.7 |
| 6 | 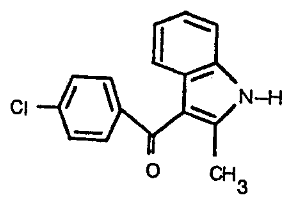 | 10 | 12.3 | 6.0 | 36.0 | 28.9 |
| 7 | 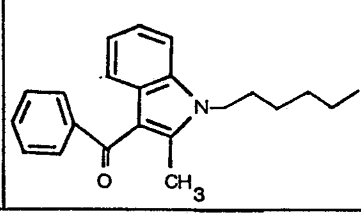 | 10 | 62.3 | 9.5 | – | – |

Example 80

Action of causing trouble to the mucous membrane of the stomach:—

Male S.D. rats (7 weeks old) were caused to fast for 24 hours while allowing them to drink water freely. A test compound dissolved in propylene glycol was orally administered to these rates, and these rats were kept while causing them to fast. Then, the rats were knocked out to death, and lesions generated in the stomach were measured in the following manner.

Ten minutes before killing the rats, 1% Evans blue was intravenously injected to the rats in a dose of 0.5 ml/rat to stain their body. Then, the rats were knocked out to death, and the stomach was taken out from each rat. 12 ml of 1% formalin was injected into the stomach to inflate it. The stomach was then dipped for more than 10 minutes in a 1% formalin solution, and fixed. After fixing, the stomach was incised along the glandular portion, and the length of a dye-leaked portion that occurred in the stomach was measured under a solid microscope, and defined as the ulcer index (mm). The results are shown in Table 3.

For details of the test on the action of causing trouble to the mucous membrane of the stomach, see. E. Adami, E. Marazzi-Uberti, and C. Turba, Arch, int. Pharmacadyn., *147* (1964).

TABLE 3

| Experiment No. | Acylated indole derivative | Dosage (mg /kg) | Number of the rats | Ulcer Index (mm ± S.E.) | Incidence (%) |
|---|---|---|---|---|---|
| 1 | (300) | 100 | 10 | 1.1 ± 0.5 | 40 |
| | | 200 | 9 | 1.2 ± 0.3 | 55.6 |
| | | 400 | 9 | 1.4 ± 0.5 | 66.7 |
| 2 | (302) | 200 | 6 | 0.3 ± 0.2 | 33.3 |
| | | 400 | 10 | 0.9 ± 0.3 | 50 |
| 3 | (705) | 400 | 6 | 0 | 0 |
| 4 | (706) | 400 | 6 | 0.1 | 16.7 |
| Comparison 3 | 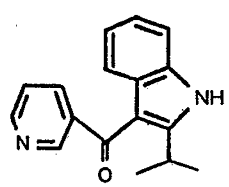 | 100 | 6 | 1.5 ± 0.6 | 66.7 |
| | | 400 | 6 | 7.9 ± 2.3 | 100 |
| 5 | Control a) | — | 7 | 0 | 0 |

a) : Propyleneglycol 2ml /kg

## Example 81

Inclusion compound of the acylated indole derivative of this invention with cyclodextrin:—

Ten grams of the compound (No. 300) of this invention and 55.41 g [1.5 moles per mole of the compound (No. 300)] were mixed well in a mortar. Then 25 ml of water was added, and the mixture was stirred for 30 minutes. The mixture was then air-dried, finely pulverized and sieved to form an inclusion compound of the compound (No. 300).

In the same way as above, inclusion compounds of the compounds Nos. (105), (302), (307), (312) with cyclodextrin.

## Example 82

Forty parts by weight of microcrystalline cellulose, 5 parts by weight of carboxymethyl cellulose calcium and 0.5 part by weight of magnesium stearate were added to 40 parts by weight of the inclusion compound of the compound (No. 300) obtained in Example 81, and they were well mixed. The mixture was formed into tablets in a customary manner.

## Example 83

Four parts by weight of 3% starch paste was added to 4 parts by weight of the inclusion compound of the compound (No. 300) obtained in Example 81. The mixture was extruded by a granulator to form granules.

## Example 84

Preparations of tablets:—

Tablets were prepared each of which had the following composition.

| | |
|---|---|
| Active ingredient | 100 mg |
| Lactose | 280 mg |
| Potato starch | 80 mg |
| Polyvinyl pyrrolidone | 11 mg |
| Magnesium stearate | 5 mg |
| | 476 mg |

The active ingredient, lactose and potato starch were mixed, and the mixture was equally wetted with a 20% ethanol solution of polyvinyl pyrrolidone. It was passed through a sieve having a mesh opening of 2.0 mm and dried at 45°C. The dried product was again passed through a sieve having a mesh opening of 1.5 mm. The resulting granules were mixed with magnesium stearate, and compressed into tablets.

Compounds (No. 300) and (No. 302) were used as typical examples of the active ingredient.

## Example 85

Preparation of ampoules:—

Ampoules each of which had a capacity of 5 ml and the following composition were prepared.

| | |
|---|---|
| Active ingredient | 100 mg |
| Polyethylene glycol 600 | 200 mg |
| Distilled water to make | 5.0 ml |

Polyethylene glycol and the active ingredient were dissolved in water under nitrogen atmosphere, and boiled. They were cooled under nitrogen, and distilled. Pre-treated water was added to the solution to adjust the total volume to 5.0 ml, and the solution was filtered in an aseptic condition. Production of the solution in this Example was carried out under scattering light.

Filling of the solution into ampoules was carried out in a stream of nitrogen, and sterilization was carried out at 121°C for 20 minutes.

Compounds (No. 400) and (No. 402) were used as the active ingredients.

Example 86

Preparation of capsules:—

Hard gelation capsules were prepared each of which had the following composition.

| | |
|---|---|
| Active ingredient | 100 mg |
| Microcrystalline cellulose | 195 mg |
| Amorphous silica | 5 mg |
| | 300 mg |

The finely divided active ingredient, the microcrystalline cellulose and the unpressed amorphous silica were fully mixed, and packed into hard gelatin capsules.

Compounds (No. 300) and (No. 302) were used typically as the active ingredient. No experimental trouble occurred during the production.

**Claims**

1. An acylated indole derivative of the formula

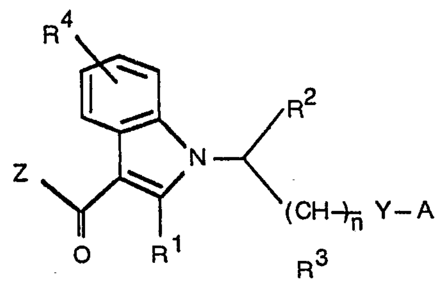

[I]

wherein $R^1$ represents hydrogen or alkyl of 1 to 6 carbon atoms, $R^2$ represents alkyl of 1 to 6 carbon atoms, $R^3$ represents hydrogen or alkyl of 1 to 6 carbon atoms, $R^4$ represents hydrogen, hydroxyl, halogen, alkyl of 1 to 6 carbon atoms, or alkoxy of 1 to 6 carbon atoms, Z represents phenyl or thienyl optionally substituted by the group $R^4$, Y represents a bond, —O—, —S—, —SO—, or —$SO_2$—, A represents hydrogen, alkyl of 1 to 6 carbon atoms, acyl of 1 to 6 carbon atoms, phenyl, phenylalkyl, pyridinyl, imidazolyl or thiadiazolyl, or phenyl, phenylalkyl, pyridinyl, imidazolyl or thiadiazolyl substituted by the group $R^4$, and n represents zero or an integer of 1 to 10 provided that when n is zero, Y represents a bond and A represents acyl of 1 to 6 carbon atoms;

or an optically active isomer thereof or a salt thereof.

2. A compound according to claim 1 wherein $R^1$ represents methyl.

3. A compound according to claim 1 or 2 wherein $R^2$ represents an alkyl group having 1 to 3 carbon atoms.

4. A compound according to claim 1 wherein n is zero or an integer of 1 to 3.

5. A compound according to claim 1 wherein said acylated indole derivative is represented by the formula

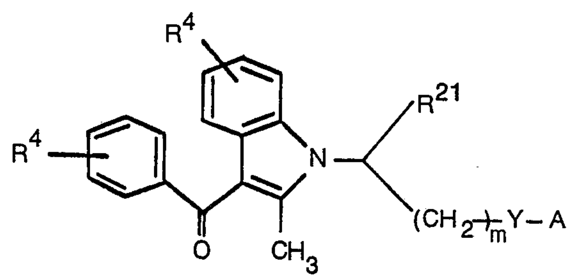

[I]-1

wherein $R^{21}$ represents an alkyl group having 1 to 3 carbon atoms, the two $R^4$'s are identical or different, $R^4$, Y and A are as defined in claim 1, and m represents zero or an integer of 1 to 3, provided that when m is zero, Y represents a bond and A represents an acyl group.

6. A compound according to claim 5 wherein said acylated indole derivative is represented by the formula

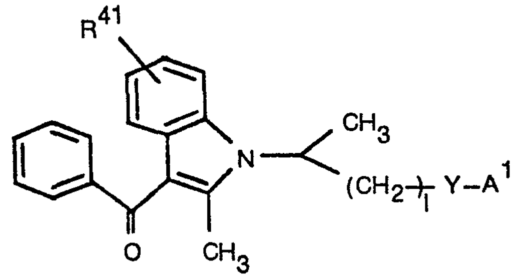

[I]-2

wherein $R^{41}$ represents a hydrogen atom, a hydroxyl group, a chlorine atom, a methyl group or a methoxy group, $A^1$ represents a hydrogen atom, an alkyl group having 1 to 4 carbon atoms, an acyl group having 1 to 4 carbon atoms, a phenyl, pyridinyl, imidazolyl or thiadiazolyl group, and l is 0, 1 or 2 provided that when l is 0, Y represents a bond and $A^1$ represents said acyl group.

7. A process for producing an acylated indole derivative of the formula

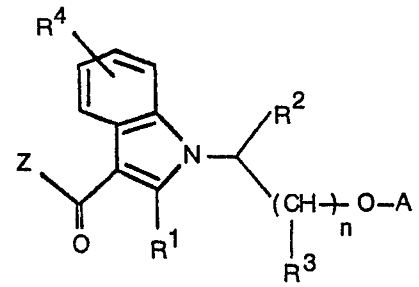

[I]-a

wherein $R^1$, $R^2$, $R^3$, $R^4$ and Z are as defined in claim 1, A is as defined in claim 1 except that $R^4$ when present as a substituent does not represent hydroxyl, and n represents an integer of 1 to 10, which process comprises condensing an indole derivative of the formula

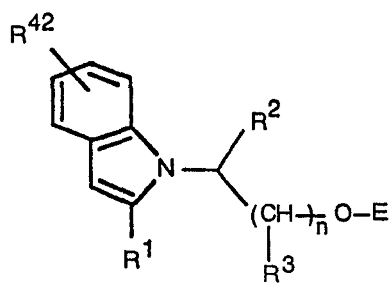

[II]-a

wherein $R^{42}$ represents hydrogen, halogen, alkyl or alkoxy or 1 to 6 carbon atoms, and E represents alkyl of 1 to 6 carbon atoms, acyl of 1 to 6 carbon atoms, phenyl, phenylalkyl, pyridinyl, imidazolyl, or thiadiazolyl, or phenyl, phenylalkyl, pyridinyl, imidazolyl, or thiadiazolyl substituted by the group $R^{42}$,

with an acid anhydride of the formula

$$Z^1-\underset{\underset{O}{\|}}{C}-O-\underset{\underset{O}{\|}}{C}-Z^1 \qquad [III]$$

wherein $Z^1$ represents a phenyl or thienyl group or a phenyl or thienyl group substituted by the group $R^{42}$ or a protected hydroxyl group,

in the presence of hydrogen iodide, and if desired, hydrolyzing the resulting product.

8. A process for producing an acylated indole derivative of the formula

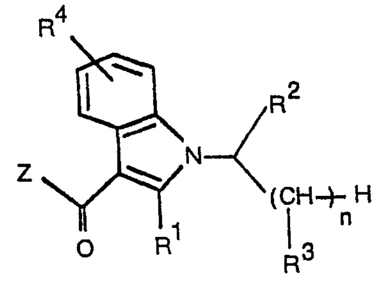

[I]-b

wherein $R^1$, $R^2$, $R^3$ $R^4$, n and Z are as defined with respect to claim 7. which process comprises condensing an indole derivative of the formula

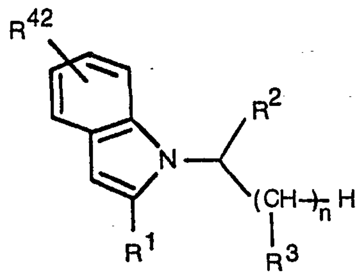

[II]-b

wherein $R^{42}$ is as defined in claim 7, with an acid anhydride of the formula

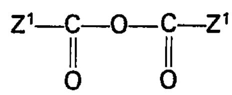

[III]

wherein $Z^1$ is as defined in claim 7, in the presence of hydrogen iodide, and if desired, hydrolyzing the resulting product.

9. A process for producing an acylated indole derivative of the formula

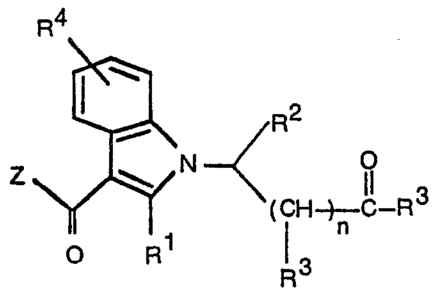

[I]-c

wherein $R^1$, $R^2$, $R^3$, $R^4$, Z and n are as defined with respect to claim 7, which process comprises condensing an indole derivative of the formula

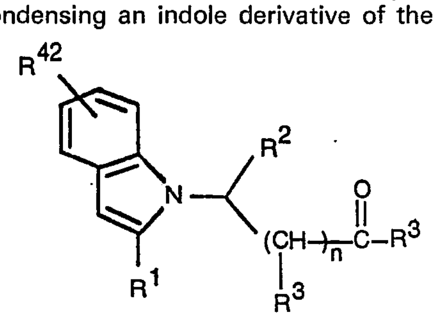

[II]-c

wherein $R^{42}$ is as defined in claim 7, with an acid anhydride of the formula

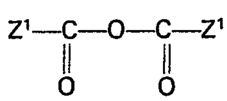

[III]

wherein $Z^1$ is as defined in claim 7, in the presence of hydrogen iodide, and if desired, hydrolyzing the resulting product.

10. A process for producing an acylated indole derivative of the formula

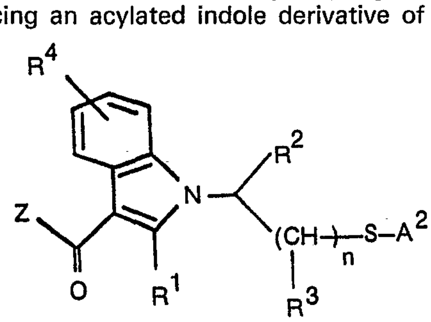

[I]-d

wherein $R^1$, $R^2$, $R^3$, $R^4$, n and Z are as defined with respect to claim 7, and $A^2$ is as defined below, which process comprises condensing an N-(hydroxyalkyl) acylated indole derivative of the formula

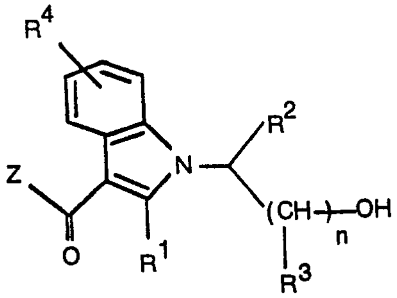

[I]-$a_1$

with an organic disulfide compound of the formula

$$A^2—SS—A^2 \qquad [IV]$$

wherein $A^2$ represents alkyl of 1 to 6 carbon atoms, or phenyl, phenylalkyl, pyridinyl, imidazolyl or thiadiazolyl optionally substituted by a protected hydroxyl group or $R^{42}$ as defined in claim 7, in the presence of an organic phosphine compound and a nitrogen-containing basic compound.

11. A process for producing an acylated indole derivative of the formula

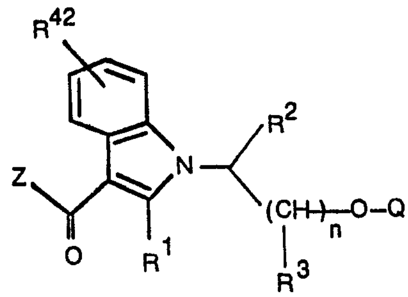

[I]-e

wherein $R^1$, $R^2$, $R^3$, $R^{42}$, n and Z are as defined with respect to claim 7, and Q represents alkyl of 1 to 6 carbon atoms, a phenylalkyl group, or a phenylalkyl group substituted by $R^{42}$, which process comprises condensing an N-(hydroxyalkyl) acylated indole derivative of the formula

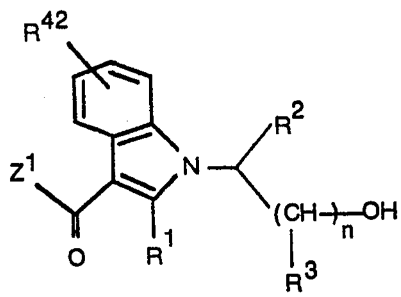

[I]-$a_2$

wherein $Z^1$ is as defined in claim 7, with a halogen compound of the formula

$$Q—X \qquad [V]$$

wherein Q is as defined above, and X represents a halogen atom, in the presence of a basic compound, and if desired, hydrolyzing the resulting product.

12. A process for producing an acylated indole derivative of the formula

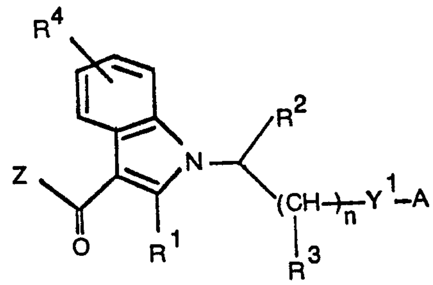

[I]-f

wherein $Y^1$ represents a bond or —O—, and $R^1$, $R^2$, $R^3$, $R^4$, n, A and Z are as defined with respect to claim 7,

which process comprises condensing a tetrahydroindole derivative of the formula

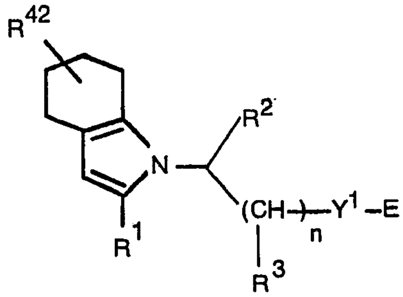

[VI]

wherein $R^{42}$ and E are as defined with respect to claim 7,

with an acid anhydride of the formula

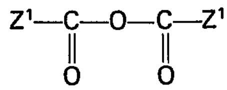

[III]

wherein $Z^1$ is as defined in claim 7,

in the presence of hydrogen iodide to form an acylated tetrahydroindole derivative of the following formula

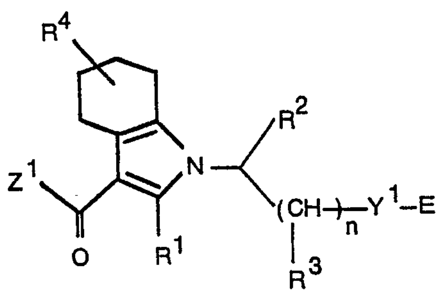

[VII]

and then dehydrogenating the resulting product, and if desired, hydrolyzing the dehydrogenation product.

13. A process for producing acylated indole derivative of the formula

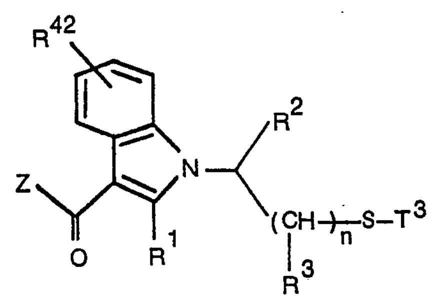

[I]-g

wherein $R^1$, $R^2$, $R^3$, $R^{42}$, n and Z are as defined with respect to claim 7, and $T^3$ represents a hydrogen atom, or $T^2$ defined below,

which process comprises reacting an N-(halo- or sulfonyloxyalkyl) acylated indole derivative of the formula

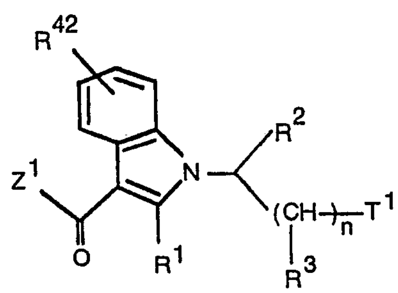

[VIII]

wherein $Z^1$ is as defined in claim 7, and $T^1$ represents a halogen atom,

$—OSO_2CH_3$, or $—OSO_2$ 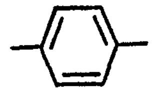 $CH_3$,

with a thiocarboxylic acid or thiourea or an alkali metal salt thereof having the formula

$$T^2—S—M \qquad [IX]$$

wherein $T^2$ represents a lower acyl or amidino group, and M represents a hydrogen atom or an alkali metal, and then if desired, hydrolyzing the resulting product.

14. A process for producing an acylated indole derivative of the formula

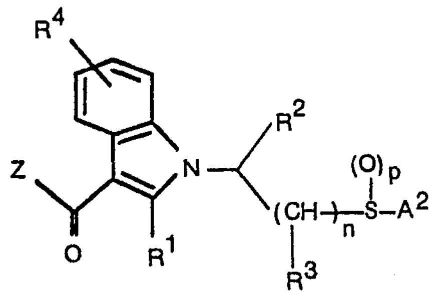

[I]–h

wherein $R^1$, $R^2$, $R^3$, $R^4$, n and Z are as defined with respect to claim 7, and $A^2$ is as defined in claim 10, and p is 1 or 2, which process comprises oxidizing an acylated indole derivative of the formula

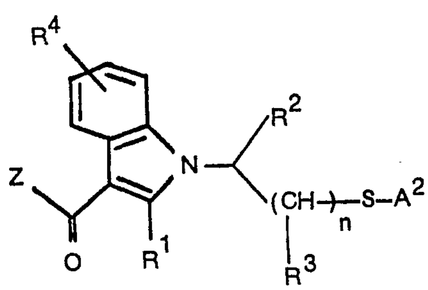

[I]–d

in a manner known *per se*.

15. A pharmaceutical composition for use in the inhibition of platelet aggregation, comprising (1) a pharmaceutically effective amount of an acylated indole derivative of formula [I]—1 as defined in claim 5 or an optically active isomer thereof or a salt thereof, and (2) a pharmaceutically acceptable carrier.

16. A pharmaceutical composition for use in the inhibition of platelet aggregation, comprising (1) a pharmaceutically effective amount of an acylated indole derivative of the formula [I]—2 as defined in claim 6 or an optically active isomer thereof or a salt thereof, and (2) a pharmaceutically acceptable carrier.

17. A drug for use in the inhibition of platelet aggregation comprising a pharmaceutically effective amount of an acylated indole derivative of formula [I]—a as defined in claim 7 or an optically active isomer thereof or a salt thereof, and a pharmaceutically acceptable carrier.

18. A drug according to claim 17 in a form for oral administration.

**Revendications**

1. Dérivés de l'indole acylés ayant la formule:

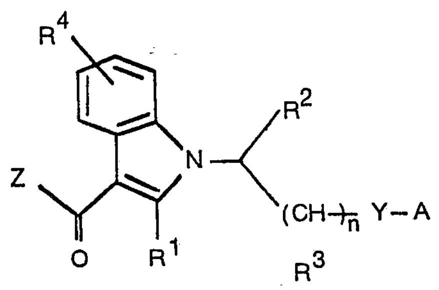

[I]

dans laquelle $R^1$ représente de l'hydrogène ou un groupe alkyle ayant 1 à 6 atomes de carbone; $R^2$ représente un groupe alkyle ayant 1 à 6 atomes de carbone; $R^3$ représente de l'hydrogène ou un groupe alkyle ayant 1 à 6 atomes de carbone; $R^4$ représente de l'hydrogène, un groupe hydroxyle, halogéno, alkyle ayant 1 à 6 atomes de carbone ou alcoxy ayant 1 à 6 atomes de carbone; Z représente un group phényle ou thiényle éventuellement substitués par le groupe $R^4$; Y représente une liaison, —O—, —S—, —SO—, ou —$SO_2$—; A représente de l'hydrogène, un groupe alkyle ayant 1 à 6 atomes de carbone, acyle ayant 1 à 6 atomes de carbone, phényle, phénylalkyle, pyridinyle, imidazolyle ou thiadiazolyle, ou phényle, phénylalkyle, pyridinyle, imidazolyle ou thiadiazolyle substitués par le groupe $R^4$, et n représente zéro ou un nombre entier de 1 à 10 à condition que lorsque n est zéro, Y représente une liaison et A représente un groupe acyle ayant 1 à 6 atomes de carbone; ou bien un isomère optiquement actif de ces dérivés ou un de leurs sels.

2. Composé selon la revendication 1 dans lequel $R^1$ représente le groupe méthyle.

3. Composé selon les revendications 1 ou 2, dans lequel $R^2$ représente un groupe alkyle ayant 1 à 3 atomes de carbone.

4. Composé selon la revendication 1, dans lequel n est zéro ou un nombre entier de 1 à 3.

5. Composé selon la revendication 1, dans lequel ledit dérivé de l'indole acylé est représenté par la formule:

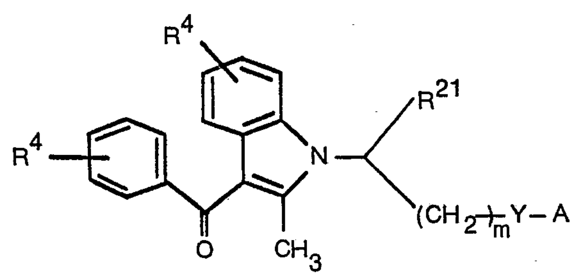

[I]-1

dans laquelle $R^{21}$ représente un groupe alkyle ayant 1 à 3 atomes de carbone, les deux $R^4$ sont identiques ou différents; $R^4$, Y et A sont tels que définis dans la revendication 1, et m représente zéro ou un nombre entier de 1 à 3, à condition que si m est zéro, Y représente un liaison et A représente un groupe acyle.

6. Composé selon la revendication 5, dans lequel ledit dérivé de l'indole acylé est représenté par la formule:

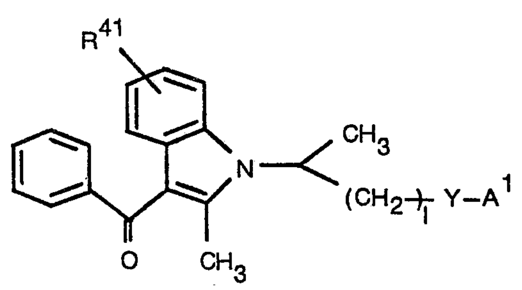

[I]-2

dans laquelle $R^{41}$ représente un atome d'hydrogène, un groupe hydroxyle, un atome de chlore, un groupe méthyle ou un groupe méthoxy; $A^1$ représente un atome d'hydrogène, un groupe alkyle ayant 1 à 4 atomes de carbone, un groupe acyle ayant 1 à 4 atomes de carbone, un groupe phényle, pyridinyle, imidazolyle ou thiadiazolyle et l est 0, 1 ou 2 à condition que lorsque l est 0, Y répresente un liaison et $A^1$ ledit groupe acyle.

7. Procédé pour la préparation d'un dérivé de l'indole acylé ayant la formule:

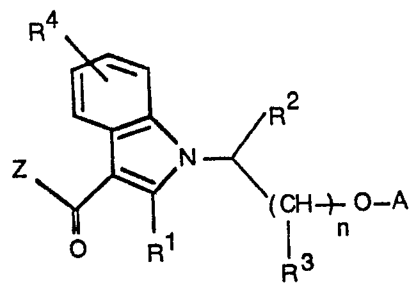

[I]–a

dans laquelle $R^1$, $R^2$, $R^3$, $R^4$ et Z sont tels que définis dans la revendication 1; A est tel que défini dans la revendication 1 sauf que $R^4$, quand il est présent comme substituant, ne représente pas de groupe hydroxyle, et n représente un nombre entier de 1 à 10, procédé qui comprend la condensation d'un dérivé de l'indole ayant le formule:

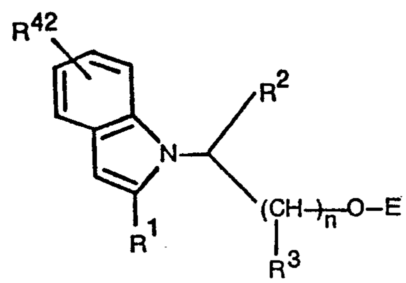

[II]–a

dans laquelle $R^{42}$ représente de l'hydrogène, un groupe halogèno, un groupe alkyle ou alcoxy ayant 1 à 6 atomes de carbone, et E représente un groupe alkyle ayant 1 à 6 atomes de carbone, acyle ayant 1 à 6 atomes de carbone, phényle, phénylalkyle, pyridinyle, imidazolyle ou thiadiazolyle, ou bien phényle, phénylalkyle, pyridinyle, imidazolyle ou thiadiazolyle, substitués par le groupe $R^{42}$, avec un anhydride d'acide ayant la formule:

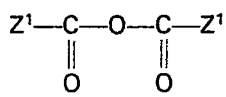

[III]

dans laquelle $Z^1$ représente un groupe phényle ou thiényle ou bien un groupe phényle ou thiényle, substitués par le groupe $R^{42}$, ou un bien un groupe hydroxyle protégé, en présence d'acide iodhydrique et, si on le désire, l'hydrolyse du produit résultant.

8. Procédé pour la préparation d'un dérivé de l'indole acylé ayant la formule:

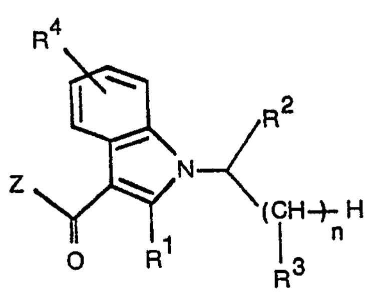

[I]–b

dans laquelle $R^1$, $R^2$, $R^3$, $R^4$, n et Z sont tels que définis par rapport à la revendication 7,

procédé qui comprend la condensation d'un dérivé de l'indole ayant la formule:

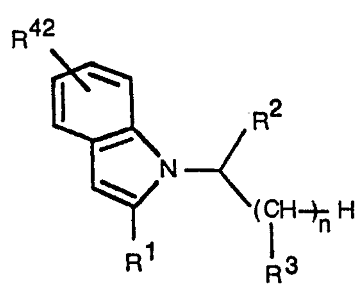

[II]–b

dans laquelle $R^{42}$ est tel que défini dans la revendication 7, avec un anhydride d'acide ayant la formule:

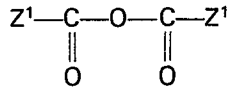

[III]

dans laquelle $Z^1$ est tel que défini dans la revendication 7, en présence d'acide iodhydrique et, si on le désire, l'hydrolyse du produit résultant.

9. Procédé pour la préparation d'un dérivé de l'indole acylé ayant la formule:

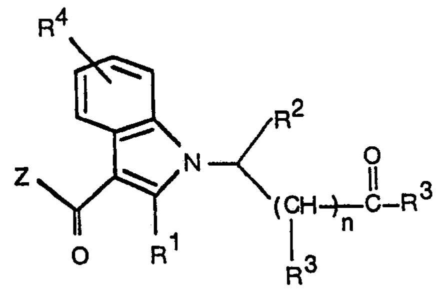

[I]-c

dans laquelle $R^1$, $R^2$, $R^3$, $R^4$, Z et n sont tels que définis par rapport à la revendication 7, procédé qui comprend la condensation d'un dérivé de l'indole ayant la formule:

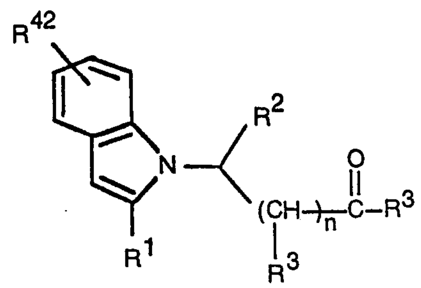

[II]-c

dans laquelle $R^{42}$ est tel que défini dans la revendication 7, avec un anhydride d'acide ayant la formule:

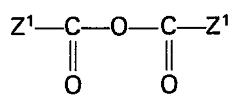

[III]

dans laquelle $Z^1$ est tel que défini dans la revendication 7, en présence d'acide iodhydrique et, si on le désire, l'hydrolyse du produit résultant.

10. Procédé pour la préparation d'un dérivé de l'indole acylé ayant la formule:

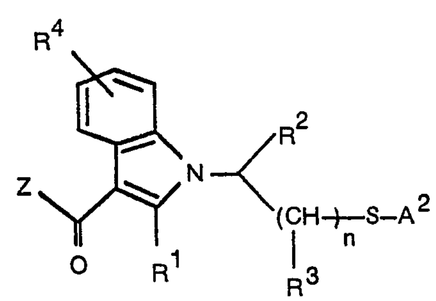

[I]-d

dans laquelle $R^1$, $R^2$, $R^3$, $R^4$, n et Z sont tels que définis par rapport à la revendication 7, et $A^2$ est tel que défini ci-dessous, procédé qui comprend la condensation d'un dérivé de indole N-(hydroxyalkyl)acylé ayant la formule:

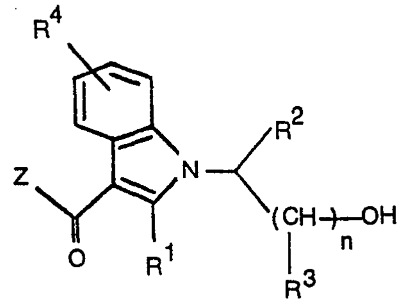

[I]-$a_1$

avec un composé disulfure organique ayant la formule:

$$A^2—SS—A^2 \qquad [IV]$$

dans laquelle $A^2$ représente un groupe alkyle ayant 1 à 6 atomes de carbone ou bien un groupe phényle, phénylalkyle, pyridinyle, imidazolyle ou thiadiazolyle, éventuellement substitués par un groupe hydroxyle protégé, ou $R^{42}$ tel que défini dans la revendication 7,
en présence d'un composé phosphine organique et d'un composé basique azoté.

11. Procédé pour la préparation d'un dérivé de l'indole acylé ayant la formule:

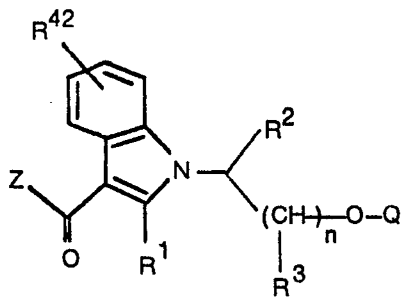

[I]-e

dans laquelle $R^1$, $R^2$, $R^3$, $R^{42}$, n et Z sont tels que définis par rapport à la revendication 7, et Q représente un groupe alkyle ayant 1 à 6 atomes de carbone, un groupe phénylalkyle ou un groupe phénylalkyle substitué par $R^{42}$,
procédé qui comprend la condensation d'un dérivé de l'indole N-(hydroxyalkyl) acylé ayant la formule:

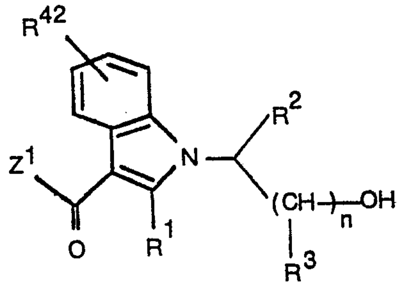

[I]-$a_2$

dans laquelle $Z^1$ est tel que défini dans la revendication 7, avec un composé halogéné ayant la formule:

$$Q—X \qquad [V]$$

dans laquelle Q est tel que défini ci-dessus, et X représente un atome d'halogène,
en présence d'un composé basique et, si on le désire, l'hydrolyse du produit résultant.

12. Procédé pour la préparation d'un dérivé de l'indole acylé ayant la formule:

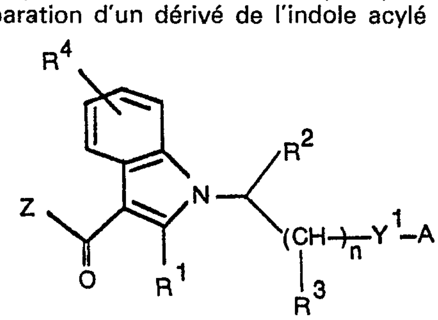

[I]-f

dans laquelle $Y^1$ représente un liaison ou —O—, et $R^1$, $R^2$, $R^3$, $R^4$, n, A et Z sont tels que définis par rapport à la revendication 7, procédé qui comprend la condensation d'un dérivé du tétrahydroindole ayant la formule:

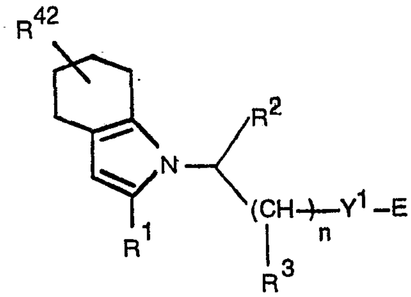

[VI]

dans laquelle $R^{42}$ et E sont tels que définis par rapport à la revendication 7, avec un anhydride d'acide ayant la formule:

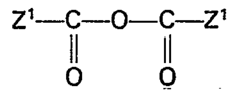

[III]

dans laquelle $Z^1$ est tel que défini dans la revendication 7, en présence d'acide iodhydrique pour former un dérivé de tétrahydroindole acylé ayant la formule suivante:

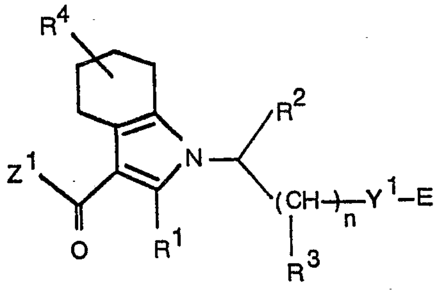

[VII]

puis la déshydrogénation du produit résultant et, si on le désire l'hydrolyse du produit de déshydrogénation.

13. Procédé pour la préparation d'un dérivé de l'indole acylé ayant la formule:

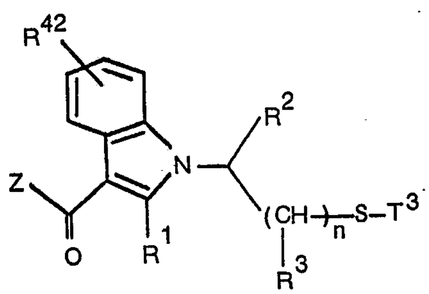

[I]–g

dans laquelle $R^1$, $R^2$, $R^3$, $R^{42}$, n et Z sont tels que définis par rapport à la revendication 7, et $T_3$ représente un atome d'hydrogène, ou bien $T^2$ défini ci-dessous, procédé qui comprend la réaction d'un dérivé de l'indole N-(halo- ou sulfonyloxy-alkyl) acylé ayant la formule:

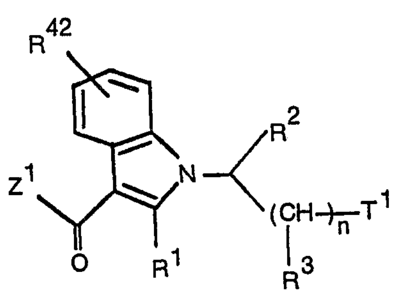

[VIII]

dans laquelle $Z^1$ est tel que défini dans la revendication 7, et $T^1$ représente un atome d'halogène, un groupe

—$OSO_2CH_3$, ou 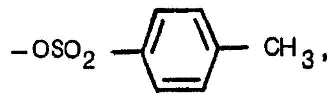

avec un acide thiocarboxylique ou une thiourée ou un sel de métal alcalin de cet acide ayant la formule:

$$T^2—S—M \quad [IX]$$

dans laquelle $T^2$ représente un groupe acyle inférieur ou amidino, et M représente un atome d'hydrogène ou un métal alcalin puis, si on le désire, l'hydrolyse du produit résultant.

14. Procéde pour la préparation d'un dérivé de l'indole acylé ayant la formule:

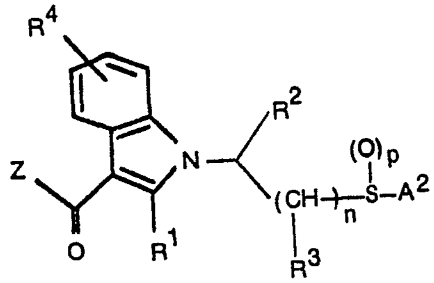

[I]-h

dans laquelle $R^1$, $R^2$, $R^3$, $R^4$, n et Z sont tels que définis par rapport à la revendication 7, et $A^2$ est tel que défini dans la revendication 10, et p est 1 ou 2,
procédé qui comprend l'oxydation d'un dérivé de l'indole acylé ayant la formule

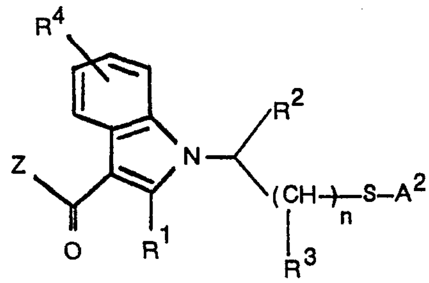

[I]-d

de façon connue en soi.

15. Composition pharmaceutique pour usage dans l'inhibition de l'agglomération des plaquettes, comprenant (1) une quantité pharmaceutiquement efficace d'un dérivé de l'indole acylé de formule [I]—1 tel que défini dans la revendication 5 ou d'un isomère optiquement actif de ce dérivé ou d'un de ses sels, et (2) un véhicule pharmaceutiquement acceptable.

16. Composition pharmaceutique pour usage dans l'inhibition de l'agglomération des plaquettes, comprenant (1) une quantité pharmaceutiquement efficace d'un dérivé de l'indole acylé ayant la formule [I]—2 tel que défini dans la revendication 6, ou d'un isomère optiquement actif de ce dérivé ou d'un de ses sels, et (2) un véhicule pharmaceutiquement acceptable.

17. Médicament pour usage dans l'inhibition de l'agglomération des plaquettes comprenant une quantité pharmaceutiquement efficace d'un dérivé de l'indole acylé ayant la formule [I]—a tel que défini dans la revendication 7 ou d'un isomère optiquement actif de ce dérivé ou d'un de ses sels, et un véhicule pharmaceutiquement acceptable.

18. Médicament selon la revendication 17 sous une forme pour l'administration orale.

**Patentansprüche**

1. Acyliertes Indol-Derivat der Formel

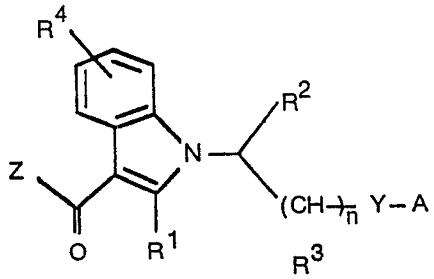

[I]

in der $R^1$ ein Wassertoffatom oder eine Alkylgruppe mit 1 bis 6 Kohlenstoffatomen, $R^2$ eine Alkylgruppe mit 1 bis 6 Kohlenstoffatomen, $R^3$ ein Wasserstoffatom oder eine Alkylgruppe mit 1 bis 6 Kohlenstoffatomen, $R^4$ ein Wasserstoffatom, eine Hydroxylgruppe, ein Halogenatom, eine Alkylgruppe mit 1 bis 6 Kohlenstoffatomen oder eine Alkoxygruppe mit 1 bis 6 Kohlenstoffatomen, Z eine Phenyl- oder Thienylgruppe, die gegebenenfalls durch die Gruppe $R^4$ substituiert ist, Y eine Bindung, —O—, —S—, —SO—, oder —$SO_2$—, A ein Wasserstoffatom, eine Alkylgruppe mit 1 bis 6 Kohlenstoffatomen, eine Acylgruppe mit 1 bis 6 Kohlenstoffatomen, eine Phenyl; Phenylalkyl; Pyridinyl; Imidazolyl- oder Thiadiazolylgruppe, oder eine Phenyl-, Phenylalkyl-, Pyridinyl-, Imidazolyl- oder Thiadiazolylgruppe die durch die Gruppe $R^4$ substituiert ist, und n O oder eine ganze Zahl von 1 bis 10 bedeutet, vorausgesetzt daß, wenn n O ist, Y eine Bindung and A eine Acylgruppe mit 1 bis 6 Kohlenstoffatomen bedeutet, oder ein optisch aktives Isomer, oder ein Salz davon.

2. Verbindung nach Anspruch 1, wobei $R^1$ eine Methylgruppe bedeutet.

3. Verbindung nach Anspruch 1 oder 2, wobei $R^2$ eine Alkylgruppe mit 1 bis 3 Kohlenstoffatomen bedeutet.

4. Verbindung nach Anspruch 1, wobei n, O oder eine ganze Zahl von 1 bis 3 ist.

5. Verbindung nach Anspruch 1, wobei das acylierte Indol-Derivat angegeben wird durch die Formel

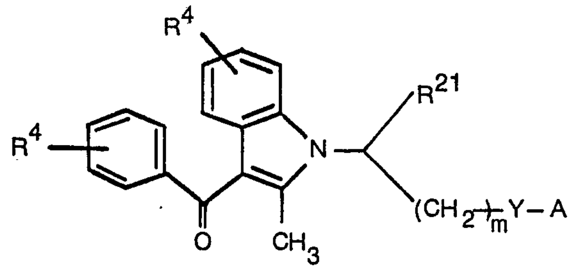

[I]–1

in der $R^{21}$ eine Alkylgruppe mit 1 bis 3 Kohlenstoffatomen bedeutet, die beiden Reste $R^4$ gleich oder verschieden sind und $R^4$, Y und A, die in Anspruch 1 angegebene Bedeutung haben und m O oder eine ganze Zahl von 1 bis 3 bedeutet, unter der Voraussetzung, daß, wenn m O ist, Y eine Bindung und A eine Acylgruppe bedeutet.

6. Verbindung nach Anspruch 5, wobei das acylierte Indol-Derivat angegeben wird durch die Formel

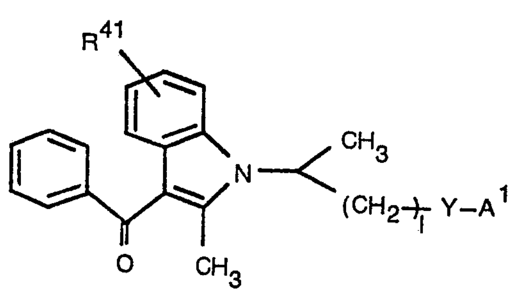

[I]–2

in der $R^{41}$ ein Wasserstoffatom, eine Hydroxylgruppe, ein Chloratom, eine Methylgruppe oder eine Methoxygruppe, $A^1$ ein Wasserstoffatom, eine Alkylgruppe mit 1 bis 4 Kohlenstoffatomen, eine Acylgruppe mit 1 bis 4 Kohlenstoffatomen, eine Phenyl-Pyridinyl-, Imidazolyl-oder Thiadiazolylgruppe und l O, 1 oder 2 bedeutet, vorausgesetzt daß, wenn l O ist, Y eine Bindung darstellt und $A^1$ die Acylgruppe ist.

7. Verfahren zur Herstellung eines acylierten Indol-Derivats der Formel

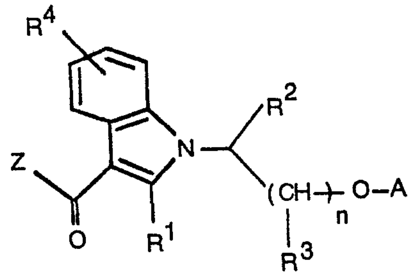

[I]-a

in der $R^1$, $R^2$, $R^3$, $R^4$ und Z die in Anspruch 1 angegebene Bedeutung haben, A die in Anspruch 1 angegebene Bedeutung hat, mit der Ausnahme, daß $R^4$, soweit es als Substituent vorhanden ist, keine Hydroxylgruppe bedeutet, und n eine ganze Zahle von 1 bis 10 ist, wobei man ein Indol-Derivat der Formel

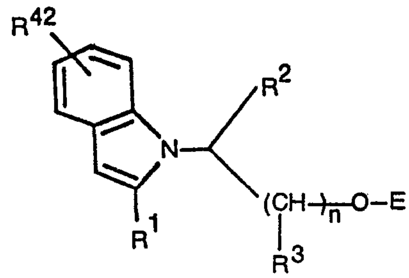

[II]-a

in der $R^{42}$ ein Wasserstoff-oder Halogenatom, oder eine Alkyl-oder Alkoxygruppe mit 1 bis 6 Kohlenstoffatomen und E eine Alkylgruppe mit 1 bis 6 Kohlenstoffatomen, eine Acylgruppe mit 1 bis 6 Kohlenstoffatomen, eine Phenyl-, Phenylalkyl-, Pyridinyl-, Imidazolyl- oder Thiadiazolylgruppe, oder eine Phenyl-, Phenylalkyl-, Pyridinyl-, Imidazolyl- oder Thiadiazolylgruppe, die durch die Gruppe $R^{42}$ substituiert ist, bedeutet, kondensiert mit einem Säureanhydrid der Formel

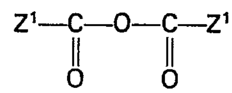

(III)

in der $Z^1$ eine Phenyl-oder Thienylgruppe, oder eine Phenyl-oder Thienylgruppe, die durch die Gruppe $R^{42}$, oder eine geschützte Hydroxylgruppe substituiert ist, bedeutet, und gegebenenfalls das erhaltene Produkt hydrolysiert.

8. Verfahren zur Herstellung eines acylierten Indol-Derivats der Formel

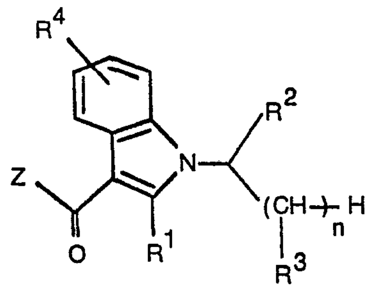

[I]-b

in der $R^1$, $R^2$, $R^3$, $R^4$, n und Z die in Anspruch 7 angegebene Bedeutung haben, wobei man ein Indol-Derivat der Formel

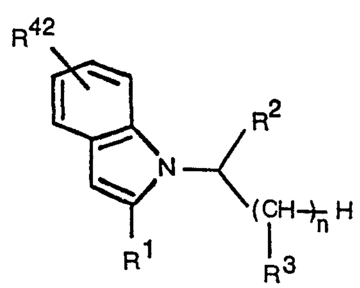

[II]-b

in der $R^{42}$ die in Anspruch 7 angegebene Bedeutung hat, kondensiert mit einem Säure anhydrid der Formel

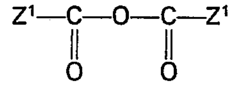

(III)

in der $Z^1$ die in Anspruch 7 angegebene Bedeutung hat, in Gegenwart von Jodwasserstoff und gegebenenfalls das erhaltene Produkt hydrolysiert.

9. Verfahren zur Herstellung eines acylierten Indol-Derivats der Formel

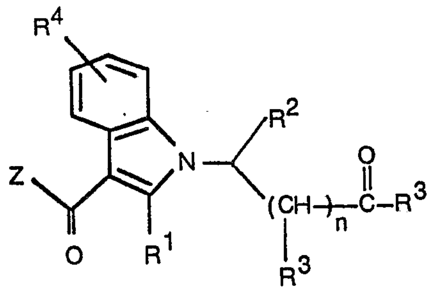

[I]−c

in der $R^1$, $R^2$, $R^3$, $R^4$, Z und n die in Anspruch 7 angegebene Bedeutung haben, wobei man ein Indol-Derivat der Formel

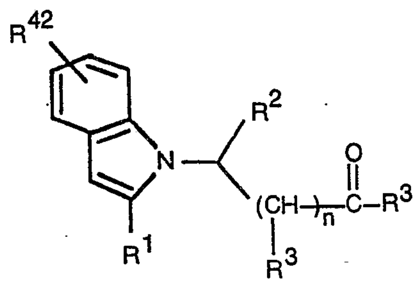

[II]−c

in der $R^{42}$ die in Anspruch 7 angegebene Bedeutung hat, kondensiert mit einem Säureanhydrid der Formel

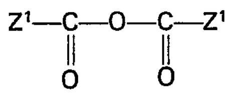

(III)

in der $Z^1$ die in Anspruch 7 angegebene Bedeutung hat, in Gegenwart von Jodwasserstoff und gegebenenfalls das erhaltene Produkt hydrolysiert.

10. Verfahren zur Herstellung eines acylierten Indol-Derivats der Formel

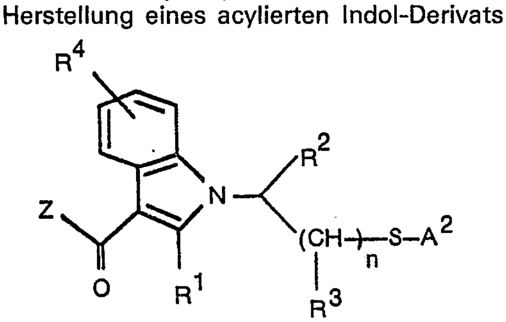

[I]−d

in der $R^1$, $R^2$, $R^3$, $R^4$, n und Z die in Anspruch 7 angegebene Bedeutung haben und $A^2$ die unten angegebene Bedeutung hat, wobei man ein durch N-(Hydroxyalkyl) acetylierter Indol-Derivat der Formel

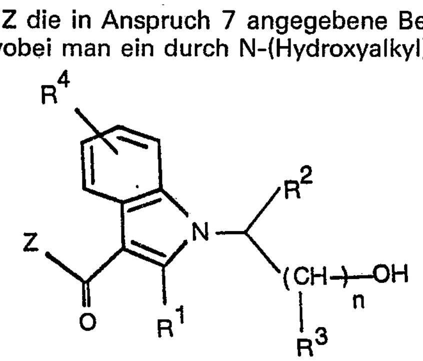

[I]−$a_1$

kondensiert mit einer organischen Disulfidverbindung der Formel

$$A^2—SS—A^2 \qquad (IV)$$

in der $A^2$ eine Alkylgruppe mit 1 bis 6 Kohlenstoffatomen oder eine Phenyl-, Phenylalkyl-, Pyridinyl-, Imidazolyl- oder Thiadiazolylgruppe, die gegebenenfalls durch eine geschützte Hydroxylgruppe oder $R^{42}$ wie in Anspruch 7 angegeben, substituiert ist, bedeutet,
in Gegenwart einer organischen Phosphinverbindung und einer stickstoffhaltigen basischen Verbindung.

11. Verfahren zur Herstellung eines acylierten Indol-Derivats der Formel

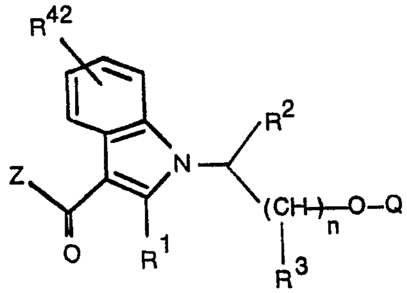

[I]-e

in der $R^1$, $R^2$, $R^3$, $R^{42}$, n und Z die in Anspruch 7 angegebene Bedeutung haben und Q eine Alkylgruppe mit 1 bis 6 Kohlenstoffatomen, eine Phenylalkylgruppe oder Phenylalkylgruppe die durch $R^{42}$ substituiert ist, bedeutet,
wobei man ein durch N-(Hydroxyalkyl) acyliertes Indol-Derivat der Formel

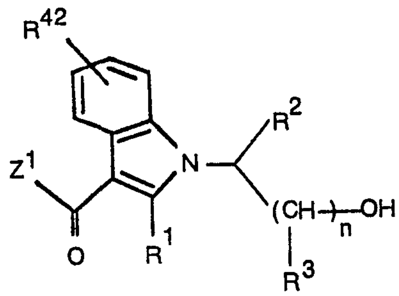

[I]-$a_2$

in der $Z^1$ die in Anspruch 7 angegebene Bedeutung hat, kondensiert mit einer Halogenverbindung der Formel

$$Q—X \qquad (V)$$

in der Q die oben angegebene Bedeutung hat und X ein Halogenatom bedeutet,
in Gegenwart einer basischen Verbindung und gegebenenfalls das erhaltene Produkt hydrolysiert.

12. Verfahren zur Herstellung eines acylierten Indol-Derivats der Formel

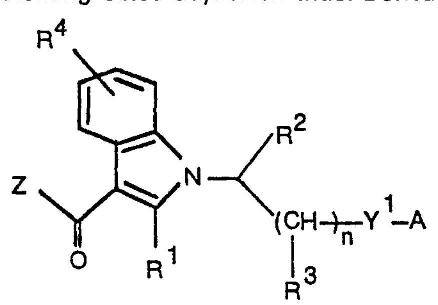

[I]-f

in der $Y^1$ eine Bindung oder —O— bedeutet und $R^1$, $R^2$, $R^3$, $R^4$, n, A und Z die in Anspruch 7 angegebene Bedeutung haben,

wobei man ein Tertrahydroindolderivat der Formel

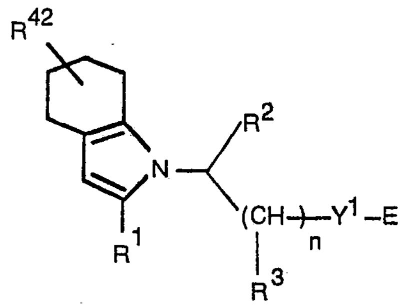

[VI]

in der $R^{42}$ und E die in Anspruch 7 angegebene Bedeutung haben, kondensiert mit einem Säureanhydrid der Formel

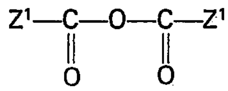

(III)

in der $Z^1$ die in Anspruch 7 angegebene Bedeutung hat, in Gegenwart von Jodwasserstoff unter Bildung eines acylierten Tetrahydroindolderivat der Formel

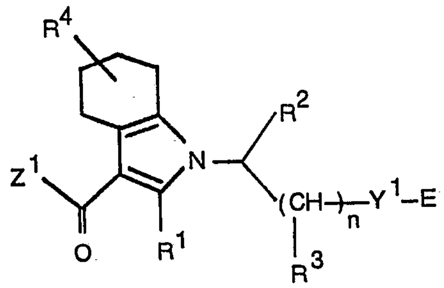

[VII]

und anschließend das erhaltene Produkt dehydriert und gegebenenfalls das dehydrierte Produkt hydrolysiert.

13. Verfahren zur Herstellung eines acylierten Indol-Derivats der Formel

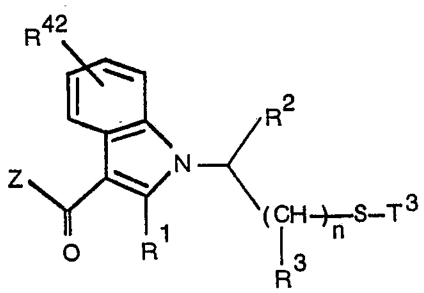

[I]–g

in der $R^1$, $R^2$, $R^3$, $R^{42}$, n und Z die in Anspruch 7 angegebene Bedeutung haben und $T^3$ ein Wasserstoffatom oder $T^2$ wie unten angegeben bedeutet, wobei man ein durch N-(Halogen-oder Sulfonyloxyalkyl) acyliertes Indol-Derivat der Formel

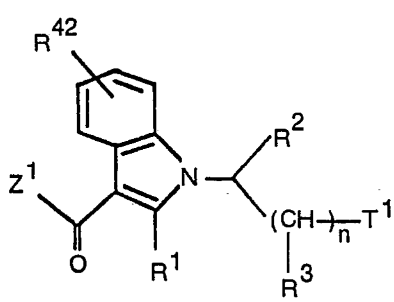

[VIII]

in der $Z^1$ die in Anspruch 7 angegebene Bedeutung hat und $T^1$ ein Halogenatom

$—OSO_2CH_3$, oder 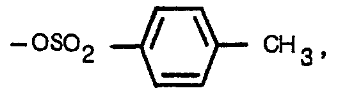

bedeutet,

umsetzt mit einer Thiocarbonsäure oder Thioharnstoff oder einem Alkalisalz davon der Formel

$$T^2—S—M \qquad (IX)$$

in der $T^2$ eine niedere Acyl-oder Amidinogruppe und M ein Wasserstoffatom oder ein Alkaliatom bedeutet, und gegebenenfalls das erhaltene Produkt hydrolysiert.

14. Verfahren zur Herstellung eines acylierten Indol-Derivats der Formel

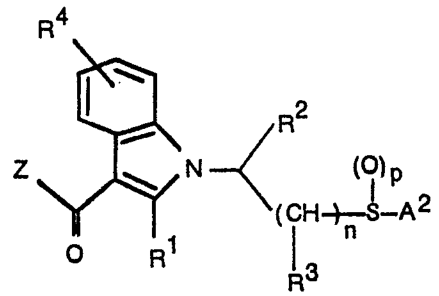

[I]-h

in der $R^1$, $R^2$, $R^3$, $R^4$, n und Z die in Anspruch 7 angegebene Bedeutung haben und $A^2$ die in Anspruch 10 angegebene Bedeutung hat und p 1 oder 2 bedeutet, wobei man ein acyliertes Indol-Derivat der Formel

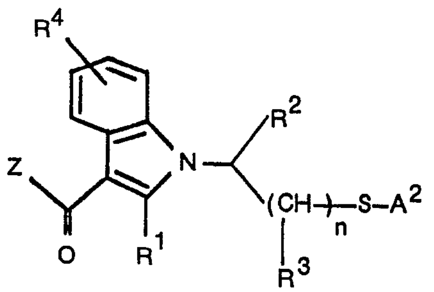

[I]-d

in an sich bekannter Weise oxidiert.

15. Pharmazeutisches Mittel zur Hemmung der Agglomaration von Blutplättchen, umfassend 1) eine pharmakologisch wirksame Menge eines acylierten Indol-Derivats der Formel [I]-1 wie in Anspruch 5 angegeben, oder eines optisch aktiven Isomers, oder eines Salzes davon und 2) einen pharmakologisch annehmbaren Träger.

16. Pharmazeutisches Mittel zur Hemmung der Agglomaration von Blutplättchen, umfassend 1) eine pharmakologisch wirksame Menge eines acylierten Indol-Derivats der Formel [I]-2 wie in Anspruch 6 angegeben oder eines optisch aktiven Isomers oder eines Salzes davon und 2) einen pharmakologisch annehmbaren Träger.

17. Arzneimittel zur Hemmung der Agglomaration von Blutplättchen, umfassend eine pharmakologisch wirksame Menge eines acylierten Indol-Derivats der Formel [I]-a wie in Anspruch 7 angegeben, oder eines optisch aktiven Isomers oder eines Salzes davon und einen pharmakologisch annehmbaren Träger.

18. Arzneimittel nach Anspruch 17, in einer Form zur oralen Verabreichung.